# EUROPEAN PATENT SPECIFICATION

(11) **EP 4 523 606 B1**
(45) Date of publication and mention of the grant of the patent: **27.08.2025**
(21) Application number: 23197119.3
(22) Date of filing: 13.09.2023
(51) Int. Cl.: A61B 3/00, A61B 3/10, A61B 3/12, G01B 9/02091

(54) **A FOURIER-DOMAIN OCT APPARATUS FOR OPTORETINOGRAPHY**
FOURIER-DOMÄNEN-OCT-VORRICHTUNG FÜR DIE OPTORETINOGRAPHIE
APPAREIL OCT À DOMAINE DE FOURIER POUR OPTORÉTINOGRAPHIE

(43) Date of publication of application: 19.03.2025
(73) Proprietor: Optos plc, Dunfermline, Scotland KY11 8GR (GB)
(72) Inventor: Preciado, Miguel, Dunfermline, Scotland, KY11 8GR (GB); McCool, Paul, Dunfermline, Scotland, KY11 8GR (GB); Normand, Margaret, Dunfermline, Scotland, KY11 8GR (GB); Rycroft, Ewan, Dunfermline, Scotland, KY11 8GR (GB); Anderson, Alan, Dunfermline Scotland KY11 8GR (GB)
(74) Representative: Hoffmann Eitle

(56) References cited:
- US-A1- 2021 022 601
- US-B2- 11 154 194
- US-B2- 7 506 978
- TAE-HOON KIM ET AL: "Functional Optical Coherence Tomography for Intrinsic Signal Optoretinography: Recent Developments and Deployment Challenges", ARXIV.ORG, CORNELL UNIVERSITY LIBRARY, 201 OLIN LIBRARY CORNELL UNIVERSITY ITHACA, NY 14853, 18 February 2022 (2022-02-18), XP091161748

## Description

### [Field]

Example aspects herein generally relate to the field of optical coherence tomography (OCT) imaging systems and, in particular, to Fourier-domain OCT imaging systems for acquiring optoretinography (ORG) data indicative of a physiological response of a retina of an eye of a subject to an optical stimulus.

### [Background]

Optical coherence tomography (OCT) is an imaging technique based on low-coherence interferometry, which is widely used to acquire high-resolution two- and three-dimensional images of optical scattering media, such as biological tissue.

OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between light in the reference arm and light in the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back-reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

OCT imaging systems can also be classified as being point-scan (also known as "point detection" or "scanning point"), line-scan or full-field, depending on how the imaging system is configured to acquire OCT data at locations on the imaging target. A point-scan OCT imaging system acquires OCT data by scanning a focused sample beam across the surface of the imaging target, typically along a single line (which may be straight, or alternatively curved so as to define a circle or a spiral, for example) or along a set of (usually substantially parallel) lines on the surface of the imaging target, and acquiring an axial depth profile (A-scan) for each of a plurality of points along the line(s), one single point at a time, to build up OCT data comprising a one- or two-dimensional array of A-scans representing a two-dimensional (i.e. a B-scan) or three-dimensional (i.e. a C-scan or volumetric scan) reflectance profile of the sample.

A line-scan OCT imaging system acquires OCT data by scanning a focused line of light across the surface of the imaging target. Measured reflectance from the imaging target is used to generate OCT data comprising a two-dimensional reflectance profile (i.e. a B-scan) of the sample. By scanning the focused line of light across a plurality of locations on the imaging target, OCT data comprising a three-dimensional reflectance profile (i.e. a C-scan or volumetric scan) of the sample can be obtained. Typically, the focused line of light is straight and is scanned in a direction perpendicular to it, although in some instances it may be curved with the scanning direction adjusted accordingly. A full-field OCT imaging system acquires OCT data by projecting a beam of light onto the imaging target to acquire OCT data comprising a three-dimensional reflectance profile (i.e. a C-scan or volumetric scan) of the sample.

OCT imaging systems can also be classified as being phase-resolved, where both the intensity and phase of the light reflected from the imaging target are measured as a function of axial depth. Modern FD-OCT imaging systems often have a degree of phase stability that allows them to function as phase-resolved OCT imaging systems.

Optoretinography (ORG) generally refers to the detection of a physiological response of a retina of an eye to an optical stimulus (i.e. light-induced functional activity of the retina). ORG techniques include the non-invasive optical imaging of this physiological response of the retina. For example, OCT imaging systems can be used to image retinal neurons exhibiting a change in dimension (size) in response to excitation by the optical stimulus. These changes in dimension have been shown to be detectable by phase-resolved OCT imaging systems and are typically changes in length of the outer segments of cone photoreceptors in the retina that are detected by measuring the change in axial depth of the inner-outer segment (IS/OS) junction and of the cone outer segment tip (COST) of the cone photoreceptors.

Further background is provided in the following documents.

US 11,154,194 B2 discloses a device and method with different modalities for synchronous focal or global retinal stimulation along with phase-measurements by optical interferometry to detect retinal activity. The developed optical interferometry based Focal detection of Focally stimulated Optical RetinoGram (FFORG) coupled with multifractal analysis enables measurement of sub-nanometer optical path-length (OPL) changes during functional activation in photo-transducing circuitry of the retina. In one example, Optical RetinoGram (ORG) is obtained for in-vivo label free detection of retinal layer activity under different conditions by using optical low coherence reflectometry scanning on retinal layer. In presence or absence of stimulation, specific activity in the retinal layer can be quantitatively obtained from the acquired optical interferogram by monitoring the change of optical path length variation with time. The ORG signal is a convoluted change in physical path length and refractive index in retina during visual stimulation. FFORG allows diagnosis of disease state in targeted areas of retina based on its response to focal stimulation. The FFORG system a 3D near-infrared image of backscattered light to be captured, thereby achieving precise signal localization and minimizing additional undesired photo stimulation. This depth-resolved functional imaging enables resolving layer specific retinal activity for monitoring progression of photoreceptor degeneration and/or retinal-circuitry dysfunction. FFORG provides accurate assessment of retinal function by testing retinal sensitivity at specific 3D location. Methods and systems for detecting retinal function from a specific position by axial A scan (FORG) are described. Generally, FORG/FFORG uses any methodology of low coherence optical interferometry, and provide a method of extracting a full set of optical properties from the back-reflected intrinsic optical signal and extracting optical information (phase and scattering) from the optical interference data set. The optical path-length change during optical stimulation can be measured from the phase data encoded in a specified frequency or frequency range obtained from the FFT analysis of the interferogram. A data acquisition card and software interface is used for data collection. Spectral interferograms are digitized and stored raw files for post-processing. Each spectral interferogram is Fourier transformed and phase at a spatial frequency channel corresponding to a particular retinal layer is calculated as a function of time, φ(d,t). Temporal phase difference (Δφ(d,tₙ )) at a given time is calculated by subtracting the measured phase of A-Scan at time tₙ from the first (baseline) A-Scan at the start of the experiment.

The article titled "Functional Optical Coherence Tomography for Intrinsic Signal Optoretinography: Recent Developments and Deployment Challenges" by Tae-Hoon Kim et al., Cornell University Library, 18 February 2022, discloses developments of functional OCT for ORG measurement, OCT intensity- and phase-based IOS processing.

### [Summary]

There is provided, in accordance with a first example aspect herein, a Fourier-domain optical coherence tomography (FD-OCT) apparatus arranged to acquire optoretinography, (ORG) data that is indicative of a physiological response of a retina of an eye of a subject to an optical stimulus. The FD-OCT apparatus comprises an optical system operable to apply the optical stimulus to the retina, an FD-OCT imaging system operable to acquire OCT data by imaging a portion of the retina, and a controller. The controller is arranged to: control the FD-OCT imaging system to acquire OCT data of a plurality of portions of the retina such that, for each portion of the plurality of portions of the retina, at least some of the OCT data acquired from the portion is acquired after a respective optical stimulus has been applied to the portion by the optical system; generate, based on the acquired OCT data, respective ORG data for each portion of the plurality of portions of the retina which is indicative of the respective response of the portion of the retina to the optical stimulus applied to the portion of the retina by the optical system; and store, for each portion of the plurality of portions of the retina, the respective ORG data generated for the portion in association with a respective indication of a position in a visual field of the eye of a point that is optically conjugate with a corresponding position of the portion on the retina.

The controller may be further arranged to determine a respective indication of a position on the retina of each portion of the plurality of portions of the retina at which OCT data is to be acquired by the FD-OCT imaging system by determining, for each point of a plurality of points in the visual field of the eye, a respective indication of a corresponding position on the retina that is optically conjugate with the point. Alternatively, the controller may be further arranged to: determine a respective indication of a position on the retina of each portion of the plurality of portions of the retina at which OCT data is to be acquired by the FD-OCT imaging system based on an image of the retina of the eye; and determine, for each portion of the plurality of portions of the retina, the respective indication of the position in the visual field of the eye of the point that is optically conjugate with the position of the portion on the retina.

The FD-OCT apparatus may further comprise a display device, and the controller may be further arranged to: acquire data indicating how measurements of the subject's ability to see optical stimuli applied confined to respective different portions of the retina of the eye at different respective locations on the retina are distributed over at least a part of a visual field of the eye; control the display device, based on at least some of the acquired data, to display a first map indicative of how the subject's ability to see the optical stimuli is distributed over the at least a part of a visual field of the eye; and control the display device, based on at least some of the stored ORG data and the associated stored indications, to display a second map for comparison with the first map, the second map indicating how the physiological response of the retina to the optical stimulus indicated by at least some of the stored ORG data is distributed over the at least a part of the visual field of the eye.

There is provided, in accordance with a second example aspect herein, a Fourier-domain optical coherence tomography (FD-OCT) apparatus arranged to acquire optoretinography (ORG) data that is indicative of a physiological response of a retina of an eye of a subject to an optical stimulus. The FD-OCT apparatus comprises an optical system operable to apply the optical stimulus to the retina, an FD-OCT imaging system operable to acquire OCT data by imaging a portion of the retina, and a controller. The controller is arranged to: control the FD-OCT imaging system to acquire OCT data of a plurality of portions of the retina such that, for each portion of the plurality of portions of the retina, at least some of the OCT data acquired from the portion is acquired after a respective optical stimulus has been applied to the portion by the optical system; generate, based on the acquired OCT data, respective ORG data for each portion of the plurality of portions of the retina which is indicative of the respective response of the portion of the retina to the optical stimulus applied to the portion of the retina; store, for each portion of the plurality of portions of the retina, the respective ORG data generated from OCT data acquired from the portion in association with a respective indication of a location of the portion on the retina; and acquire data indicating how measurements of the subject's ability to see optical stimuli that are confined to respective different portions of the retina of the eye at different respective locations on the retina are distributed over the at least a part of the retina of the eye. The controller may be arranged to acquire the data, which indicates how measurements of the subject's ability to see optical stimuli applied to respective different locations on the retina of the eye are distributed over at least a part of the retina of the eye, by: receiving visual field test data acquired from the eye by a visual field-testing device, the visual field test data comprising measurements of the subject's ability to see optical stimuli applied from a plurality of points in the visual field of the eye of the subject; and determining, for each point of the plurality of points in the visual field of the eye, a respective indication of a corresponding position on the retina that is optically conjugate with the point. Alternatively, the controller may be arranged to acquire the data, which indicates how measurements of the subject's ability to see optical stimuli applied to respective different locations on the retina of the eye are distributed over at least a part of the retina of the eye, by: receiving indications, provided by the subject, of whether the subject saw the respective optical stimulus applied to each portion of the plurality of portions of the retina by the optical system; and associating each of the received indications with a respective indication of a location of the respective portion on the retina. In either case, the FD-OCT apparatus may further comprise a display device, and the controller may be further arranged to: control the display device, based on at least some of the acquired data, to display a first map indicative of how the subject's ability to see the optical stimuli with the eye is distributed over at least a part of the retina of the eye; and control the display device, based on at least some of the generated ORG data and positions of the portions of the retina from which the at least some of the ORG data was generated, to display a second map for comparison with the first map, the second map being indicative of how the response of the retina to optical stimulus, which is indicated by the at least some of the generated ORG data is distributed over the at least a part of the retina of the eye.

The controller may be further arranged to compare data of the first map with data of the second map and identify, based on the comparison, at least one of: one or more first regions of the at least a part of the visual field, indicating that the subject is able to see visual field test optical stimuli in the one or more first regions of the at least a part of the visual field, and indicating that one or more corresponding regions of the retina provided a physiological response to the optical stimulus which satisfies a predetermined condition; one or more second regions of the at least a part of the visual field, indicating that the subject has an inability to see visual field test optical stimuli in the one or more second regions of the at least a part of the visual field, and indicating that one or more corresponding regions of the retina provided a physiological response to the optical stimulus which satisfies the predetermined condition; one or more third regions of the at least a part of the visual field, indicating that the subject is able to see visual field test optical stimuli in the one or more third regions of the at least a part of the visual field, and indicating that one or more corresponding regions of the retina provided a physiological response to the optical stimulus which does not satisfy the predetermined condition; or one or more fourth regions of the at least a part of the visual field, indicating that the subject has an inability to see visual field test optical stimuli in the one or more fourth regions of the at least a part of the visual field, and indicating that one or more corresponding regions of the retina provided a physiological response to the optical stimulus which does not satisfy the predetermined condition.

The FD-OCT apparatus of an example embodiment may further comprise a fixation target arranged to fix a gaze direction of the eye, and the optical system may be operable to apply the optical stimulus to the retina such that an illumination of the retina by the optical stimulus is confined to a portion of the retina, the optical system being controllable by the controller to vary a location on the retina at which the optical stimulus is to be applied and comprising a light source arranged to generate light which provides the optical stimulus, and one or more scanning elements arranged to direct the light to the retina. In this example embodiment, the controller may be further arranged to: acquire an indicator of a target location on the retina at which the optical stimulus is to be applied by the optical system; use the acquired indicator to control the one or more scanning elements of the optical system, while a position of the fixation target relative to the eye remains fixed, to direct the light to a first portion of the retina which is at the target location; control the FD-OCT imaging system to acquire OCT data of a second portion of the retina, wherein at least a part of the second portion of the retina is disposed in relation to the first portion so as to be stimulated by the applied optical stimulus during acquisition of at least some of the OCT data; and generate ORG data based on the acquired OCT data of the second portion of the retina.

In the FD-OCT apparatus of the example embodiment, the FD-OCT imaging system may further comprise an interferometer having a sample arm and a reference arm, and a detector arranged to detect an interference between sample OCT light propagating along the sample arm after having been scattered from the retina, and reference OCT light propagating along the reference arm. At least one of the one or more scanning elements may be further arranged to direct the sample OCT light toward the second portion of the retina, and the sample OCT light scattered from the second portion of the retina toward the detector.

Alternatively, the FD-OCT imaging system may comprise an interferometer having a sample arm and a reference arm, one or more scanning elements, and a detector arranged to detect an interference between sample OCT light propagating along the sample arm after having been scattered from the retina, and reference OCT light propagating along the reference arm. Furthermore, the one or more scanning elements may be arranged to direct the sample OCT light toward the second portion of the retina, and the sample OCT light scattered from the second portion of the retina toward the detector. In this alternative, the one or more scanning elements of the optical system are different from the one or more scanning elements of the FD-OCT imaging system. In addition, the controller may be arranged to use the acquired indicator to control the one or more scanning elements of the optical system independently from the one or more scanning elements of the FD-OCT imaging system while the position of the fixation target relative to the eye remains fixed.

Each first portion of the retina may be smaller than a region of the retina over which the FD-OCT imaging system is operable to acquire OCT data.

There is provided, in accordance with a third example aspect herein, a computer-implemented method of controlling a Fourier-domain optical coherence tomography (FD-OCT) apparatus to acquire optoretinography (ORG) data indicative of a physiological response of a retina of an eye of a subject to an optical stimulus. The FD-OCT apparatus comprises an optical system operable to apply the optical stimulus to the retina, and an FD-OCT imaging system operable to acquire OCT data by imaging a portion of the retina. The method comprises: controlling the FD-OCT imaging system to acquire OCT data of a plurality of portions of the retina such that, for each portion of the plurality of portions of the retina, at least some of the OCT data acquired from the portion is acquired after a respective optical stimulus has been applied to the portion by the optical system; generating, based on the acquired OCT data, respective ORG data for each portion of the plurality of portions of the retina which is indicative of the respective response of the portion of the retina to the optical stimulus applied to the portion of the retina by the optical system; and storing, for each portion of the plurality of portions of the retina, the respective ORG data generated for the portion in association with a respective indication of a position in a visual field of the eye of a point that is optically conjugate with a corresponding position of the portion on the retina.

There is provided, in accordance with a fourth example aspect herein, a computer-implemented method of controlling a Fourier-domain optical coherence tomography (FD-OCT) apparatus to acquire optoretinography (ORG) data indicative of a physiological response of a retina of an eye of a subject to an optical stimulus. The FD-OCT apparatus comprises an optical system operable to apply the optical stimulus to the retina, and an FD-OCT imaging system operable to acquire OCT data by imaging a portion of the retina. The method comprises: controlling the FD-OCT imaging system to acquire OCT data of a plurality of portions of the retina such that, for each portion of the plurality of portions of the retina, at least some of the OCT data acquired from the portion is acquired after a respective optical stimulus has been applied to the portion by the optical system; generating, based on the acquired OCT data, respective ORG data for each portion of the plurality of portions of the retina which is indicative of the respective response of the portion of the retina to the optical stimulus applied to the portion of the retina; storing, for each portion of the plurality of portions of the retina, the respective ORG data generated from OCT data acquired from the portion in association with a respective indication of a location of the portion on the retina; and acquiring data indicating how measurements of the subject's ability to see optical stimuli that are confined to respective different portions of the retina of the eye at different respective locations on the retina are distributed over the at least a part of the retina of the eye.

There is provided, in accordance with a fifth example aspect herein, a computer program comprising computer-readable instructions which, when executed by a computer, cause the computer to perform a method according to at least one of the third example aspect and the fourth example aspect above. The computer program may be stored on a non-transitory computer-readable storage medium or may be carried by a signal, for example.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an FD-OCT apparatus 100 according to an example embodiment herein.
Figure 2 is a schematic front view of a display device 200 of the FD-apparatus 100, which displays a graphic 210 as an example of a fixation target for fixing a gaze direction of an eye.
Figure 3A is a schematic illustration of an optical system 300 and an FD-OCT imaging system 320 according to the example embodiment herein.
Figure 3B is a schematic illustration of an example optical system 330 and an example FD-OCT imaging system 331 that share an example scanning system 332, which includes lens-based optics instead of the curved mirrors 313 and 315 in Figure 3A.
Figure 3C is a schematic illustration of an optical system 340 and an FD-OCT imaging system 341 according to an alternative implementation of the example embodiment herein.
Figure 3D is a schematic illustration of an example optical system 350 and an example FD-OCT imaging system 351 that share an example scanning system 352, which includes lens-based optics instead of the curved mirrors 313 and 315 in Figure 3C.
Figure 3E is a schematic illustration of an optical system 360 and an FD-OCT imaging system 361 according to a further alternative implementation of the example embodiment.
Figure 3F is a schematic illustration of an example optical system 370 and an example FD-OCT imaging system 371 that share an example scanning system 372 using a two-dimensional scanner 373 instead of the third scanning element 363 and the fourth scanning element 364 in Figure 3E.
Figure 4 is a schematic illustration of a programmable signal processing hardware, which may be configured to perform the functions of the controller 140 described herein.
Figure 5 is a flow diagram illustrating a process by which controller 140 generates ORG data 150 according to the example embodiment herein.
Figures 6A, 6B and 6C are schematic illustrations of example first portions and second portions of the retina in the performance of processes S52 and S53 in Figure 5 using the optical system 300 and the FD-OCT imaging system 320 of the example embodiment.
Figures 6D and 6E are schematic illustrations of example first portions and second portions of the retina in the performance of processes S52 and S53 in Figure 5 using the optical system 360 and the FD-OCT imaging system 361 in Figure 6E.
Figure 6F is a schematic illustration of a first portion and a second portion in the performance of processes S52 and S53 in Figure 5 using the optical system 340 and the FD-OCT imaging system 341 in Figure 3C.
Figure 7 is a flow diagram illustrating a process by which controller 140 may generate ORG data 150 for a plurality of locations on the retina.
Figure 8A shows an example plurality of first portions 801 to 807 and an example plurality of second portions 811 to 817 in the performance of processes S72 and S73 in Figure 7 during an ORG data capture session.
Figure 8B shows an example plurality of first portions 821 to 824 and an example plurality of second portions 825 to 824 in the performance of processes S72 and S73 in Figure 7 during an ORG data capture session.
Figure 8C shows an example plurality of first portions 831 to 834 and an example plurality of second portions 835 to 838 in the performance of processes S72 and S73 in Figure 7 during an ORG data capture session.
Figure 8D shows an example plurality of first portions 841 to 844 and an example plurality of second portions 845 to 848 in the performance of processes S72 and S73 in Figure 7 during and ORG data capture session.
Figure 8E shows an example plurality of first portions 851, 852, 855 and 856, and an example plurality of second portions 853, 854, 857 and 858, in the performance of processes S72 and S73 in Figure 7 during an ORG data capture session.
Figure 9 shows an example first portion 901 and an example second portion 902 in the performance of processes S52 and S53 in Figure 5, or processes S72 and S73 in Figure 7, during an ORG data capture session.
Figure 10 shows an example first portion 1001 and an example second portion 1002 in the performance of processes S52 and S53 in Figure 5, or processes S72 and S73 in Figure 7, during an ORG data capture session.
Figure 11 is a flow diagram illustrating a process by which the controller 140 of the example embodiment may determine indicators of target locations on the retina at which the optical stimulus is to be applied, based on points in a visual field of the eye.
Figure 12 is a flow diagram illustrating a process by which the controller 140 may select the indicators and corresponding positions at which OCT data is to be acquired, and map positions on the retina at which OCT data has been acquired to corresponding positions in the visual field of the eye.
Figure 13 is a schematic illustration of a data structure 1300 which may be stored by the controller 140, wherein respective ORG data generated for each portion of a plurality of separate portions of the retina is stored in association with a respective indication of a position in the visual field of the eye of a point which is optically conjugate with the position of the portion.
Figure 14 is a flow diagram illustrating a process by which the controller 140 acquires data indicating how measurements of the subject's ability to see optical stimuli applied to respective different locations on the retina of the eye are distributed over at least a part of a visual field of the eye, and controls a map display device 142 to display maps described herein.
Figure 15 shows a flow diagram illustrating a process by which the controller 140 may acquire the data in process S1401 of Figure 14.
Figure 16 shows an example first map 1400, which may be displayed by the map display device 142 in process S1402 of Figure 14.
Figure 17 shows an example second map 1500, which may be displayed by the map display device 142 in process S1403 of Figure 14.
Figure 18 shows an example third map 1600, which may be displayed by the map display device 142 in process S1405 of Figure 14.
Figure 19 is a flow diagram illustrating a process by which the controller 140 may acquire data indicating how measurements of the subject's ability to see optical stimuli applied to respective different locations on the retina of the eye 160 are distributed over at least a part of the retina of the eye 160, and control the map display device 142 to display a fourth map and a fifth map described herein
Figure 20A shows a flow diagram illustrating a process by which the controller 140 may acquire the data in process S2102 of Figure 19.
Figure 20B shows a flow diagram illustrating an alternative process by which the controller 140 may acquire the data in process S2102 of Figure 19.
Figure 21 shows an example fourth map 1700, which may be displayed by the map display device 142 in process S2103 of Figure 19.
Figure 22 shows an example fifth map 1800, which may be displayed by the map display device 142 in process S2104 of Figure 19.
Figure 23 is a flow diagram illustrating a computer-implemented method of controlling a FD-OCT apparatus to acquire ORG data according to an example embodiment.
Figure 24 is a flow diagram illustrating a computer-implemented method of controlling a FD-OCT apparatus to acquire ORG data according to another example embodiment.

### [Detailed Description of Example Embodiments]

Visual field tests are widely used to determine the extent of, and function within, the visual field of an eye. A visual field test relies on the subject to provide an indication (often in the form of a button press or by verbal communication) that they are able to see an optical stimulus presented to the subject from a location within the visual field of the eye being tested. Perimetry is a type of visual field test where the visual field of the eye is systematically measured. A variety of perimetry techniques are known such as automatic static perimetry, the Goldmann method and microperimetry, for example.

Such visual field tests are based on the output of the whole visual pathway of the eye, i.e. the collective functionally of the visual pathway which results in the subject's perception of whether or not they are able to see an optical stimulus being presented to them. Accordingly, it is difficult or impossible to attribute an inability of a subject to see an applied optical stimulus within the visual field of their eye recorded in a visual field test to a particular intermediary within the visual pathway of the eye, and this limits both the extent and accuracy to which a clinically useful diagnosis can be made. Prior efforts to fill this gap in physiological information regarding the visual pathway have tended to focus on microperimetry, whereby the visual field data is displayed on a retinal image to facilitate the correlation of functional abnormalities with structural features on the retina. However, this relies on an interpretation of structural features on the retina's surface, rather than providing any definitive indication of the function of the retina at a given point in the eye.

In view of the above-described problems, the inventors have devised a Fourier-domain optical coherence tomography (FD-OCT) apparatus which is arranged to acquire optoretinography (ORG) data and store this data in association with an indication of a position in a visual field of the eye of a point that is optically conjugate with a corresponding position of the OCT data used to generate the ORG data, so that the ORG data may be displayed alongside visual field test data acquired by a perimetry device, for example. The inventors have also devised a FD-OCT apparatus which is arranged to acquire data indicating how the subject's ability to see optical stimuli is distributed over a part of the retina (as may be acquired from a microperimetry device, for example) and, for comparison, further data which is derived from acquired ORG data and indicates how the response of the retina to optical stimuli is distributed over the part of the retina.

The ORG data may thus be used to provide information, complementary to that acquired from a visual field test or the like, regarding the functionality of the photoreceptor cells of the eye that form part of within the visual pathway of the subject. This information may allow the clinician to gain a valuable insight quickly and easily into possible causes of a vision loss (or impaired vision) in a region of the eye's visual field or retina identified by the results of a visual field test. The information may, for example, allow the clinician to quickly and accurately identify a region of the eye's visual field or retina for which a loss of impairment of vision due to the abnormal functionality of photoreceptor cells can be ruled out with a reasonable degree of certainty. The information may further enable the identification of false positives in the visual field test (e.g. where the subject has indicated they are able to see the optical stimulus but there has been no observed retinal response to corroborate this) and the affirmation of negative results, thus improving the accuracy of the visual field test data.

Example embodiments of the aforementioned FD-OCT imaging apparatus will now be described in detail with reference to the accompanying drawings.

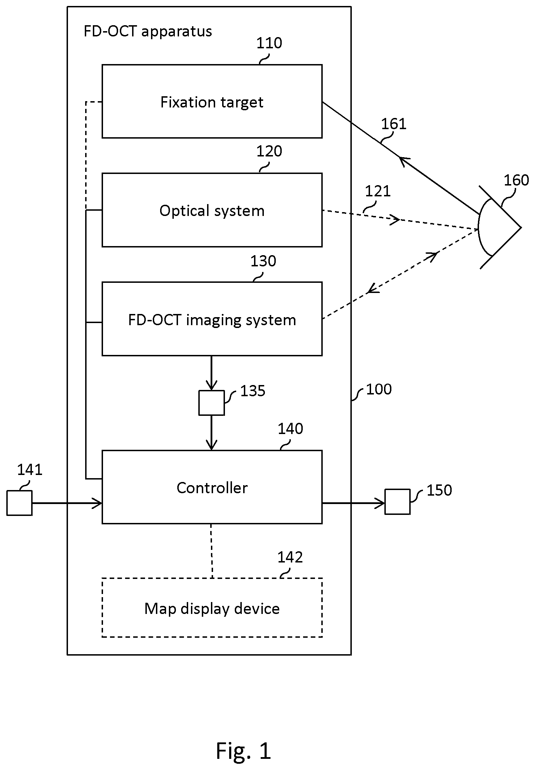

Figure 1 is a schematic illustration of a FD-OCT apparatus 100 arranged to acquire optoretinography (ORG) data 150 that is indicative of a physiological response of a retina of an eye 160 of a subject to an optical stimulus, according to an example embodiment herein. The FD-OCT apparatus 100 comprises a fixation target 110, an optical system 120, an FD-OCT imaging system 130, a controller 140 and, optionally, a map display device 142.

The fixation target 110 is arranged to fix a gaze direction 161 of the eye 160 (i.e. a direction away from the eye 160 along the visual axis of the eye 160). This fixation may occur be when the eye 160 fixates on the fixation target 110. That is, the fixation target 110 is arranged to be viewable by the eye 160 so as to fix the gaze direction 161 of the eye 160 when gazed at by the eye 160, during acquisition of the ORG data 150. However, the fixation target 110 may alternatively be arranged to fix the gaze direction 161 of the eye 160 when the other eye of the subject fixates on the fixation target 110. In the majority of subjects that do not have strabismus or the like, the muscles that control eye movement work together and point both eyes in the same direction so that the fixation of the gaze direction of one of the eyes by the fixation target 110 results in the other eye (even when this other eye cannot see the fixation target 110) having the same gaze direction. For example, where the eye 160 is the right eye of the subject then the gaze direction 161 of the eye 160 may fixed by virtue of the left eye of the subject fixating on the fixation target 110. The position of the fixation target 110 relative to the eye 160 may, as in the present example embodiment, be controllable by the controller 140 so as to control a gaze direction 161 of the eye 160 when the eye 160 fixates on the fixation target 110. However, the position of the fixation target 110 may alternatively be set in a fixed position, relative to the expected position of the eye 160 (i.e. the location where the eye 160 is placed relative to the FD-OCT imaging system 130 for the FD-OCT imaging system 130 to acquire the AS-OCT image 130) during manufacture or installation/set-up of the FD-OCT imaging system 130. The fixation target 110 may be integrated into the FD-OCT apparatus 100 so as to be viewable by the eye 160 in various ways that are well-known to those skilled in the art or as is described in US 11,253,146 B2, the contents of which are hereby incorporated by reference in their entirety.

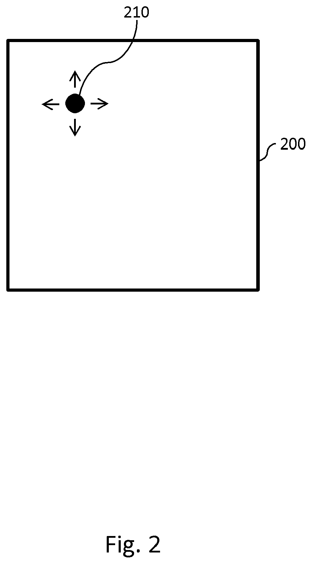

Figure 2 is a schematic illustration of an example implementation of the fixation target 110 in the form of a graphic 210, which is displayed by a display device 200 forming part of the FD-OCT apparatus 100. The display position of the graphic 210 relative to the eye 160 is controllable by the controller 140 so as to control a gaze direction 161 of the eye 160 when the eye 160 fixates on the graphic 210. In other words, the position of the eye 160 relative to the FD-OCT imaging system 130 is fixed (e.g. by placing the patient's chin on a chin rest of the FD-OCT imaging system 130), and the display position of the graphic 210 on the display area of the display device 200 is adjustable, as indicated by the arrows in Figure 2, so as to set (steer) the gaze direction 161 of the eye 160 when the eye 160 fixates on the graphic 210. Although the graphic 210 takes the form of a dot in Figure 2, it will be appreciated that the form of the graphic is not so limited, and may alternatively be provided as a cross, circle or any shape onto which the eye 160 can fixate.

Furthermore, the fixation target 110 may be implemented in a form other than a graphic displayed by a display device 200. For example, the fixation target 110 may include a light source (e.g. a light-emitting diode) attached to an actuator that is controllable by the controller 140 to move the light source relative to the eye 160 so as to control a gaze direction 161 of the eye 160 when the eye 160 fixates on the light source.

The optical system 120 is operable to apply the optical stimulus to the retina of the eye 160 such that the illumination of the retina by the optical stimulus is confined to (and may span or fill) a portion of the retina, the optical system 120 being controllable, while the gaze direction of the eye 160 remains fixed by the fixation target 110, to vary a location on the retina of the eye 160 at which the optical stimulus is to be applied. In other words, the optical system 120 is controllable to apply the optical stimulus to each of a plurality of different portions of the retina of the eye 160 whereby, when the optical stimulus is applied to a portion of the plurality of portions of the retina of the eye 160, the optical stimulus is not applied to any of the other portions of the plurality of portions of the retina. The optical system 120 is controllable to vary the location on the retina at which the optical stimulus is to be applied while a position of the fixation target 130 relative to the eye 160 remains fixed (and the gaze direction 161 of the eye 160 remains fixed on the fixation target 130), as described in more detail below.

The optical stimulus may, as in the present example embodiment, be a flash of light of a predetermined duration which is applied to the retina of the eye 160 along an optical path 121 of the optical system 120. The duration of the flash of light is typically much shorter than the time period within which OCT data is acquired by FD-OCT imaging system 130 for generating the ORG data 150, and may be dependent on the intensity of the stimulus. The duration of the flash may be between 5 ms and 50 ms, for example. The duration of the optical stimulus may be controlled by the controller 140, as described in more detail below.

The FD-OCT imaging system 130 (which may be a phase-stable FD-OCT imaging system) may, as in the present example embodiment, be a point-scan FD-OCT imaging system. However, the FD-OCT imaging system 130 may take other forms, such as a line-scan or full-field FD-OCT imaging system. The FD-OCT imaging system 130 is operable to acquire complex OCT data 135 by imaging a portion of the retina of the eye 160. The OCT data 135 may, as in the present example embodiment, comprise a temporal sequence of OCT images, such as a temporal sequence of A-scans, B-scans or C-scans, for example.

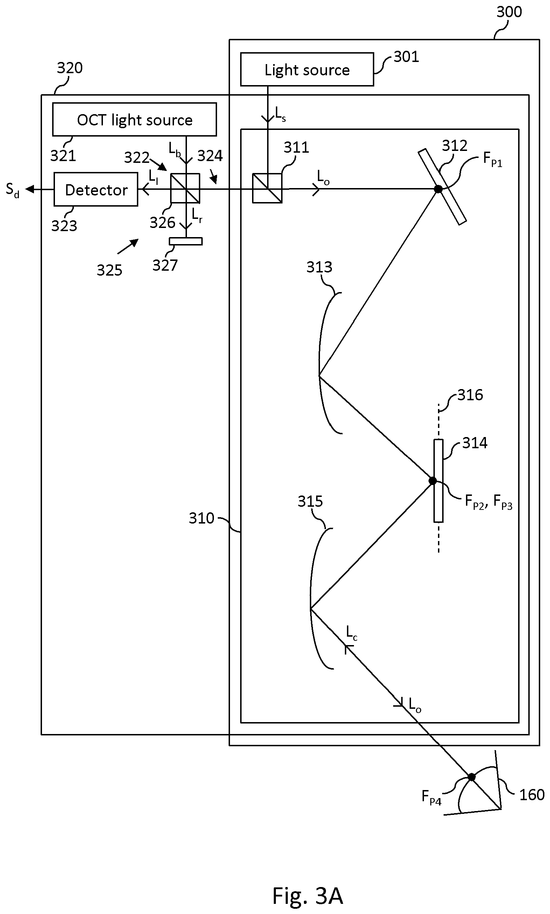

Figure 3A is a schematic illustration of an optical system 300 and an FD-OCT imaging system 320 which may, as in the present example embodiment, form example implementations of the optical system 120 and the FD-OCT imaging system 130 of Figure 1. The optical system 300 comprises a light source 301 and the FD-OCT imaging system 320 comprises an OCT light source 321, an interferometer 322 and a detector 323. A scanning system 310 is shared by both the optical system 300 and the FD-OCT imaging system 310, as described below, and comprises a beam splitter 311, a first scanning element 312, a first curved mirror 313, a second scanning element 314 and a second curved mirror 315. Although a fixation target is not illustrated in Figure 3A for clarity, one may be located in the scanning system 310, on at least one side of the first curved mirror 313, above and/or below the plane of the Figure, as described in US 11,253,146 B2. Light from such a fixation target may thus pass via the second scanning element 314 and then the second curved mirror 315 to the eye 160.

The OCT light source 321 of the FD-OCT imaging system 320 is arranged to generate an OCT beam L_{b}. The OCT light source 321 may, as in the present example embodiment, comprise an illumination source and an illumination source aperture. In this case, the illumination source is arranged to emit light through the light source aperture to generate the OCT beam L_{b}, such that the shape and size (e.g. diameter, in case of the light source aperture being circular) of the illumination source aperture defines the cross-sectional shape and size (e.g. diameter) of the OCT beam L_{b} (i.e. so that these sizes and shapes are the same). The illumination source may, where the FD-OCT imaging system 320 is a swept-source OCT (SS-OCT) imaging system, be a swept illumination source arranged to generate light having a wavelength that is swept over a range of wavelengths during a scan performed by the SS-OCT imaging system or, where the FD-OCT imaging system 320 is a spectral-domain OCT (SD-OCT) imaging system, be a broadband light source which is arranged to generate light simultaneously having a range of wavelengths (i.e. a broad spectral content) during a scan performed by the SD-OCT imaging system. The illumination source may be any known swept or broadband source (as the case may be). For example, the illumination source may comprise a laser or a light emitting diode.

The OCT light source 321 may comprise further components, such as one or more collimating lenses for collimating light from the light source, for example. Further, the OCT light source 321 may alternatively take other forms where the FD-OCT imaging system 320 is a line-field or full-field FD-OCT imaging system as would be readily appreciated by those skilled in the art. For example, where the FD-OCT imaging system 320 is a line-field FD-OCT imaging system, the OCT light source 321 may be arranged to generate a line of light and may comprise a laser and a one or more cylindrical lenses (e.g. combination of a plano-concave lens and a plano-convex lens that are arranged to focus the beam from the laser in respective directions that are orthogonal to one another to form a line of light), although any other kind of spatial light modulator for beam shaping known to those versed in the art may alternatively be employed.

The interferometer 322 is arranged to split the OCT beam L_{b} from the OCT light source 321 to propagate along a sample arm 324 of the interferometer 322, as sample OCT light Lₒ, and to propagate along a reference arm 325 of the interferometer 322, as reference OCT light Lᵣ. The interferometer 321 is further arranged to receive light L_{c} which has been scattered by a portion of the retina of the eye 160 and collected by the scanning system 310, to generate an interference light Lₗ resulting from an interference between the reference OCT light Lᵣ and the collected light L_{c}, and to output the interference light Lₗ to the detector 323. In other words, the reference OCT light Lᵣ propagating along the reference arm 325 and the collected light L_{c} which has been scattered by the portion of the retina of the eye 160 and collected by the scanning system 310 during a scan performed by the FD-OCT imaging system 320 are guided to coincide and interfere with one another, and the resulting interference line of light Lₗ is directed to and received by the detector 323.

The interferometer 322 may, as in the present example embodiment, be a Michelson interferometer using a beam-splitter 326 to split the OCT beam L_{b} to propagate along the sample arm 324 and the reference arm 325 of the interferometer 322, and to interfere the reference OCT light Lᵣ that has been reflected from a reference mirror 327 with the collected light L_{c} from the scanning system 310. Although a Michelson interferometer has been described, those skilled in the art will appreciate that the interferometer 322 is not so limited and any interferometer suitable for OCT may be used such as, for example, a Mach-Zehnder interferometer. Further, those skilled in the art will appreciate that the interferometer 322 may also be adapted as appropriate where the FD-OCT imaging system 320 is a line-scan or full-field FD-OCT imaging system. For example, where the FD-OCT imaging system 320 is a line-scan FD-OCT imaging system, the interferometer 322 may be a free-space interferometer using at least one beam splitter. Furthermore, the interferometer 322 is not limited to being a free-space interferometer, and may instead be a fibre-based interferometer. In this case, the interferometer may employ a fibre coupler to split the OCT beam L_{b} to propagate along the sample arm and reference arm of the interferometer 322, and to interfere the reference light L_{R} and the collected light L_{c}.

The scanning system 310 is arranged to perform a (e.g. a one- or two-dimensional) point-scan of the sample OCT light Lₒ across the portion of the retina of the eye 160, and collect light L_{c} which has been scattered by the portion of the retina of the eye 160 during the point scan. The scanning system 310 is therefore arranged to acquire A-scans at respective scan locations that are distributed (e.g. one- or two-dimensionally) across the portion of the retina of the eye 160, by sequentially illuminating the scan locations with the sample OCT light Lₒ, one scan location at a time, and collecting at least some of the light L_{c} scattered by the portion of the retina of the eye 160 at each scan location. By acquiring the successive A-scans, the scanning system 310 is thus able to acquire B-scans and/or C-scans, of the retina of the eye 160.

The sample OCT light Lₒ enters the scanning system 310 from the interferometer 322 and propagates to the beam splitter 311. The sample OCT light Lₒ is then reflected, in sequence, by the first scanning element 312, the first curved mirror 313, the second scanning element 314 and the second curved mirror 315, before being incident on the portion of the retina of the eye 160. The light L_{c} which has been scattered by the portion of the retina of the eye 160 and collected by the scanning system 310 follows the same optical path through the scanning system 310 as the sample OCT light Lₒ, but in reverse order, and exits the scanning system 310 after having propagated via the beam splitter 311.

The point scan is performed by the scanning system 310 by the first scanning element 312 rotating around the first axis (not shown) to scan the sample OCT light Lₒ in a first direction, or a direction opposing the first direction, across the portion of the retina of the eye 160, and/or by the second scanning element 314 rotating around the second axis 316 to scan the sample OCT light Lₒ in a second direction, or in a direction opposing the second direction, across the portion of the retina of the eye 160. The second direction may, as in the present example embodiment, be orthogonal to the first direction. Thus, by rotating the first scanning element 312 and the second scanning element 314, it is possible to steer the sample OCT light Lₒ to any position on the portion of the retina of the eye 160 that is within the field of view of the FD-OCT apparatus 100. As described above, the rotation of the first scanning element 312 and the second scanning element 314 is coordinated by the controller 140, or by a dedicated scanning system controller (not shown), such that the sample OCT light Lₒ is scanned across the portion of the retina of the eye 160 in accordance with a predefined scan pattern. The predefined scan pattern may be any suitable scan pattern known to those versed in the art, for example a unidirectional scan (wherein a set of parallel scan lines are followed in a common direction, along which they extend), a circular scan, a serpentine scan or spiral scan, which may either be present, or at manufacture selected by the user of the FD-OCT apparatus 100.

The first curved mirror 313 and the second curved mirror 315 may, as in the present example embodiment, be respective ellipsoidal mirrors each having a first focal point and a conjugate second focal point. The first scanning element 312 is located at the first focal point F_{P1} of the first curved mirror 313, and the second scanning element 314 is located at the second focal point F_{P2} of the first curved mirror 313. The second scanning element 314 is also located at the first focal point F_{P3} of the second curved mirror 315, and the eye 160 is in a vicinity of the second focal point F_{P4} of the second curved mirror 315. More specifically, the pupil of the eye 160 is located at the second focal point F_{P4} of the second curved mirror 315 such that the optical path of the scanning system 310 may be steered in two-dimensions across a region of the retina of the eye 160. However, the first curved mirror 313 and the second curved mirror 315 may be any reflective components having an aspherical reflective surface, such as a shape of a conical section like a parabola or hyperboloid, or may, more generally, have a shape described by one or more polynomial functions of two variables.

The use of curved mirrors in the scanning system 310 allows the FD-OCT imaging system 130 to function as a wide-field FD-OCT imaging system, or an ultra-widefield (UWF) FD-OCT imaging system, as in described in further detail in WO 2014/53824 A1, the content of which is hereby incorporated by reference in its entirety. However, the scanning system 310 is not so limited.

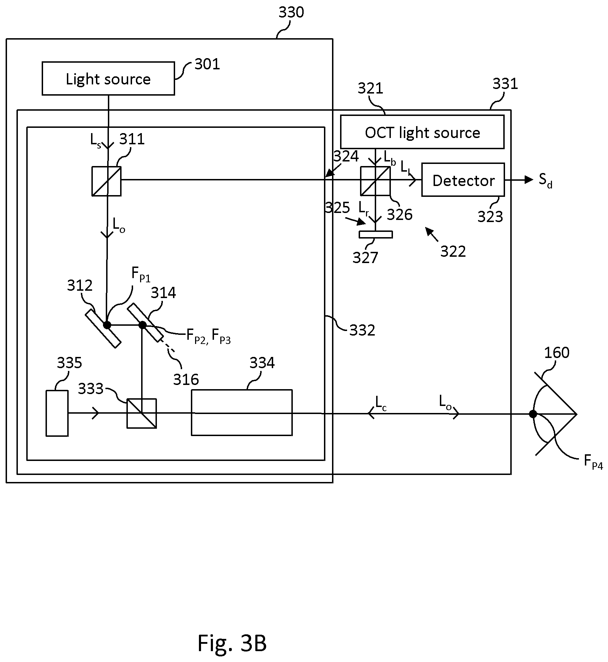

Figure 3B is a schematic illustration of an example optical system 330 and an example FD-OCT imaging system 331 of an alternative implementation, which share a lens-based scanning system 332 (where like reference numerals indicate elements that are the same as in Figure 3A). As shown in Figure 3B, instead of being guided by the curved mirrors 313 and 315, light is directed from the first scanning element 312 to the second scanning element 314 before being directed, via a beam splitter 333, through a lens barrel 334 comprising one or more lenses to the retina of the eye 160. The beam splitter 253 may take a similar form as the beam splitter 311 (e.g. it may be a beam splitter cube or a dichroic mirror). Further, the scanning system 331 may comprise a first lens relay and a second lens relay (which may leave equal focal lengths) between the first scanning element 312 and the second scanning element 314 (as the only intervening optics). A fixation target 335 may be provided as an example of the fixation target 110 of Figure 1. Light from the fixation target 335 may pass through the beam splitter 333, the lens relay 334 and then to the eye 160.

The first scanning element 312 and the second scanning element 314 may, as in the present example embodiment, each be a galvanometer optical scanner (a "H-galvo" and a "V-galvo", respectively). However, another type of scanning element could alternatively be used, such as a MEMS scanning mirror or a resonant scanning mirror, for example.

The detector 323 is arranged to detect the interference light Lₗ. That is, the detector 323 is arranged to receive the interference light Lₗ from the interferometer 322 and generate a detection signal S_{d} based on the received interference line of light Lₗ. The detector 323 generates the detection signal S_{d} by performing a photoelectric conversion of the interference light Lₗ that is incident on photodetector elements of the detector 323. The specific form of the detector 323 depends on the form in which the FD-OCT imaging system 320 is implemented. For example, where the FD-OCT imaging system 320 is implemented as an SD-OCT imaging system, the detector 322 comprises a spectrometer, which may have a diffraction grating, Fourier transform lend, and a detector array (or a line scan camera). Where the FD-OCT imaging system 320 is implemented as a SS-OCT imaging system, the light detector 120 may comprise a balanced photodetector set-up comprising two photodetectors (e.g. reverse-biased photodiodes), whose output photocurrents are subtracted from one another, with the subtracted current signal being converted into a voltage detection signal by a transimpedance amplifier. The detection signal S_{d} may, as in the present example embodiment, then be processed by OCT data processing hardware of the FD-OCT imaging system 320 to generate the OCT data 135. However, the functions of the OCT data processing hardware may alternatively be performed by the controller 140 (i.e. the detection signal S_{d} may be received and processed by the controller 140 to generate the OCT data 135).

Where the FD-OCT imaging system 320 is line-scan system rather than a point scan system, the OCT light source 321 generates a line of light, as described above. In such a case, the interferometer 322 is instead arranged to split the line of light, rather than the OCT beam L_{b}, into the sample OCT light as a sample OCT line of light. The scanning system 310 is arranged to perform a line-scan of the sample OCT line of light across the portion of the retina of the eye 160 and collect light L_{c} which has been scattered by the portion of the retina of the eye 160 during the line scan. Accordingly, the scanning system 310 may further comprise a lens arranged to focus the sample OCT line of light at the first focal point F_{P1} of the first scanning element 312. The sample OCT line of light is then reflected, in sequence, by the first scanning element 312, the first curved mirror 313, the second scanning element 314 and the second curved mirror 315, before being incident on the portion of the retina of the eye 160, and the light scattered from the portion of the retina of the eye 160 travels back to the detector 323 (i.e. as adapted for a line-scan system) via the scanning system 310, in the same manner described above for the point-scan implementation. The sample OCT line of light may thus be steered, in two dimensions, within the eye by rotating the first scanning element 312 and the second scanning element 314 such that a scan of eye 160 may be performed by the scanning system 310 as coordinated by the controller 140 or the dedicated scanning system controller. However, as a sample OCT line of light is incident on the portion of the retina, the scanning system 310 thus acquires at least one B-scan (e.g. successive B-scans forming a C-scan) of the retina of the eye 160 rather than acquiring at least one A-scan as in the point-scan implementation.

The light source 301 of the optical system 300 is arranged to generate light Lₛ as the optical stimulus. The light Lₛ may, as in the present example embodiment, be of one or more wavelengths in the visible spectrum of the human eye, although it may more generally be of any wavelength(s) for stimulating a physiological response of a retina of an eye 160. The light source 301 may be arranged to generate a plurality of lights of different wavelengths, each of which may be used as the optical stimulus as desired, (e.g. by use of broadband spectrum source, which may produce white light, filtered by at least one tuneable or removable spectral filter) although this may alternatively be achieved by the optical system 300 comprising additional light sources arranged to generate light of different wavelengths to that of the light Lₛ which may be the optical stimulus (these light sources may be combined to a single output of the light source 301 using fibre couplers, wavelength division multiplexing (WDM) fibres, beam splitters or dichroic mirrors).

In addition, the light source 301 may be controllable by the controller 140 to generate the light Lₛ at a desired light intensity.

The light source 301 may, as in the present example embodiment, comprise an illumination source (e.g. a light-emitting diode) and an illumination source aperture in a similar manner to as described above with the OCT light source 321. The light source 301 may comprise further components, such as one or more collimating lenses for collimating light from the light source, for example. The controller 140 controls the light source 301 of the optical system 300 to generate the light Lₛ as the optical stimulus, as described below, and may further control the light source 301 to vary the duration of the optical stimulus provided by the light source 301.

The beam splitter 311 may, as in the present example embodiment, be a cube beam splitter. However, it may instead be a dichroic mirror, for example.

The optical system 300 shares the scanning system 310 used by the FD-OCT imaging system 320 to perform the point-scan. This is achieved by using the beam splitter 311 to couple the optical path along which the light Lₛ travels with the optical path along which the sample OCT light Lₒ travels through the scanning system 310, although any other suitable arrangement for coupling the two optical paths may be used. The optical path along which the light Lₛ travels through the scanning system 310 when generated by the light source 301 may be coupled to the optical path along which the sample OCT light Lₒ travels through the scanning system 310 with the beam splitter 311 such that these optical paths run along a common axis, although the optical paths may alternatively run along axes offset by a predetermined amount (e.g. by adjusting the incident location of one of the optical paths on the beam splitter 311).

The scanning system 310 is thus further arranged to direct the light Lₛ to the retina of the eye 160. The light Lₛ enters the scanning system 110 via the beam splitter 311, and is then reflected, in sequence, by the first scanning element 312, the first curved mirror 313, the second scanning element 314 and the second curved mirror 315, before being applied to the retina of the eye 160.

In the same manner as with the sample OCT light Lₒ, the optical system 300 is controllable to vary a location on the retina of the eye 160 at which the light Lₛ is to be applied by the first scanning element 312 rotating around the first axis (not shown) to move the optical path of the scanning system 310 in the first direction, or in the direction opposite the first direction, across the retina of the eye 160, and by the second scanning element 314 rotating around the second axis 316 to move the optical path of the scanning system 310 in the second direction, or in the direction opposite the second direction, across the retina of the eye 160. By rotating the first scanning element 312 and the second scanning element 314, it is thus possible to steer, in two-dimensions, the optical path along which the light Lₛ travels such that the light Lₛ may be applied to any position on the retina of the eye 160. Accordingly, by controlling the timing and the duration of the light Lₛ generated by the light source 301, and the ranges and rates of rotation of the first scanning element 312 and/or the second scanning element 314, the controller 140 can cause any position on the retina of the eye 160 to be stimulated by the light lₛ for a required duration of time. This duration of time may be controlled by the controller 140 along with the intensity of the light Lₛ generated by the light source 301 so as to obtain a predetermined degree of bleaching of the retina of the eye 160. For example, the degree of bleaching obtained may be between 10% and 66%. Higher bleaching values may be preferable for studying the alpha wave in the ORG data 150 (i.e. the fast retina response).

By the optical system 300 sharing the scanning system 310 with the FD-OCT imaging system 320, the full field of view of the FD-OCT imaging system 320 may be accessed by the optical system 300 without any additional scanning hardware. This reduces the complexity of the FD-OCT apparatus 100 and may allow easier integration of the optical system 300 into complex sample arms, for example where the FD-OCT imaging system 320 is a UWF FD-OCT imaging system, as described above.

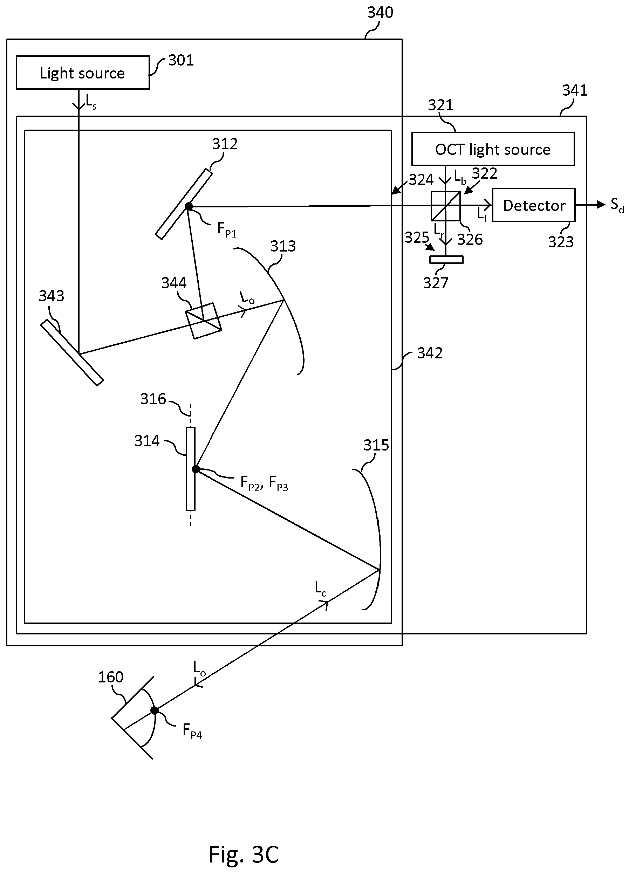

Figure 3C is a schematic illustration of an optical system 340 and an FD-OCT imaging system 341 which may, in accordance with an alternative implementation of the present example embodiment, form example implementations of the optical system 120 and the FD-OCT imaging system 130 in Figure 1 (with like reference numerals indicating the same elements as in Figure 3A). The optical system 340 and the FD-OCT imaging system 341 are the same as the optical system 300 and the FD-OCT imaging system 320 of Figure 3A, respectively, but differ from these components in Figure 3A by sharing the scanning system 342 rather than the scanning system 310. The scanning system 342 is the same as the scanning system 310, other than comprising a third scanning element 343 and a beam splitter 344. The third scanning element 343 may be implemented in the same form as the first scanning element 312 but is used by the scanning system 342 (instead of the first scanning element 312) to direct the light Lₛ from the light source 301 to the retina of the eye 160, and to vary the location on the retina of the eye 160 at which the light Lₛ is to be applied in the first direction and the direction opposing the first direction. The beam splitter 344 may be implemented in the same form as the beam splitter 311, and is located so as to direct the light Lₛ from the light source 301 to the first curved mirror 313, and direct the sample OCT scan Lₒ to the first curved mirror 313 (and the collected light L_{c} to the first scanning element 312). Although a fixation target is not illustrated in Figure 3C for clarity, one may be located in the scanning system 310, on at least one side of the first curved mirror 313, above and/or below the plane of the Figure, as described in US 11,253,146 B2. Light from such a fixation target may thus pass via the second scanning element 314 and then the second curved mirror 315 to the eye 160.

As the third scanning element 343 used by the scanning system 342 to direct the light Lₛ to the retina of the eye 160 is different to the first scanning element 312, it may be independently controlled by the controller 140. Accordingly, the optical path along which the light Lₛ travels through the scanning system 310 when generated by the light source 301 can be varied in the first direction (or in the direction opposite the first direction) independently of the optical path along which the sample OCT light Lₒ travels through the scanning system 310. Thus, the optical path along which the light Lₛ travels through the scanning system 310 is coupled to the optical path along which the sample OCT light Lₒ travels through the scanning system 310 in one dimension only, which adds an additional degree of freedom to the variation of the optical stimulus on the retina of the eye 160 as compared to the implementations of Figure 3A and 3B, although at the expense of increasing the complexity of the FD-OCT apparatus 100. For example, this degree of freedom may be advantageous for studying photoreceptor pathways within the retina by removing at least some of the coupling between the optical path of the light Lₛ and the optical path of the sample OCT beam Lₒ (e.g. to introduce a spatial offset between the centre of the first portion and the centre of the second portion of the retina).

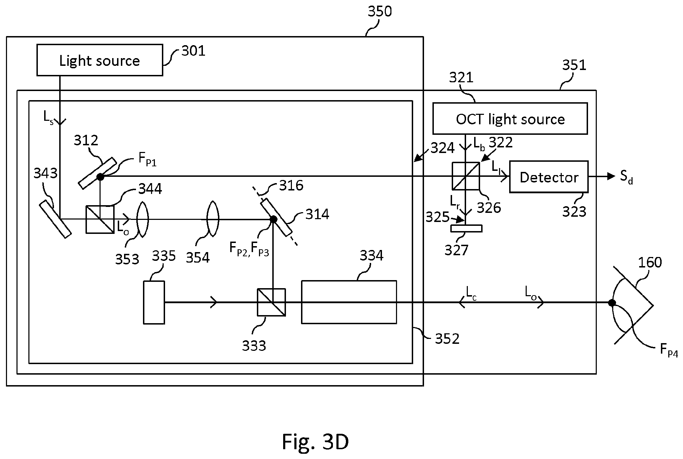

Although the example embodiment of Figure 3C includes the curved mirrors 313 and 315, these may be omitted as shown in Figure 3D, which is a schematic illustration of an example optical system 350 and an example FD-OCT imaging system 351 sharing a scanning system 352 which omits the curved mirrors 313 and 315 (whereby like reference numerals indicate elements that are the same as in Figures 3A to 3C). As shown in Figure 3D, light is directed from the first scanning element 312 and from the third scanning element 343 to the second scanning element 314 via a first lens relay 353 and a second lens relay 354 (which may have equal focal lengths) that are situated between the third scanning element 343 and the second scanning element 314. The first lens relay 353 and the second lens relay 354 are optional, and the only intervening optics between the first scanning element 312 and the second scanning element 314, and between the third scanning element 343 and the second scanning element 314, may be the beam splitter 344 in an alternative example embodiment. As illustrated in Figure 3D, the fixation target 335 may be provided in the scanning system 352 of Figure 3D in a similar way as in the scanning system 332 of Figure 3B.

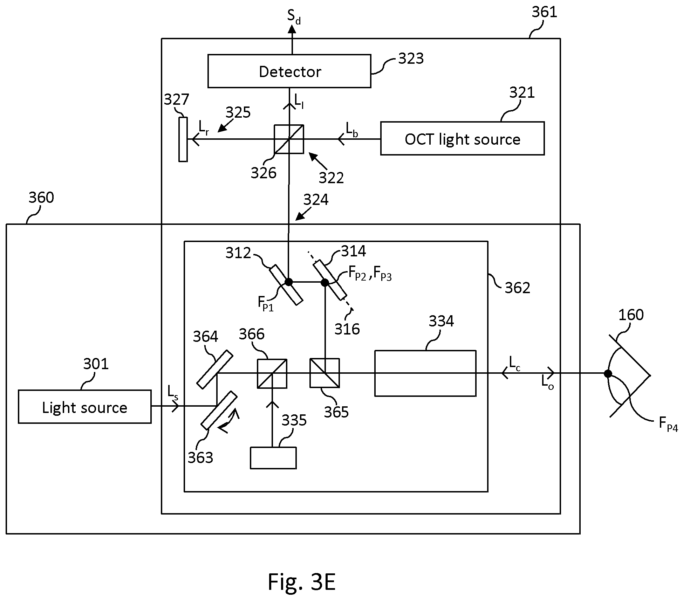

Figure 3E is a schematic illustration of an optical system 360 and an FD-OCT imaging system 361 constituting further alternative implementations of the optical system 120 and the FD-OCT imaging system 130 of the example embodiment. The optical system 360 differs from the optical system 300 of Figure 3A, and the FD-OCT imaging system 361 differs from the FD-OCT imaging system 320 of Figure 3A, only by sharing a scanning system 362 (rather than the scanning system 310). The scanning system 362 is similar to scanning system 332 of Figure 3B, in comprising one or more lenses in a lens barrel 334 instead of the curved mirrors 313 and 315 of Figure 3A, for example, but differs from scanning system 332 by further comprising a third scanning element 363, a fourth scanning element 364 and a beam splitter 365 instead of beam splitters 331 and 333 of Figure 3B. The third scanning element 363 may be implemented in the same form as the first scanning element 312, and the fourth scanning element 364 may be implemented in the same form as the second scanning element 313, although the third scanning element 363 and the fourth scanning element 364 are used by the scanning system 362 (instead of the first scanning element 312 and the second scanning element 313) to direct the light Lₛ to the retina of the eye 160 and to vary the location on the retina of the eye 160 at which the light Lₛ is to be applied in the first direction, the direction opposing the first direction, the second direction and the direction opposing the second direction. The scanning system 362 may further comprise a first relay lens and a second relay lens (not shown) between the first scanning element 312 and the second scanning element 314, and may comprise a third relay lens and a fourth relay lens (not shown) between the third scanning element 363 and the fourth scanning element 364. The beam splitter 365 may be implemented in the same form as the beam splitter 333, and may be arranged to direct light from the fourth scanning element 364 through the lens barrel 334 towards the eye 160.

The scanning system 362 may further comprise a beam splitter 366, and the fixation target 335 as described above in relation to Figure 3B. Light from the fixation target 335 may be directed by the beam splitter 366 through the beam splitter 365, the lens relay 334 and then to the eye 160.

The third scanning element 363 and the fourth scanning element 364 used by the scanning system 362 to direct the light Lₛ to the retina of the eye 160 are different to the first scanning element 312 and the second scanning element 314 that are used by the scanning system 362 to direct the sample OCT light Lₒ to the retina, and scanning elements 263 and 364 may be controlled by the controller 140 independently of scanning elements 312 and 314. Accordingly, the optical path, along which the light Lₛ travels through the scanning system 362 when generated by the light source 301 can be varied in the first direction (or in the direction opposite the first direction), and in the second direction (or in the direction opposite the second direction), independently of the optical path along which the sample OCT light Lₒ travels through the scanning system 362. Thus, the optical path along which the light Lₛ travels through the scanning system 362 is not coupled to the optical path along which the sample OCT light Lₒ travels through the scanning system 362, allowing the variation of the optical stimulus on the retina of the eye 160 to be independently controlled with two degrees of freedom, although at the expense of increasing the complexity of the FD-OCT apparatus 100 as compared to the implementations described above with reference to Figures 3A to 3D.

Although the scanning system 362 comprises a third scanning element 363 and a fourth scanning element 364, these may alternatively be replaced by a single two-dimensional scanner (e.g. a micro-electromechanical system (MEMS) scanner).

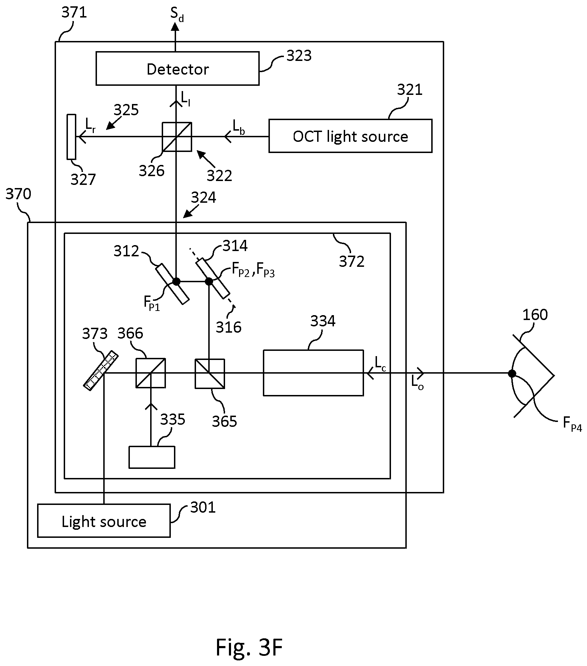

Figure 3F is a schematic illustration of an example optical system 370 and an example FD-OCT imaging system 371 that share a scanning system 372 which comprises a two-dimensional scanner 373. In Figure 3F, elements that are the same as in Figures 3A to 3E are labelled with like numerals. The scanning system 372 is the same as scanning system 362 other than by comprising the two-dimensional scanner 373 (in place of the third scanning element 363 and the fourth scanning element 364), which is used to direct the light Lₛ to the retina of the eye 160 and vary the location on the retina of the eye 160 at which the light Lₛ is to be applied in the first direction, the direction opposing the first direction, the second direction and the direction opposing the second direction.

The scanning system 372 may further comprise a beam splitter 376, and the fixation target 335 as described above in relation to Figure 3B. Light from the fixation target 335 may be directed by the beam splitter 376 through the beam splitter 375, the lens relay 334 and then to the eye 160.

As a further alternative implementation of the example embodiment, the optical system 120 may comprise a spatial light modulator, which is controllable by the controller 140 to vary a location on the retina at which the light L_{S} is to be applied. For example, the spatial light modulator may comprise a projector having the light source 301, a collimator and a dynamic amplitude mask (e.g. in the form of a digital micromirror device (DMD), which is arranged to be illuminated by the collimated light and is controllable by the controller 140 to allow the collimated light L_{S} generated by the light source 301 to pass via only a predefined portion of the dynamic amplitude mask so as to vary the location on the retina where light from the light source 301 is incident. Where the dynamic amplitude mask is provided in the form of a DMD, the DMD may comprise an array of rotatable micromirrors, which are individually controllable by the controller 140 to switch from being in one of a first and a second, different orientation to the other of the first and second orientation. More specifically, the DMD may be configured to set each micromirror in the DMD either to a first orientation, to reflect light from the light source 301 towards the eye 160, or to a second orientation such as to reflect incident light away from the eye 160 and thus prevent light from the light source 301 from reaching the eye 160. In this manner, the use of DMD allows binary amplitude modulation of the light received at each micromirror position on the DMD. It should be noted, however, that the functionality of the dynamic amplitude mask may be provided by any suitable type of spatial light modulator other than a DMD, such an array of liquid crystal cells, or an analog micromirror array, for example. For example, in an alternative example embodiment, where the dynamic amplitude mask comprises an array of liquid crystal cells, the liquid crystal in each liquid crystal cell of the array may be individually switchable between a first liquid crystal phase and a second liquid crystal phase. A liquid crystal cell that is in the first liquid crystal phase transmits light L_{S} incident thereon towards the eye 160. A liquid crystal cell that is in the second liquid crystal phase, on the other hand, blocks incident light L_{S}, preventing it from being transmitted to the eye 160. Furthermore, the unmasked portion of the dynamic amplitude mask may consist of liquid crystal cells of the array having liquid crystals in the first phase, while the masked portion of the dynamic amplitude mask may comprise liquid crystal cells of the array having liquid crystals in the second phase. The spatial light modulator may, as a further example, comprise an array of light sources (e.g. LEDs) that are arranged to provide corresponding collimated, spatially separated beams of light, and which are controllable by the controller 140 so as to provide control of the location(s) on the retina at which the optical stimulus is applied.

In addition, the spatial light modulator may be used to vary the location on the retina of the eye 160 at which the light L_{S} is to be applied when the FD-OCT imaging system 130 is a full-field FD-OCT imaging system, by way of example.

It should be noted that, although the above-described arrangements for varying the location on the retina at which the optical stimulus is to be applied are described within the context of an FD-OCT imaging system 130 which is arranged to deliver a single sample OCT beam Lₒ to the retina of the eye 160, the present disclosure is not so limited, and these arrangements for varying the location on the retina at which the optical stimulus is to be applied may also be used within multi-beam FD-OCT imaging systems that are arranged to simultaneously deliver multiple sample OCT beams to the retina.

The above-described arrangements may allow the location on the retina at which the optical stimulus is applied to be set with greater accuracy than in a case where this location is set by eye steering, e.g. by the fixation target 110 being moved laterally relative to the eye 160 (i.e. in the field of view of the eye 160) to vary the location on the retina at which the optical stimulus is applied. In addition, keeping the fixation target 110 at a fixed location during the acquisition of the ORG data 150 may improve patient comfort and improve the ease of use of the system. Further, some of the above-described arrangements for varying the location on the retina at which the optical stimulus is to be applied make use of the existing optics the OCT sample arm, thus enabling easier integration of the optical system 120 into existing OCT systems.

Returning to Figure 1, the controller 140 is arranged to acquire at least one indicator 141 of a target location on the retina at which the optical stimulus is to be applied by the optical system 120, and to use the acquired indicator 141 to control the optical system 120, while a position of the fixation target 110 relative to the eye 160 remains fixed, to apply the optical stimulus to a first portion of the retina which is at the target location. The controller 140 is further arranged to control the FD-OCT imaging system 130 to acquire OCT data 135 of a second portion of the retina, wherein at least a part of the second portion of the retina is disposed in relation to the first portion so as to be stimulated by the applied optical stimulus during acquisition of at least some of the OCT data 135. The controller 140 is further arranged to generate the ORG data 150 based on the acquired OCT data 135, as described in more detail below.

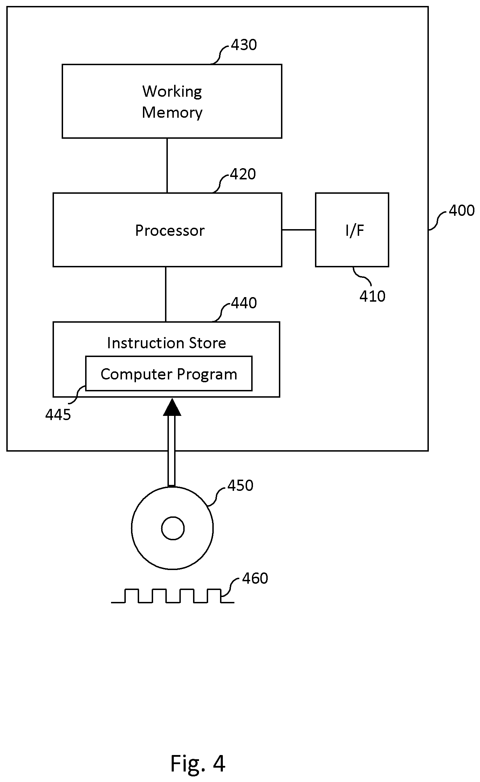

The controller 140 (and the dedicated scanning system controller, where provided) may be provided in any suitable form, for example as a programmable signal processing hardware 400 of the kind illustrated schematically in Figure 4. The programmable signal processing apparatus 400 comprises a communication interface (I/F) 410, for receiving the OCT data 135 (or the detection signal S_{d}) from the FD-OCT imaging system 130 and, in some cases, receiving the indicator 141, outputting control signals to the FD-OCT imaging system 130 and outputting the ORG data 150 and/or a graphical representation thereof to the map display device 142 (such as a computer screen or the like) to be displayed thereby. The signal processing hardware 400 further comprises a processor 420 (e.g. a Central Processing Unit, CPU, and/or a Graphics Processing Unit, GPU), a working memory 430 (e.g. a random-access memory) and an instruction store 440 storing a computer program 445 comprising the computer-readable instructions which, when executed by the processor 420, cause the processor 420 to perform various functions including those of the controller 140 described herein. The working memory 430 stores information used by the processor 420 during execution of the computer program 445. The instruction store 440 may comprise a ROM (e.g. in the form of an electrically erasable programmable read-only memory (EEPROM) or flash memory) which is pre-loaded with the computer-readable instructions. Alternatively, the instruction store 440 may comprise a RAM or similar type of memory, and the computer-readable instructions of the computer program 445 can be input thereto from a computer program product, such as a non-transitory, computer-readable storage medium 450 in the form of a CD-ROM, DVDROM, etc. or a computer-readable signal 460 carrying the computer-readable instructions. In any case, the computer program 445, when executed by the processor 420, causes the processor 420 to perform the functions of the controller 140 as described herein. In other words, the controller 140 of the example embodiment may comprise a computer processor 420 and a memory 440 storing computer-readable instructions which, when executed by the computer processor 420, cause the computer processor 420 to acquire the indicator 141 of the target location on the retina at which the optical stimulus is to be applied by the optical system 120, to use the acquired indicator 141 to control the optical system 120, while the position of the fixation target 110 relative to the eye 160 remains fixed, to apply the optical stimulus to the first portion of the retina which is at the target location, to control the FD-OCT imaging system 130 to acquire the OCT data 135 of the second portion of the retina, wherein the at least a part of the second portion of the retina is disposed in relation to the first portion so as to be stimulated by the applied optical stimulus during acquisition of the at least some of the OCT data 135, and to generate the ORG data 150 based on the acquired OCT data 135, as described herein.

It should be noted, however, that the controller 140 may alternatively be implemented in non-programmable hardware, such as an ASIC, an FPGA or other integrated circuit dedicated to performing the functions of the controller 140 described above, or a combination of such non-programmable hardware and programmable hardware as described above with reference to Figure 4. Further, while the controller 140 is described in relation to a single programmable signal processing hardware 400, the controller 140 is not so limited and its functions may be split between a plurality of programmable signal processing hardware of the kind shown in Figure 4, or parts thereof, and/or the aforementioned non-programmable hardware. For example, the controller 140 may be implemented by programmable signal processing hardware of the kind shown in Figure 4 which is arranged to acquire the indicator 141 of the target location on the retina at which the optical stimulus is to be applied by the optical system 120, to use the acquired indicator to control the optical system 120, while the position of the fixation target 110 relative to the eye 160 remains fixed, to apply the optical stimulus to the first portion of the retina which is at the target location and to control the FD-OCT imaging system 130 to acquire the OCT data 135 of the second portion of the retina, wherein the at least a part of the second portion of the retina is disposed in relation to the first portion so as to be stimulated by the applied optical stimulus during acquisition of the at least some of the OCT data 135, as described herein. In this case, a separate processor (e.g. one similar to processor 420 in Figure 4) may be arranged to receive the acquired OCT data 135 from the controller 140 and generate the ORG data 150 based on the acquired OCT data 135 (and output the ORG data 150 to, for example, the map display device 142), as described herein.

The map display device 142 may, as in the present example embodiment, be arranged to receive data from the controller 140 for displaying the user (e.g. a clinician), for example, and the communication interface 410 may be arranged to receive inputs from the user of the FD-OCT apparatus 100 such as those as required by the controller 140. For example, the map display device 142 may receive and display the ORG data 150, or a graphical representation thereof, or may be controlled by the controller 140 to display the maps described herein below. The inputs from the user of the FD-OCT apparatus 100 may include, for example, the indicator 141, or an indication of the second portion of the retina as will be later described, which the map display device 142 may transmit to the controller 140. The map display device 142 may be an LCD screen, for example, and may comprise programmable signal processing hardware 400 of the kind illustrated schematically in Figure 4, which may be used to generate the graphical representation of the ORG data 150, process inputs provided by the user (e.g. by processing touch inputs from the user where the map display device 142 is a touchscreen) and interface with the controller 140. However, the map display device 142 is optional and may be omitted. For example, the aforementioned functions of the map display device 142 may be provided by an external computer or, in some cases, there may be no need to display the ORG data 150 and it may instead be stored on an external server or within the memory of the controller 140 for later retrieval.

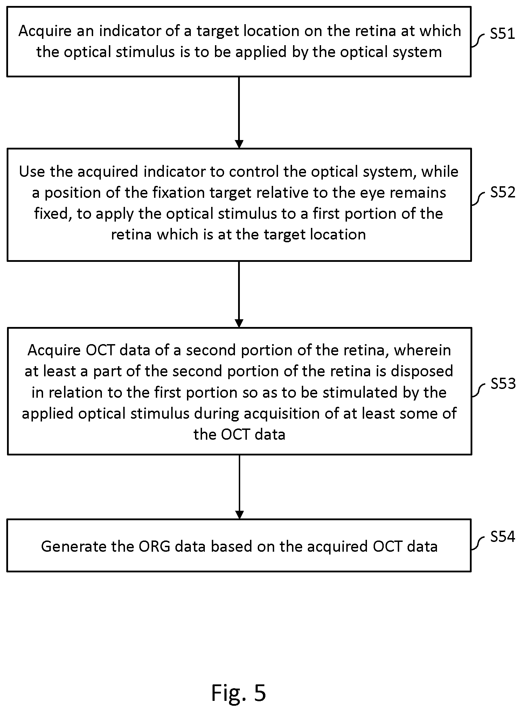

Figure 5 is a flow diagram illustrating a process by which the controller 140 may, as in the present example embodiment, generate the ORG data 150. This process defines an ORG capture session, which may be initiated by a period of dark adaptation of the retina of the eye 160. For example, the FD-OCT apparatus 100 may be arranged to prevent light (both internal to and external to the FD-OCT apparatus 100) from being incident on the retina of the eye 160 for a predetermined duration of time (e.g. approximately 5 minutes) or the patient may, more simply, be instructed to close their eyes or wait in a dark room for the predetermined duration of time.

In process S51 of Figure 5, the controller 140 acquires an indicator 141 of a target location on the retina of the eye 160 at which the optical stimulus is to be applied by the optical system 120. For example, the indicator 141 may be indicative of a location of a point on the retina of the eye 160 at which the optical stimulus applied by the optical system 120 is to be centred on. The indicator 141 of the target location may be generated automatically, based on a predetermined location on the retina defined in relation to one or more anatomical landmarks (e.g. the optic disk, macula, etc.) and taking into account the gaze direction 161 of the eye 160). The indicator 141 of the target location may alternatively be input to the controller 140 by the user selecting the target location on an image of the retina (e.g. an en-face OCT image previously acquired by the FD-OCT imaging system 130 or a reflectance image of the retina previously acquired using a scanning laser ophthalmoscope (SLO), which may be included in the FD-OCT imaging apparatus 100 and share the scanning system 310 with the FD-OCT imaging system 130, for example) displayed on the map display device 142 (or an external display, such as a computer screen or the like), using a mouse and/or keyboard, or via one or more touch interactions in case the display is a touchscreen, for example.

In process S52 of Figure 5, the controller 140 uses the acquired indicator 141 to control the optical system 120, while a position of the fixation target 110 relative to the eye 160 remains fixed and the eye 160 fixates on the fixation target 110 (i.e. while a location of the fixation target in a field of view (FoV) of the eye 160 remains fixed, where the FoV covers everything the eye 160 is able to see as it pivots in its eye socket while the head remains in a fixed orientation), to apply the optical stimulus to a first portion of the retina of the eye 160 which is at the target location. The controller 140 may use the acquired indicator 141 to control the optical system 120 to vary, while a position of the fixation target 110 relative to the eye 160 remains fixed, the location on the retina at which the optical stimulus is to be applied to the target location, and to apply the optical stimulus to the first portion of the retina which is at the target location. The controller 140 may be configured to transform the location on the retina indicated by the indicator 141 into a corresponding set of one or more control parameters for steering the optical system 120 to apply the optical stimulus at substantially the same location on the retina as that indicated by the indicator 141. This can be done in one of a number of different ways. For example, the controller 140 may use a mapping between the locations on the retina and corresponding values of the control parameters, which may be provided in the form of a look-up table (LUT) or a function defined by a set of parameters, for example. The mapping may be determined by calibration, using techniques known to those skilled in the art.

In the present example embodiment, as shown in Figure 3A, the controller 140 controls the scanning system 310 to rotate the first scanning element 312 and the second scanning element 314 such that an optical path, along which the light Lₛ travels through the scanning system 310, is incident upon the target location, and the controller 140 controls the light source 301 to generate the light Lₛ so as to propagate along the optical path either after the aforementioned rotation or, as the case may be, during the aforementioned rotation. The first portion of the retina may, as in the present example embodiment, be smaller than the field of view of the FD-OCT imaging system 130 (e.g. less than 0.1, 1 or 10% of the areas covered by the field of view of the FD-OCT imaging system 130). Examples of the first portion of the retina are shown and described in more detail below.

In process S53 of Figure 5, the controller 140 controls the FD-OCT imaging system 130 to acquire the OCT data 135 of a second portion of the retina of the eye 160. The controller 140 may acquire a second indicator indicative of the location of the second portion of the retina of the eye 160 by the user selecting the location of the second portion of the retina on the map display device 142 in a similar scanner to as described for the indicator 141 in process S51. Alternatively, the second indicator may be generated by the controller 140 based on at least one of the indicator 141 or the first portion of the retina of the eye 160, as described in more detail below. The second portion may have a pre-determined, fixed positional relationship to the first portion, for example.

The acquisition of the OCT data 135 may, as in the present example embodiment, be of a temporal sequence of OCT images of the second portion of the retina of the eye 160 within a first period of time (i.e. a period of time between the time of acquisition of the first OCT image in the temporal sequence of OCT images and the time of acquisition of the last OCT image in the temporal sequence of OCT images). Accordingly, in the present example embodiment as shown in Figure 3A, the controller 140 controls the rotation of one or both of the first scanning element 312 and the second scanning element 314 to perform a point scan of the second portion of the retina of the eye 160 in accordance with the predetermined scan pattern to acquire the temporal sequence of OCT images. The controller 140 may alternatively instruct the dedicated scanning system controller (where provided) to control one or both of scanning elements 313 and 314.

At least a part of the second portion of the retina of the eye 160 is disposed in relation to the first portion so as to be stimulated (or at least partially illuminated) by the applied optical stimulus during acquisition of at least some of the OCT data 135. That is, during the performance of process S53 for a second portion of the retina, the controller 140 previously performs process S52 and uses the acquired indicator 141 to control the optical system 120 to apply the optical stimulus to the corresponding first portion of the retina of the eye 160 at a first time which falls within the first period of time so that at least some of the OCT images in the temporal sequence of OCT images are acquired after the first time, and at least a part of the second portion of the retina of the eye 160 in each of these OCT images is stimulated (or at least partially illuminated) by the applied optical stimulus. The second portion of the retina may at least partially overlap the corresponding first portion of the retina, or it may alternatively be disposed away from but sufficiently close to the corresponding first portion for the light incident on the first portion to scatter into at least some of the second portion or otherwise cause at least a part of the second portion of the retina to be provided with an optical stimulus for stimulating the region of the retina therein. Examples of the location of second portions of the retina of the eye 160 relative to corresponding first portions of the retina of the eye 160 are described in more detail below.

In a variant of the example embodiment, the controller 140 may first acquire an indication of the location of the second portion of the retina, as described above, at which the OCT data 135 is to be acquired, and may subsequently generate the indicator 141 such that the first portion of the retina, which is at the target location, stimulates at least a part of the second portion of the retina (i.e. such that the ORG data 150 may be generated at the location of the second portion of the retina). For example, the location of first portion may be selected to lie along a predetermined scan pattern which is used to acquire the OCT data 135. Accordingly, the same rotation of one or both of the first scanning element 312 and the second scanning element 314 in Figure 3A or 3B, or the second scanning element 314 in Figure 3C or 3D, for example, may be used to both acquire the OCT data 135 and apply the optical stimulus to the first portion of the retina. This may allow the optical stimulus to be applied to the eye 160 within a smaller time window than may be possible where the controller 140 needs to coordinate differing rotations of the scanning elements in process S52 and S53, which may be important when the temporal sequence of OCT images acquired in process S53 are closely spaced in time (as may be desired to, for example, reduce motion artefacts in the OCT images). Note that this advantage can similarly be achieved where the location of the first portion does not lie along the predetermined scan pattern on the retina but is displaced from the scan pattern by use of an arrangement as described above with reference to Figures 3C to 3F (although at the expense of increasing the system complexity).

Referring again to Figure 5, in process S54, the controller 140 generates the ORG data 150 based on the acquired OCT data 135. The controller 140 may, as in the present example embodiment, generate the ORG data 150 based on phase information in the acquired OCT data 135. The ORG data 150 generated by the controller 140 may be displayed on the map display device 142 to the user or output to an external computer or server for analysis, for example.

The controller 140 may generate the ORG data 150 based on the acquired OCT data 135 using any of the techniques known to those skilled in the art, for example, the velocity-based ORG technique described in Kari V. Vienola, et al., "Velocity-based optoretinography for clinical applications," Optica 9, 1100-1108 (2022), the content of which is herein incorporated by reference in its entirety. In brief, this velocity-based ORG technique generates ORG data based on acquired OCT data (which, in this case, comprises a temporal sequence of B-scans) by firstly flattening each of the B-scans such that the photoreceptor inner and outer segment (IS/OS) and cone outer segment (COST) reflections lie at the same height for each A-scan in each respective B-scans. Then, a moving (e.g. 10 ms) time window is used to select a group of (e.g. five) sequential B-scans with motion corrected relative to the first B-scan in the series. The phase data cube of each complex data cube for each spatial coordinate pair in the volume is then unwrapped in the temporal dimension to minimise the magnitude of the difference in phase between data cubes of consecutive phase B-scans. After unwrapping, a rate of phase change is calculated for each coordinate pair by using a least-squares linear fit with respect to time to calculate the instantaneous velocity for each spatial location. These instantaneous velocities and the B-scan amplitude, if desired, are averaged in the lateral dimension to give instantaneous, depth-dependent measures of velocity and backscattering, respectively. By shifting the (10 ms) time window a time series of depth profiles is constructed, separately for velocity and reflectively. Both of these may be visualised in time-depth coordinates, as M-scans. The velocities of the IS/OS and COST layers are subsequently extracted and the difference between them is the rate of the contraction/elongation of the OS in the region as a function of time, which may form the ORG data 150 (or, alternatively, the OS length response may be the ORG data 135) . However, these techniques may be applied to other retinal layers such as, for example, the rod photoreceptors by extracting the velocities of the IS/OS and ROST layers.

Alternatively, the magnitude of the so-called "alpha wave" (i.e. the fast retinal response, which is typically on a timescale of a few milliseconds) in the data of the rate of contraction/elongation of the outer segment (OS) as a function of time may be determined and saved as (or as a part of) the ORG data 150. Additionally or alternatively, the magnitude of the so-called "beta wave" (i.e. the slow response, which is typically on a timescale of a few seconds) may be determined and saved as (or as a part of) the ORG data 150. As a further alternative, the ORG data 150 may comprise a value (e.g. on a predefined scale, for example a scale of 1 to 10) which is indicative of a quality of a retinal response at the location of the second portion of the retina which is generated by comparing the retinal response indicated by the acquired ORG data with the response of a healthy retina. The healthy retinal response may be obtained from ORG data at a location on the retina of the eye 160 which is assessed by a clinician to be healthy or may be obtained from ORG data of a sample healthy eye, for example.

The controller 140 may generate the ORG data 150 based on the acquired OCT data 135 using intensity-based ORG techniques such as, for example, the OCT brightness change and OCT band analysis techniques described in Kim T-H, Ma G, Son T and Yao X, "Functional Optical Coherence Tomography for Intrinsic Signal Optoretinography: Recent Developments and Deployment Challenges", Front. Med. 9:864824, (2022), the contents of which are incorporated by reference herein in their entirety. OCT brightness change techniques may be used to detect local variations in pixel intensity value caused by the light stimulus on the retina. The data processing technique used in OCT brightness change analysis techniques may comprise registering raw OCT B-scans to account for eye movements, normalizing the pixel intensities based on the inner retinal intensity to limit the effect of pupillary response, identifying "active" intrinsic optical signal (IOS) pixels (i.e. pixels exhibiting a significant change in intensity after the light stimulus, which can be positive where intensity increased or negative where intensity decreased) and quantifying the number of these active IOS pixels for analysis. OCT band analysis techniques may include, for example, deconvolution methods for band analysis (e.g. of the hyper- and hypo-reflective bands in the retina). Where intensity-based ORG techniques are used, the OCT data may be acquired by OCT imaging systems which are not phase stable.

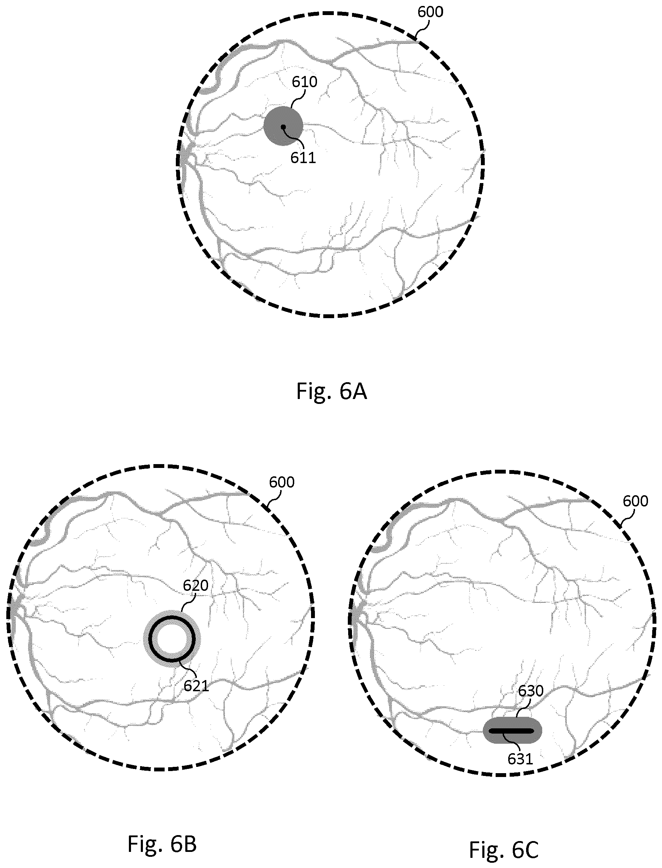

Figures 6A, 6B and 6C are schematic illustrations of example first portions of the retina of the eye 160 and example corresponding second portions of the retina of the eye 160 in processes S52 and S53 described above, which are respectively illuminated by the optical system 300 and imaged by the FD-OCT imaging system 320 according to the example embodiment. These figures show a region 600 of the retina of eye 160 which corresponds to the field of view of the FD-OCT imaging system 130. That is, the second portion of the eye 160 (at which the OCT data 135 is acquired) can be at any location within the region 600 to which the scanning system 300 can direct the sample OCT light Lₒ. As the optical system 300 shares the scanning system 310 with the FD-OCT imaging system 320, the first portion of the eye 160 can also be at any location within the region 600 to which the scanning system 300 can direct the light Lₛ. Note that the light Lₛ may be generated by the light source 301 as a light beam which may be of differing diameters, which may result in the differing sizes (widths) of the respective first portions as shown in each of Figures 6A, 6B and 6C. Different beam sizes (e.g. diameters) for the light lₛ may be achieved using differing illumination source apertures, for example.

In particular, where the optical system 300 shares the scanning system 310 with the FD-OCT imaging system 320, the radius of the light beam (as the light Lₛ generated by the light source 301) may be set such that the light beam illuminates the whole of the second portion of the eye 160 while the light beam is at each scan location within the second portion of the eye 160. For example, where the second portion is a straight portion corresponding to a straight B-scan, the radius of the light beam may be set so as to illuminate the whole straight portion when the light beam is at the first scan location (corresponding to the first A-scan of the straight B-scan) and at each subsequent scan location. This arrangement prevents the 'flickering' which may be perceived by a part of the retina due to the alternate illumination and lack thereof of the part of the retina by the light beam as it moves between scan locations, which may improve the quality of the generated ORG data.

Figure 6A shows a circular first portion 610 as an example of the first portion of the retina in process S52, and a second portion 611, corresponding to an A-scan, as an example of the second portion of the retina in process S53. The controller 140 begins process S53 of controlling the FD-OCT imaging system 320 to acquire the OCT data 135, which is a temporal sequence of the A-scans of the second portion 611, by rotating the first scanning element 312 and the second scanning element 314 in the scanning system 310 to a first pair of respective angular positions such that each of the A-scans is acquired at the location of the second portion 611. At the aforementioned first time, the controller 140 performs process S52 of using the acquired indicator 141 to control the optical system 300 to apply the optical stimulus to the circular first portion 610 via the generation of the light Lₛ by the light source 301 (the diameter of the circular first portion being the diameter of the light Lₛ incident upon the retina). As the circular first portion 610 is concentric with the second portion 611, the first scanning element 312 and the second scanning element 314 remain at the first pair of respective angular positions during the acquisition of the ORG data 150, thus simplifying this process.

Figure 6B shows a hollow circular first portion 620 as an example of the first portion of the retina in process 552, and a second portion 621, corresponding to a circular B-scan, as an example of the second portion of the retina in process S53 of Figure 5. In this case, the controller 140 begins process S53 of controlling the FD-OCT imaging system 320 to acquire the OCT data 135, which is a temporal sequence of the circular B-scans of the second portion 621, by rotating the first scanning element 312 and the second scanning element 314 in the scanning system 310 to perform a two-dimensional point scan using a circular scan pattern such that each of the circular B-scans is of the second portion 621. At the aforementioned first time, the controller 140 performs process S52 of using the acquired indicator 141 to control the optical system 300 to apply the optical stimulus to the hollow circular first portion 620 by rotating the first scanning element 312 and the second scanning element 314 in the scanning system 310 while the light Lₛ is being generated by the light source 301 (the thickness of the hollow circular first portion 620 being the diameter of the light Lₛ incident upon the retina). As the hollow circular first portion 620 and the second portion 621 following the same scan pattern, the same rotation of the first scanning element 312 and the second scanning element 314 (coordinated by the controller 140) can be used to provide the optical stimulus and acquire the OCT data 135, which simplifies operation of the FD-OCT apparatus 100.

Figure 6C shows a straight first portion 630 as an example of the first portion of the retina in process S52, and a straight second portion 631, corresponding to a straight B-scan, as an example of the second portion of the retina in process S53 of Figure 5. The controller 140 performs processes S51 to S53 of Figure 5 in a similar manner to as described above in Figure 6B (the thickness of the straight first portion 630 being the diameter of the light Lₛ incident upon the retina), except that the predetermined scan pattern is a unidirectional scan along a single scan axis (for the single straight B-scan). The straight first portion 630 and the straight second portion 631 run along a common straight line such that the same rotation of the first scanning element 312 and/or the second scanning element 314 (controlled by the controller 140) can be used to provide the optical stimulus and acquire the OCT data 135, which simplifies operation of the FD-OCT apparatus 100.

Although the respective first portions and the respective second portions in Figures 6A, 6B and 6C have been described as concentric, or running along a common line, this is not necessarily the case, and any other kind of scan pattern may be used. Further, although the circular first portion 610, the hollow circular first portion 620 and the straight first portion 630 have been described as having respective diameters or thicknesses corresponding to the diameter of the light Lₛ incident upon the retina, the diameter of the light Lₛ incident upon the retina may alternatively be smaller than the intended width of the first portion, and the controller 140 may coordinate the rotation of the first scanning element 312 and the second scanning element 314 to progressively illuminate the whole of each first portion.

Further, although the respective first portions are shown to be larger than the respective second portions in Figures 6A, 6B and 6C, this need not be the case. For example, an illumination source aperture of the light source 301 may be selected to provide light Lₛ of a diameter smaller than that of the sample OCT light Lₒ, or the second portion may extend outside of the first portion of the eye 160 by the controller correspondingly controlling the rotation of one of or both the first scanning element 312 and the second scanning element 314.

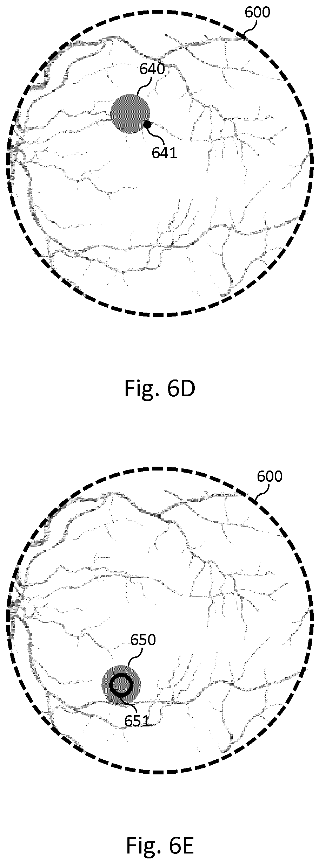

Figures 6D and 6E are schematic illustrations of example respective first portions of the retina of the eye 160 and corresponding example second portions of the retina of the eye 160 within the region 600 in processes S52 and S53 described above, which are respectively illuminated by the optical system 360 and imaged by the FD-OCT imaging system 361 of Figure 3E.

Figure 6D shows a circular first portion 640 as an example of the first portion of the retina in process S52, and a second portion 641, corresponding to an A-scan, as an example of the second portion of the retina in process S53 of Figure 5. As shown in Figure 6D, the second portion 641 is not concentric with the circular first portion 640, and this is enabled by the independent control of the location of the optical stimulus. In contrast to Figures 6A to 6C, the optical system 360 may apply the optical stimulus independently of where the sample OCT beam Lₒ is directed onto the retina, such that the optical stimulus can be applied to any required portion of the retina without interrupting the acquisition of the temporal sequence of OCT images.

Figure 6E shows a circular first portion 650 as an example of the first portion of the retina in process S52 of Figure 5, and a second portion 651, corresponding to a circular B-scan (acquired using a circular scan as the predetermined scan pattern), as an example of the second portion of the retina in process S53 of Figure 5. These geometries can likewise be achieved by the independent control of where the optical stimulus is applied to the retina and where the OCT data are acquired from the retina.

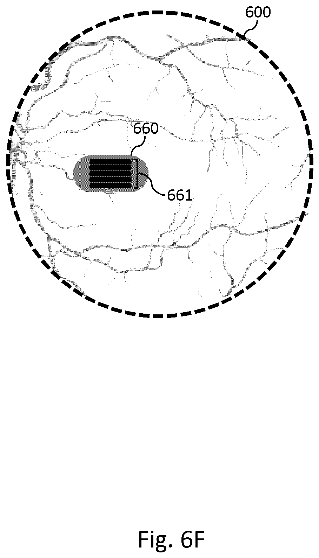

Figure 6F is a schematic illustration of a straight first portion 660 as an example of the first portion of the retina in process S52 of Figure 5, and a second portion 661, corresponding to a plurality of parallel straight B-scans forming a C-scan (acquired using a raster scan as the predetermined scan pattern), as an example of the second portion of the retina in process S53 of Figure 5, where the controller 140 controls the optical system 340 to provide the optical stimulus, and FD-OCT imaging system 341 to acquire the OCT data 135. In this case, the optical stimulus is controllable independently from the sample OCT beam Lₒ in the first direction, and the direction opposite to the first direction, which is perpendicular to the straight B-scans. This allows the optical stimulus to be applied to the straight first portion 630 without affecting the movement of the sample OCT beam Lₒ in the second direction between the intended location of each of the plurality of parallel straight B-scans. Note that similar C-scans may be obtained both by using the optical system 360 and FD-OCT imaging system 361, at the expense of increased system complexity, and using the optical system 300 and FD-OCT imaging system 320 at the expense of interrupting the movement of the sample OCT beam Lₒ in the second direction between the intended location of each of the plurality of parallel straight B-scans.

Rather than acquiring a single indicator 141 of a single target location and corresponding OCT data 135 acquired for that target location, the controller 140 may alternatively acquire a plurality of indicators each indicative of a respective target location on the retina, and process respective OCT data of respective second portions of the retina corresponding to respective first portions of the retina at each of the target locations to generate respective ORG data during the ORG capture session.

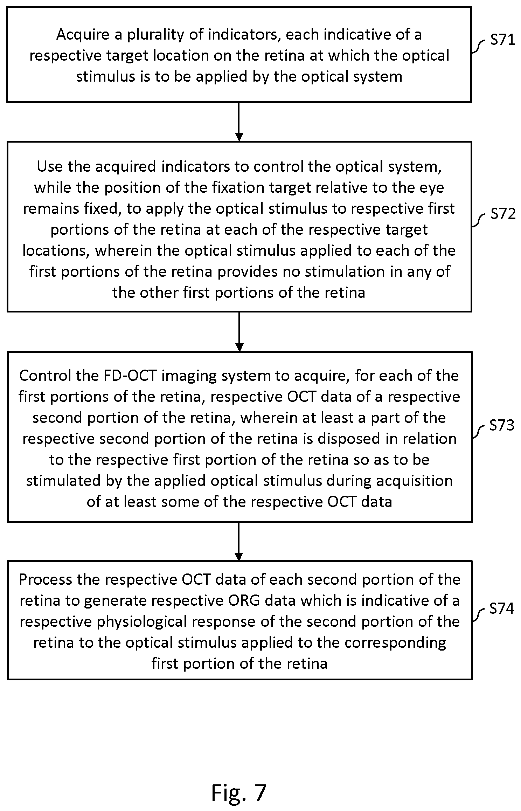

Figure 7 is a flow diagram showing a process by which the controller 140 may acquire and process respective OCT data of each second portion of the retina to generate respective ORG data which is indicative of a respective physiological response of the second portion of the retina to the optical stimulus applied to the corresponding first portion of the retina. The process shown in Figure 7 may be preceded by a period of dark adaptation of the retina of the eye 160, to improve the accuracy and reliability of the ORG data 150, in the same manner as described above with reference to Figure 5.

In process S71 of Figure 7, the controller 140 acquires a plurality of indicators, each indicative of a respective target location on the retina of the eye 160 at which the optical stimulus is to be applied by the optical system 120. Each of the plurality of indicators is of the same form as the indicator 141 described above, and the plurality of indicators may be acquired by the controller 140 in the same manner as described for the indicator 141.

In process S72 of Figure 7, the controller 140 uses the acquired indicators to control the optical system 120, while the position of the fixation target 110 relative to the eye 160 remains fixed, to apply the optical stimulus to respective separate first portions of the retina of the eye 160 at each of the target locations. The controller 140 controls the optical system 120 using each of the acquired indicators, in turn, in the same manner as described above (with reference to Figure 5) for the indicator 141. The optical stimulus applied to each of the first portions of the retina of the eye 160 provides no stimulation in any of the other first portions of the retina of the eye 160. That is, each of the plurality of indicators is of a respective target location on the retina which is different to the target locations indicated by the other indicators, so that each of the first portions of the retina do not overlap on the retina with any of the other first portions. Each of the first portions of the retina is smaller than the field of view of the FD-OCT imaging system 130 (e.g. less than 0.1, 1 or 10% of the area covered by the field of view of the FD-OCT imaging system 130). Examples of the first portions of the retina are described in more detail below.

In process S73 of Figure 7, the controller 140 controls the FD-OCT imaging system 130 to acquire, for each of the first portions of the retina of the eye 160, respective OCT data of a respective second portion of the retina of the eye 160. The controller 140 acquires second indicators that indicate the respective locations on the retina of the second portions of the retina, and controls the FD-OCT imaging system 130 to acquire respective OCT data of the second portions of the retina of the eye 160 in the same manner as described above with reference to process S53 of Figure 5. At least a part of each respective second portion of the retina is disposed in relation to its respective first portion of the retina so as to be stimulated by the optical stimulus applied to the first portion during acquisition of at least some of the respective OCT data, in a similar manner as described with reference to process S53 of Figure 5 for the first and second portion of the retina. Each second portion of the retina may be separate (spaced apart) from every other second portion of the retina. However, at least some of the second portions may, in some cases, at least partially overlap which at least some of the other second portions. Examples of the locations of second portions of the retina relative to respective first portions of the retina are described in more detail below.

In process S74 of Figure 7, the controller 140 processes the respective OCT data of each second portion of the retina to generate respective ORG data for the second portion, which ORG data is indicative of a respective physiological response of the second portion of the retina to the optical stimulus applied to the corresponding first portion of the retina. The respective ORG data is in the same form as described with the ORG data 150 in Figure 5.

It is noted that the controller 140 may, as in the present example embodiment, first acquire a first indicator of the plurality of indicators 141 and use this to acquire first OCT data corresponding to the first indicator. The controller 140 may then repeat this OCT data acquisition process (workflow) using each of the remaining indicators of the plurality of indicators 141 until respective OCT data has been acquired for each of the remaining indicators 141. The controller 140 may then process the respective OCT data to generate the respective ORG data in process S74 of Figure 7. However, the generation of the ORG data may be performed in parallel with the acquisition of the respective OCT data. That is, after the first respective OCT data is acquired, the controller 140 may begin performing process S74 to generate respective ORG data based on the first respective OCT data whilst repeating the aforementioned workflow for each of the remaining indicators 141, generating respective ORG data based on the respective OCT data corresponding to each of the plurality of indicators in parallel as it is acquired. Further, process S71 of Figure 7 may be performed to acquire all of the plurality of indicators before any of processes S72, S73 and S54 is performed for the first indicator of the plurality of indicators.

The controller 140 may perform processes S72 and S73 of Figure 7 (for all of the plurality of indicators 141) during the ORG capture session after only a single period of dark adaptation of the retina of the eye 160. This is made possible by a combination of the applied optical stimulus being confined to a first portion of the retina which is smaller than the field of view of the FD-OCT imaging system 130, and the location of the applied optical stimulus on the retina being changeable among a plurality of different locations on the retina by the optical system 120. As the optical stimulus applied to each of the first portions of the retina of the eye 160 provides no stimulation in any of the other first portions of the retina of the eye 160, the need to provide a period of dark adaptation after each application of the optical stimulus to the retina of the eye 160 (as with the existing ORG techniques described in the background section) is avoided. Further, the controller 140 may perform processes S72 and S73 (for all of the plurality of indicators) to acquire ORG data for a plurality of retinal locations on a much smaller timescale than that required for light adaptation, (e.g. within a period not exceeding 0.1, 1 or 3 seconds, for example), which is not achievable using the conventional approaches described above.

It is noted that the controller 140 may first acquire respective second indicators of the plurality of second portions of the retina at which the respective OCT data is to be acquired, and may then generate the plurality of indicators 141 such that each first portion of the retina, whose location on the retina is indicated by the respective one of the indicators 141, stimulates at least a part of a respective second portion of the retina (i.e. such that respective ORG data may be generated based on respective OCT data at the respective second portion of the retina) when the optical stimulus is applied to the first portion of the retina.

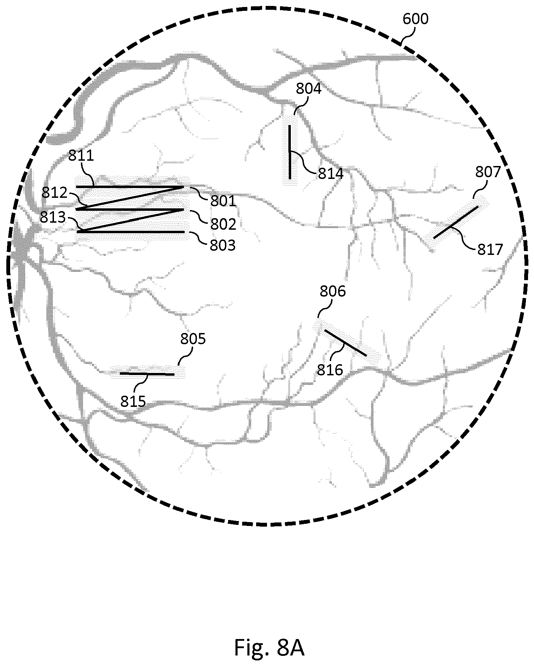

Figures 8A to 8E are schematic illustrations of example first portions of the retina of the eye 160 and example second portions of the retina of the eye 160 in processes S72 and S73 of Figure 7, which are respectively illuminated by any of the implementations of the optical system 120, and imaged by any of the implementations of the FD-OCT imaging system 130 according to the example embodiment (e.g. the optical system 300 and the FD-OCT imaging system 320), unless otherwise stated. These figures show the region 600 of the eye 160 corresponding to the field of view of the FD-OCT imaging system 130 shown in Figures 6A to 6F.

Figure 8A shows a plurality of first portions 801 to 807 as an example of the plurality of first portions in process S72 of Figure 7, and a plurality of second portions 811 to 817 as an example of the plurality of second portions in process S73 of Figure 7, in the ORG capture session. The controller 140 performs processes S72 and S73 to stimulate the first portion and acquire OCT data 135 from the corresponding second portion for each pair of first and second portions 801 and 811, 802 and 812, 803 and 813, etc. that are disposed at different locations on the retina, in turn, in the manner described above in relation to the straight first portion 630 and the second portion 631 in Figure 6C, for example.

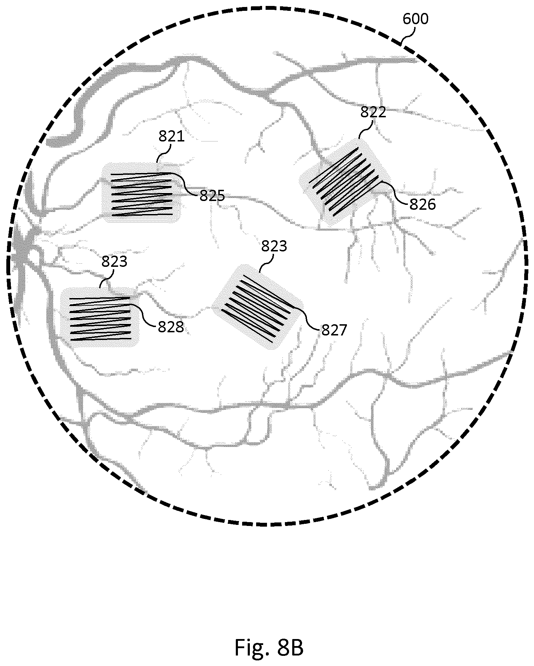

Figure 8B shows a plurality of first portions 821 to 824 as an example of the plurality of first portions in process S72 of Figure 7, and a plurality of second portions 825 to 824 as an example of the plurality of second portions in process S73 of Figure 7, in the ORG capture session. The controller 140 performs processes S72 and S73 to stimulate the first portion and acquire OCT data 135 from the corresponding second portion for each pair of first and second portions 821 and 825, 822 and 826, 823 and 827, etc. that are disposed at different locations on the retina, in turn, in the manner described above in relation to the straight first portion 660 and the second portion 661 in Figure 6F, for example.

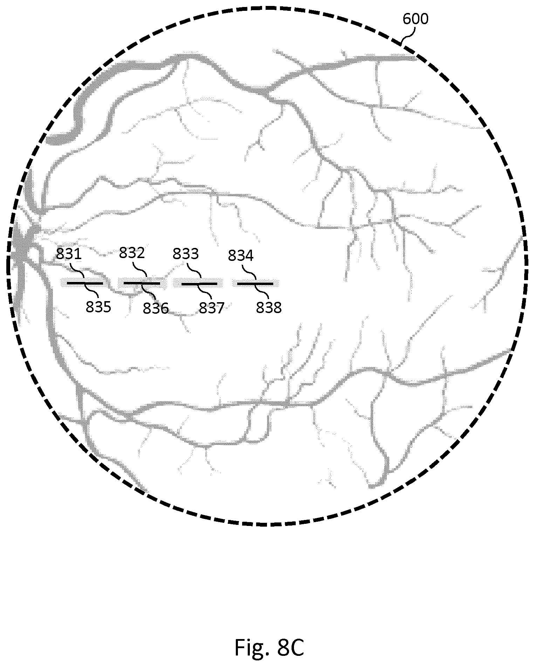

Figure 8C shows a plurality of first portions 831 to 834 as an example of the plurality of first portions in process S72 of Figure 7, and a plurality of second portions 835 to 838 as an example of the plurality of second portions in process S73 of Figure 7, in the ORG capture session, which may be used to perform a radial trend analysis of the eye 160 via analysing the variation of the retinal response of the eye 160 with eccentricity of the eye 160 (e.g. from the periphery of the eye 160 to the fovea of the eye 160). The controller 140 performs processes S72 and S73 to stimulate the first portion and acquire OCT data 135 from the corresponding second portion for each pair of first and second portions 831 and 835, 832 and 836, 833 and 837, etc. that are disposed at different locations along a line on the retina, in turn, in the manner described with reference to the straight first portion 630 and the second portion 631 in Figure 6C, for example.

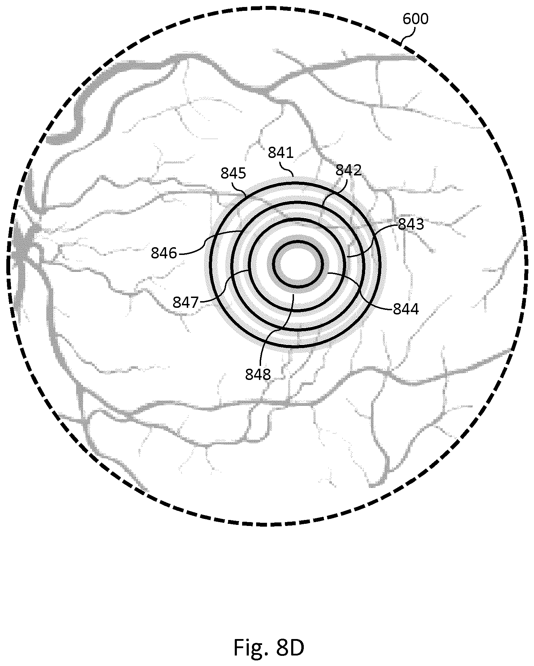

Figure 8D shows a plurality of first portions 841 to 844 as an example of the plurality of first portions in process S72 in Figure 7, and a plurality of second portions 845 to 848 as an example of the plurality of second portions in process S73 of Figure 7, in the ORG capture session, which may be also used to perform a radial trend analysis of the eye 160, where the variation of the retinal response of the eye 160 with eccentricity of the eye 160 is analysed. To achieve this, each of the first and second portions may form concentric ring-like regions that are centred on the fovea of the eye 160, which correspond to different degrees of eccentricity of the eye 160. The controller 140 performs processes S72 and S73 to stimulate the first portion and acquire OCT data 135 from the corresponding second portion for each pair of first and second portions 841 and 845, 842 and 846, 843 and 847, etc. that are disposed at different distances from the fovea, in turn, in the manner described above with reference to the hollow circular first portion 620 and the second portion 621 in Figure 6B.

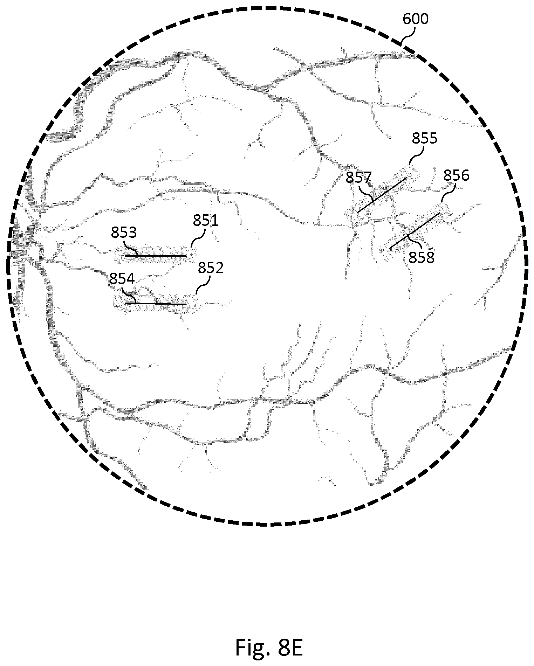

Figure 8E shows a plurality of first portions 851, 852, 855, 856 as an example of the plurality of first portions in process S72 of Figure 7, and a plurality of second portions 853, 854, 857, 858 as an example of the plurality of second portions in process S73 of Figure 7, in the ORG capture session. The controller 140 performs processes S72 and S73 to stimulate the first portion and acquire OCT data 135 from the corresponding second portion for each pair of first and second portions 851 and 853, 855 and 857, etc. that are disposed at different locations on the retina, in turn, in the manner described with reference to the straight first portion 630 and the second portion 631 in Figure 6C, for example. In this case, the controller 140 controls the optical system 120 to apply a first optical stimulus to the first portions 851 and 852 on the retina, and to applies a second optical stimulus to the second portions 855 and 856, wherein the first optical stimulus is different to the second optical stimulus (e.g. by the light source of the optical system 120 generating light at two different wavelengths or the optical system 120 comprising two differing light sources arranged to generate light at different wavelengths). Although two differing optical stimuli are described, the optical system 120 is not so limited and may be operable to generate further differing optical stimuli (e.g. as described with reference to the light source 301 of the optical system 300 in Figure 3A).

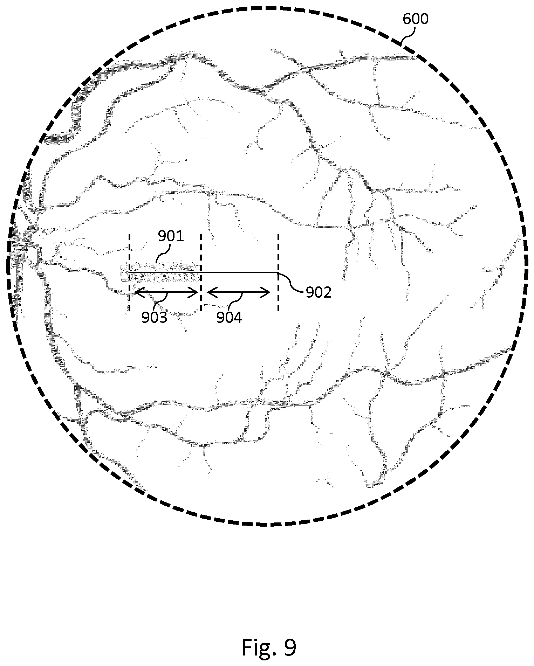

Figure 9 shows a first portion 901 and a second portion 902 in the region 800, which may respectively be examples of the first portion in process S52 of Figure 5 and the second portion in process S53 of Figure 5. A stimulus applied to the first portion 901 stimulates a first part 903 of the second portion 902 but does not stimulate a second part 904 of the second portion 902. Thus, the second part 904 provides a baseline unstimulated region which can be used for comparison and/or normalisation purposes. This technique of partially stimulating the second portion (by performing a temporal modulation of a signal used to generate the optical stimulus) may also be used in any of the other examples describe above with reference to Figures 8A to 8E. Further, although this technique has been described in relation to a straight first portion 630 and a straight second portion 631, it will be appreciated that this technique is not so limited, and the principles described may be applied to other geometries of first and second portions of the retina.

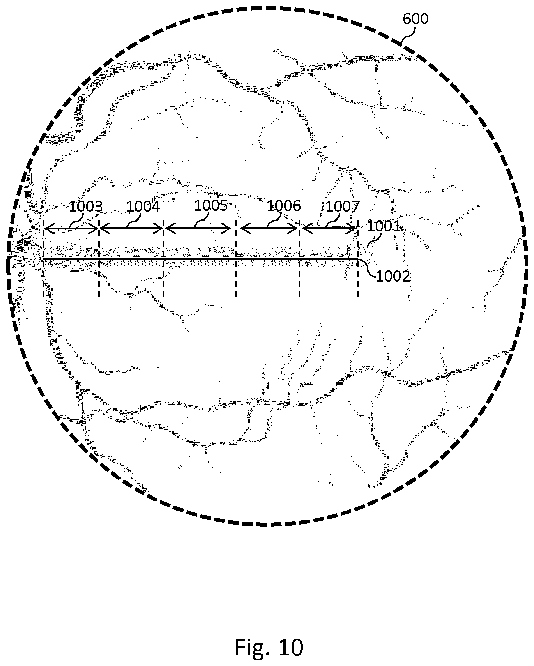

In some cases, the controller 140 may advantageously split the processing of the OCT data, to generate the ORG data 150, into smaller steps. Figure 10 shows an example first portion 1001 and an example second portion 1002 in the region 800 which may, respectively, provide examples of the first portion in process S52 and the second portion in process S53 of Figure 5 or, respectively, a portion of the plurality of first portions in process S72 of Figure 7 and a portion of the plurality of second portions in process S73 of Figure 7. As shown in Figure 10, the OCT data of the second portion 1002 may be divided into subsets of the OCT data respectively corresponding to regions 1003 to 1007, and each of the subsets of the OCT data may be separately processed using the above-described techniques to generate the ORG data 150 for each of the parts 1003 to 1007 on the retina of the eye 160. For example, in the velocity-based ORG technique described above, the temporal sequence of partial B-scans acquired from each region of the regions 1003 to 1007 may be used as the temporal sequence of B-scans in this algorithm to generate ORG at the respective location of the temporal sequence of the partial B-scans. This technique of splitting up the processing of the second portion may also be performed for at least some of the remaining portions of the plurality of second portions (if any). Further, while this technique has been shown and described in relation to a straight first portion 1001 and a straight second portion 1002, it will be appreciated that this technique is not so limited, and the principles described may be applied to other geometries of first and second portions of the retina.

The controller 140 of FD-OCT apparatus 100 may, as in the present example embodiment, be used to store, for each second portion of the second portions of the retina of the eye 160 described with reference to Figure 7, respective ORG data generated for the second portion (the ORG data being indicative of a respective physiological response of the second portion of the retina to the optical stimulus applied to the corresponding first portion of the retina) in association with a respective indication of a position of a point in a visual field of the eye 160 which is optically conjugate with the position of the second portion on the retina of eye 160 (e.g. the position of a point on the second portion, such as a geometric centre of the second portion). The stored ORG data and the stored associated indications may provide an indication of the retinal function of the eye 160 at a plurality of points in the visual field of the eye 160, which may aid in the diagnosis of a range of pathological conditions of the eye. Further, the stored ORG data and the stored associated indications may be used to compliment the results of a visual field test of the eye 160 (e.g. an automated static perimetry test, a kinetic visual field test or frequency doubling perimetry), specifically by providing further information that may help to establish the cause of any blind spots, or reduced retinal sensitivity, within the visual field of the eye 160 as determined by the visual field test, as described in more detail below.

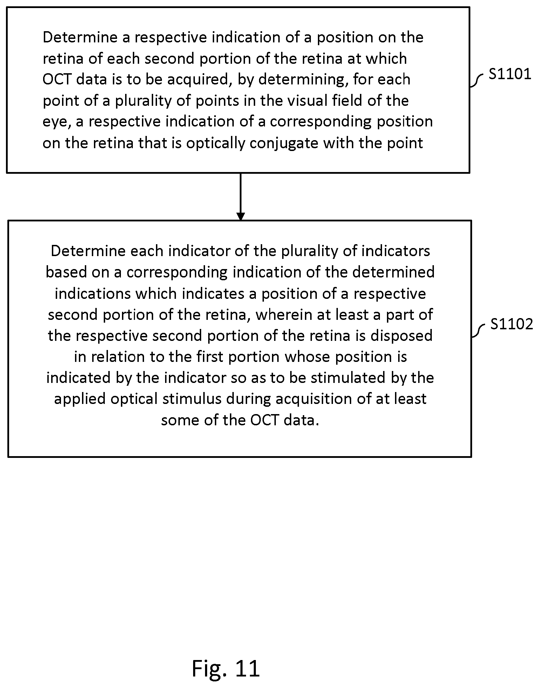

The data items that are stored in association with one another by the controller 140 as described above may be generated in different ways. For example (as discussed in more detail below, with reference to Figure 11), the locations of points on the retina that are to be stimulated by the optical system 120 and have OCT data (for ORG data generation by the controller 140) acquired therefrom by the FD-OCT imaging system 130 may be selected by the controller 140 based on predefined locations in a visual field of the eye 160. This scheme may be useful where the ORG data 150 generated by the controller 140 is to be compared with measurements that have been (or will be) acquired at those same locations in a visual field test.

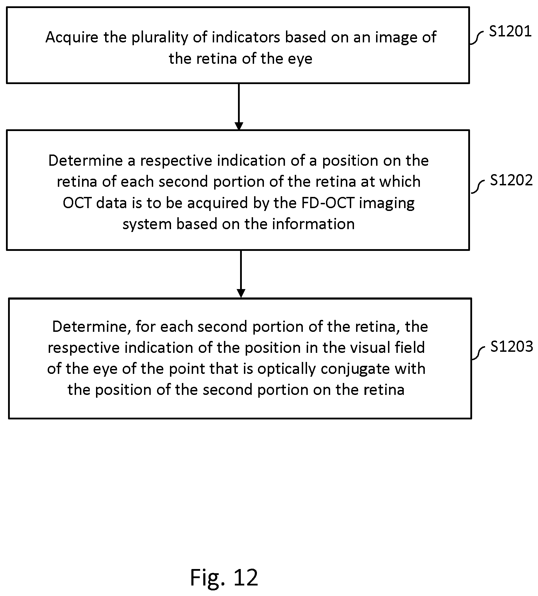

Alternatively, the locations of points on the retina that are to be stimulated by the optical system 120 and have OCT data acquired therefrom by the FD-OCT imaging system 130 may be selected by the controller 140 based on an image of the retina of the eye 160, so that the ORG data 150 that is ultimately generated by the controller 140 is derived from one or more regions of the retina that are of interest to study. The ORG data 150 generated in this way may similarly be compared with measurements that have been (or will be) acquired in a visual field test, by mapping positions on the retina associated with items of the OCT/ORG data to conjugate positions in the visual field of the eye 160. Although the items of ORG data 150 and the measurements in the visual field test may not have been acquired at respective sets of points that are optically conjugate in this case, a meaningful comparison may nevertheless be made. This alternative is described in more detail below, with reference to Figure 12.

Figure 11 is a flow diagram illustrating a process by which the controller 140 may determine indicators of target locations on the retina at which the optical stimulus is to be applied, and indications of positions on the retina at which OCT data 135 is to be acquired, on the basis of points in a visual field of the eye, in order to facilitate comparison of visual field test data with the ORG data 150 generated by the controller 140. The controller 140 may perform this process prior to performing the process described with reference to Figure 7, whereby the controller 140 controls the FD-OCT imaging system 130 to acquire the respective OCT data of each of the plurality of second portions of the retina of the eye 160 corresponding to the respective first portions of the retina where the optical stimulus is applied. However, the controller 140 may alternatively perform the processes in Figure 11 in parallel with those in Figure 7.

In process S1101 of Figure 11, the controller 140 determines a respective indication of a position on the retina of each second portion of the retina at which OCT data 135 is to be acquired by the FD-OCT imaging system 130. The controller 140 does this by determining, for each point of a plurality of separate points in the visual field of the eye 160, a respective indication of a corresponding position on the retina that is optically conjugate with the point. The plurality of points in the visual field of the eye 160 may, as in the present example embodiment, be an arrangement (e.g. on a grid or array) of points for which the results of a visual field test (e.g. an automated static perimetry test, a kinetic visual field test or frequency doubling perimetry) are available for comparison with the ORG data 150 to be generated by the controller 140, so that the ORG data 150 may be acquired from substantially the same portions of the retina as have been illuminated by the stimuli used in the visual field test and therefore had their responses to the stimuli assessed in the visual field test. The plurality of points in the visual field may be provided by a visual field-testing device such as an Octopus^{™} visual field test device or Humphrey^{™} Field Analyzer (HFA), for example.

In process S1102 of Figure 11, the controller 140 determines each indicator 141 of the plurality of indicators 141 (which indicator 141 is indicative of a target location on the retina at which the optical stimulus is to be applied by the optical system 120), so that an optical stimulus can be appropriately applied to stimulate the intended portions of the retina for which the ORG data 135 is to be acquired. This determination in process S1102 of Figure 11 is based on a corresponding indication, from among the indications determined in process S1101 of Figure 11, which indicates a position of a respective second portion of the retina. Here, at least a part of the respective second portion of the retina is disposed in relation to the first portion, whose position is indicated by the indicator 141, so as to be stimulated by the optical stimulus applied to the first portion during acquisition of at least some of the OCT data 135. The corresponding first and second portions may at least partially overlap, for example. Once at least some (i.e. one or more) target location indicators, which indicate target locations on the retina at which the optical stimulus is to be applied, have been determined in this way, the process of generating the ORG data 135 described above with reference to Figure 7 may be begin.

The position of the second portion of the retina may include a representative location of the second portion of the retina (for example, a point (e.g. a central point) along a scan line on the retina along which repeat B-scans are taken as the OCT data 135). Thus, the position of the second portion may be that of a point, defined in cartesian coordinates (xₓ, yₙ, zₙ) or in any other coordinate system, relative to a reference location (e.g. a point on the retina at which the optical axis or visual axis of the eye 160 passes, or the location of an anatomical feature on the retina, such as the fovea or the optic disc). The corresponding position in the visual field of the eye 160 that is optically conjugate with a position of a second portion of the retina may be a point whose location may, for example, be defined in terms of a first angle α from the visual axis of the eye 160 which is in the temporal-nasal direction of the eye 160, and a second angle β from the visual axis of the eye 160 which is in the superior-inferior direction of the eye 160, or in any other suitable coordinate system.

Figure 12 is a flow diagram illustrating a process by which the controller 140 may select a set of indicators 141, each indicating a target location on the retina at which an optical stimulus is to be applied by the optical system 120, and corresponding positions at which OCT data 135 is to be acquired by the FD-OCT imaging system 130, and map positions on the retina at which OCT data 135 has been acquired to corresponding positions in the visual field of the eye 160 so that the generated ORG data 150 may be compared with the results of a visual field test performed on the eye 160.

In process S1201 of Figure 12, the controller 140 acquires the plurality of indicators 141 based on an image of the retina of the eye 160. The controller 140 may acquire the plurality of indicators 141 by using received designations of retinal locations made by the user (as described above with reference to process S51 of Figure 5, for example) to set, as the indicators 141, respective coordinates of points of an array of points on the retina at which the optical stimulus is to be applied, such that the array of points is overlaid onto a region of the retina (e.g. a region of the macula) from which ORG data is required. The controller 140 may alternatively acquire the plurality of indicators 141 automatically, for example based on an image of the retina of the eye 160 and information indicative of a spatial distribution of photoreceptor cells across the retina of the eye 160, which may be estimated or predicted based on results of previous studies (measurements) of the spatial distribution in human retinas, for example as reported in "Count and density of human retinal photoreceptors" by J. B. Jonas et al., Graefe's Arch Clin Exp Ophthalmol 230, pages 505-510 (1992).

In process S1202 of Figure 12, the controller 140 determines a respective indication of a position on the retina of each second portion of the retina at which OCT data is to be acquired by the FD-OCT imaging system 130, based on the aforementioned information. The corresponding first and second portions may at least partially overlap (as in the case of the optical arrangements described above with reference to Figures 3A and 3B, for example) or they may be separated from but be in sufficiently close proximity to each other (as may be realised in the optical arrangement described above with reference to Figure 3E, for example) such that the optical stimulus applied to the first portion elicits a response from the retina in the second portion.

In process S1203 of Figure 12, the controller 140 determines, for each second portion of the retina, the respective indication of the position in the visual field of the eye 160 of the point that is optically conjugate with the position of the second portion on the retina. This may be performed by the controller 140 using a mapping between points on the retina of the eye 160 and optically conjugate points in the visual field of the eye 160. This mapping may be determined using any technique known in to those skilled in the art. For example, the mapping may be based on a ray tracing model of the eye 160 or based on a ray tracing model of an eye defined by the average parameters of a set of eyes sampled from a population, for example.

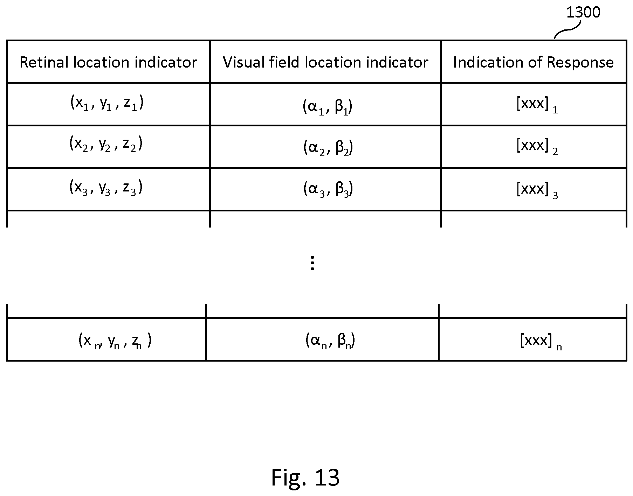

Figure 13 is a schematic illustration of a representation in tabular form of a data structure 1300 which may, as in the present example embodiment, be stored by the controller 140 in a memory of the controller 140. The data structure 1300 contains the indications of the positions of the second portions on the retina (xₙ, yₙ, zₙ) in a first (optional) column of the table, indications of the positions (αₙ, βₙ) of points in the visual field of the eye that are conjugate to positions of the second portions in a second column of the table, and the corresponding ORG data [XXX]ₙ in a third column of the table. Each row in the data structure 1300 thus stores a respective indication of a position of a respective second portion on the retina (xₙ, yₙ, zₙ), in association with a respective indication of a position (αₙ, βₙ) of the respective point in the visual field of the eye 160 which is conjugate to position of respective second portion, and in association with respective ORG data [XXX]ₙ. However, it will be appreciated that the data structure 1300 is not so limited, and may take other forms. Note that the headings of each column are included for explanatory purposes only, and the form of the ORG data [XXX]ₙ may take different forms, as described above in relation to process S54 of Figure 5.

The stored respective ORG data and associated respective indications of positions in the visual field of the eye 160 may be used to compliment a visual field test of the eye 160 in providing further information that may be helpful in determining the cause of any blind spots or reduced retinal sensitivity within the visual field of the eye 160.

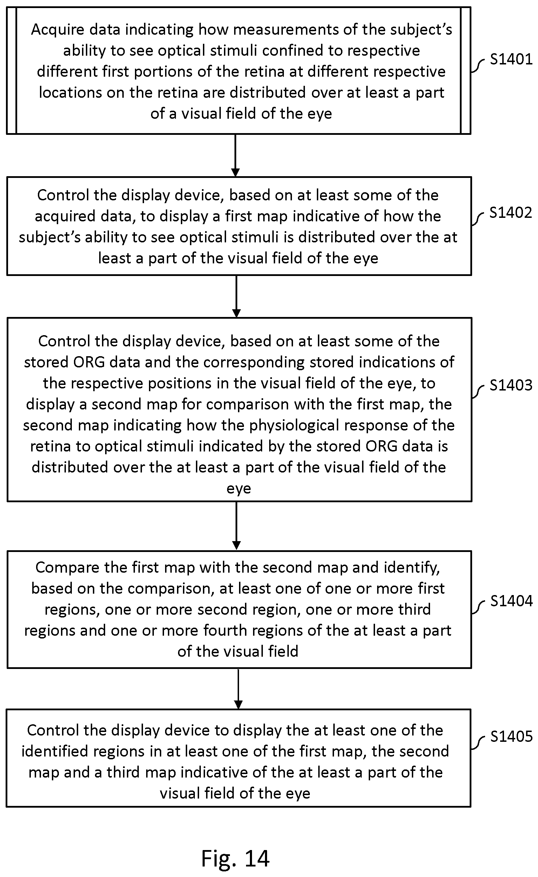

Figure 14 is a flow diagram illustrating a process by which the controller 140 may, as in the present example embodiment, acquire data indicating how measurements of the subject's ability to see optical stimuli that are confined to respective different portions of the retina of the eye 160 at different respective locations on the retina are distributed over at least a part of a visual field of the eye 160, and control the map display device 142 to display a first map, indicative of how the subject's ability to see optical stimuli with the eye 160 is distributed over the at least a part of the visual field of the eye 160, and a second map, indicating how the response of the retina to optical stimuli indicated by the stored respective indications is distributed over the at least a part of the visual field of the eye 160, for comparison purposes.

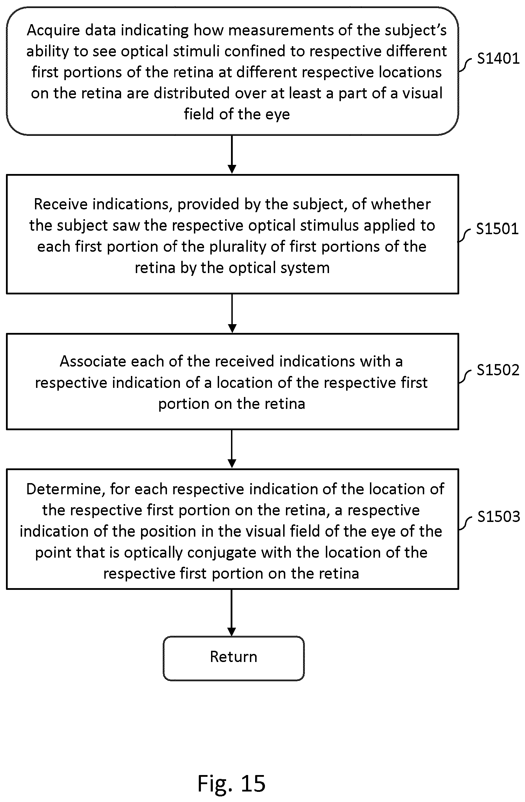

In process S1401 of Figure 14, the controller 140 acquires data indicating how measurements of the subject's ability to see optical stimuli that are confined to respective different portions of the retina of the eye 160 at different respective locations on the retina are distributed over at least a part of a visual field of the eye 160. Figure 15 is a flow diagram illustrating a process by which the controller 140 may, as in the present example embodiment, acquire this data.

Referring to Figure 15, in process 51501, the controller 140 receives respective indications, provided by the subject, of whether the subject saw the respective optical stimulus applied to each first portion of the plurality of first portions of the retina by the optical system 120. For example, the FD-OCT apparatus 110 may comprise a button (which may be a physical button or a virtual button on a display of the FD-OCT apparatus 110, for example), which can be pressed by the subject when the subject sees an optical stimulus provided by the optical stimulus.

In process 51502 of Figure 15, the controller 140 associates each of the received indications with a respective indication of a location of the respective first portion on the retina. The controller 140 may achieve this by, for example, matching the received indications from the subject with one of the time periods within which an optical stimulus was applied to a first portion of the plurality of first portions of the retina by the optical system 120.

In process S1503 of Figure 15, the controller 140 determines, for each respective indication of the location of the respective first portion on the retina, a respective indication of the corresponding position in the visual field of the eye 160 of a point that is optically conjugate with the location of the respective first portion on the retina. The controller 140 may achieve this, for example, by using the techniques described in relation to process S1203 of Figure 12.

The controller 140 may alternatively acquire the aforementioned data (indicating how measurements of the subject's ability to see optical stimuli that are confined to respective different portions of the retina of the eye 160 at different respective locations on the retina are distributed over at least a part of a visual field of the eye 160) by receiving visual field test data which has been acquired from the eye 160 (e.g. by a kinetic or static perimetry device). For example, the visual field test data may be acquired from the eye 160 by performing a visual field test on the eye 160 using a visual field-testing device such as, for example, an Octopus^{™} visual field test device or a Humphrey^{™} Field Analyzer (HFA). The visual field test data may more generally be acquired via forms of perimetry including, for example, the use of a tangent screen, the use of a Goldmann perimeter, automated perimetry and microperimetry (which is described in more detail below). Further, the form of perimetry may use either a static or kinetic presentation of the visual field test stimulus so as to perform a static perimetry test or a kinetic perimetry test, for example, and the visual field test stimulus used may be selected so as to perform a photoreceptor specific perimetry test, such as a photopic perimetry test or a scotopic perimetry test, for example.

The visual field test data acquired from the eye 160 may comprise values indicative of the subject's ability to see optical stimuli in the visual field of the eye 160 presented at each of a plurality of different locations in the visual field of the eye 160. For example, where the visual field test data is acquired from the eye 160 by performing a visual field test on the eye 160 using the HFA, the values may be indicative of the patient's retinal sensitivity (e.g. in dB) in a numerical display output from the FHA or may be the greyscale values in a greyscale plot output from the HFA. More generally, the values may be binary (e.g. a "1" or a "0"), dependent upon whether the subject indicated that they saw the optical stimuli or may be indicative of a threshold light intensity at which the subject indicated that they could see the optical stimuli a certain proportion (e.g. 50%) of the time. The locations in the visual field of the eye 160 may be defined a coordinate system similar to those described in relation to process S1101 of Figure 11. However, it will be appreciated that the form of the visual field test data is not so limited, and any form of visual field test data acquired using a visual field test of the eye 160 may be used.

Referring again to Figure 14, in optional process S1402, the controller 140 controls the map display device 142, based on at least some of the data acquired in process S1401 of Figure 14, to display a first map indicative of how the subject's ability to see optical stimuli with the eye 160 to be distributed over the at least a part of the visual field of the eye 160 (which may be as indicated by the at least some of the acquired visual field test data). For example, where the visual field test data is acquired from the eye 160 by performing a visual field test on the eye 160 using HFA, the first map may be similar to the numerical display, or the greyscale plot, output from this device.

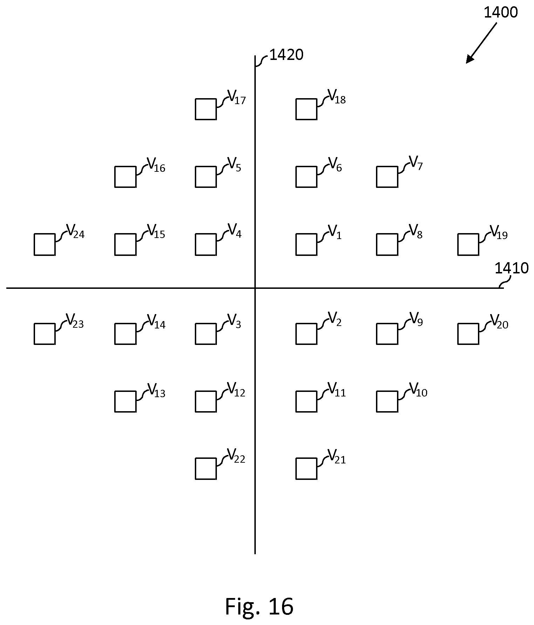

Figure 16 is a schematic illustration of an example first map 1400 which may be displayed in process 1402 of Figure 14. In this example, the acquired data is visual field test data obtained from a visual field-testing device. The visual field test data in this case comprises values V₁ to V₂₄ each indicative of a threshold light intensity at which the subject indicated that they could see the visual field optical stimuli at that location 50% of the time, as described above, plotted along a first axis 1410 corresponding to the first angle α, and a second axis 1420 corresponding to the second angle β. However, the first map 1400 may take other forms, depending on the type of visual field test used, such as a Goldmann visual field plot when the visual field test is performed using a Goldmann perimeter.

Referring again to Figure 14, in optional process S1403, the controller 140 controls the map display device 142, based on at least some of the stored ORG data 150 and the corresponding stored indications of the respective positions in the visual field of the eye 160, to display a second map for comparison with the first map. The second map is indicative of how the physiological response of the retina to optical stimuli indicated by the at least some of the stored ORG data 150 is distributed over the at least a part of the visual field of the eye 160.

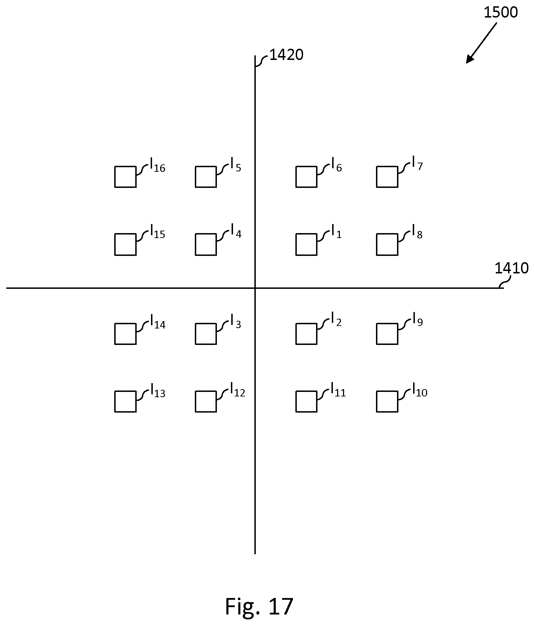

Figure 17 shows an example second map 1500, which may be displayed in process S1403 of Figure 14. The second map 1500 displays the stored ORG data I₁ to I₁₆ that is indicative of the retinal response at the corresponding locations of the second portion on the retina, as described above, on a similar plot of the visual field of the eye to the first map 1400. The stored ORG data I₁ to I₁₆ may, as in the present example embodiment, be indicative of the magnitude of the preliminary "alpha wave" in the retinal response (or the magnitude of the "beta wave" in the retinal response) at the corresponding locations of the second portion on the retina. This second map 1500 may be displayed adjacent to the first map 1400 on the map display device 142 to facilitate a comparison of the two maps, for example. However, the second map 1500 may alternatively be superimposed on the first map 1400 such that the acquired visual field test data V₁ to V₂₄ and the stored ORG data I₁ to I₁₆ are displayed on a single plot along the first axis 1410 corresponding to the first angle α and the second axis 1420 corresponding to the second angle β.

For comparison purposes, the locations in the visual field of the eye 160 at which the stored ORG data 150 are displayed on the second map of process S1403 of Figure 14 may be the same locations as those where the data acquired in process S1401 in Figure 14 is displayed on the first map of process S1402 of Figure 14, thus allowing the acquired data at each location to be easily and directly compared with an ORG response of the eye 160 at that same location. This may aid in improving the certainty of diagnosis of the cause of any defects in the visual field of the eye 160. This may be achieved, as described above with respect to the process of Figure 11, and may be particularly advantageous where the first and second maps are superimposed, as the clinician may view the respective ORG data 150 and acquired data side by side-at-each location (e.g. by displaying the respective value of the stored ORG data side-by-side with the respective value of the visual field test data at each location).

In optional process 51404 of Figure 14, the controller 140 compares data of the first map with data of the second map and identifies, based on the comparison, at least one region of the at least a part of the visual field. The controller 140 may identify, based on the comparison, at least one of: one or more first regions, one or more second regions, one or more third regions, and one or more fourth regions of the visual field or part thereof displayed in the maps. Each of these regions may, as in the present example embodiment, be identified by the controller 140 by determining whether or not the values of the visual field test data at respective locations in the visual field of the eye 160 are indicative of the subject being able to see optical stimuli (e.g. of a certain light intensity at least 50% of the time) at each respective location (in the visual field), and whether or not the respective ORG data at each corresponding location (e.g. the same location or a location which is nearest to the respective location as compared to the other remaining respective locations) is indicative of a physiological response to the optical stimulus which satisfies a predetermined condition. Satisfying the predetermined condition may indicate that the physiological response to the optical stimulus is indicative of a physiological response of a healthy eye to the optical stimulus (i.e. a healthy retinal response). For example, where the respective ORG data comprises a set of values each indicative of a magnitude of a retinal response to the optical stimulus, the predetermined condition may be that the value is greater than or equal to a threshold. Accordingly, a value which is greater than or equal to the threshold may be indicative of a healthy retinal response, while a value which is smaller than the threshold may be indicative of an unhealthy retinal response. The comparison made by the controller 140 may, as in the present example embodiment, allow the relationship between the data of the first map, and the data of the second map, to be classified into one of the aforementioned four categories for each region of a plurality of regions into which the first and second maps may be divided in any suitable way.

In the present example embodiment, the one or more first regions (i.e. different first regions) of the displayed visual field indicate that the subject is able to see optical stimuli in the one or more first regions, and further indicate that one or more corresponding regions of the retina provided a physiological response to the optical stimulus which satisfies the predetermined condition. These one or more first regions can thus be taken to correspond to regions of the retina that have a healthy (normal) functioning.

The one or more second regions (i.e. different second regions) of the displayed visual field indicate that the subject has an inability to see optical stimuli in the one or more second regions, and further indicate that one or more corresponding regions of the retina provided a physiological response to the optical stimulus which satisfies the predetermined condition. These one or more second regions can thus be taken to correspond to regions of the retina where, despite the retina responding to the optical stimulus normally, the subject could not see the applied stimuli (although this does not rule out that the subject might have been able to see stimuli of higher intensity, had these been applied in the visual field test). This indicates to a clinician that the cause of the subject's inability to see is unlikely to be malfunctioning retinal photoreceptors but may originate elsewhere, for example in the communication pathway between the retinal photoreceptors and the brain of the subject, for example. This may guide the clinician to reach the correct diagnosis more quickly.

The one or more third regions (i.e. different third regions) of the displayed visual field indicate that the subject can see optical stimuli in the one or more third regions, and further indicate that one or more corresponding regions of the retina provided a response to the optical stimulus which does not which satisfy the predetermined condition. These one or more third regions can thus be associated with the subject having falsely indicated they could see optical stimuli. Such regions may be investigated again with a further visual field test or excluded from (or weighted with reduced confidence in) the analysis of the function of the retina, thus further improving the accuracy of any diagnosis made by the clinician.

The one or more fourth regions (i.e. different fourth regions) of the displayed visual field indicate that the subject has an inability to see optical stimuli in the one or more fourth regions, and further indicate that one or more corresponding regions of the retina provided a physiological response to the optical stimulus which does not which satisfy the predetermined condition. These one or more fourth regions thus correspond to one or more regions of retina that are not functioning healthily, the identification of which may save the clinician time when performing their analysis of retinal function of the eye 160 by focussing their attention initially on these regions.

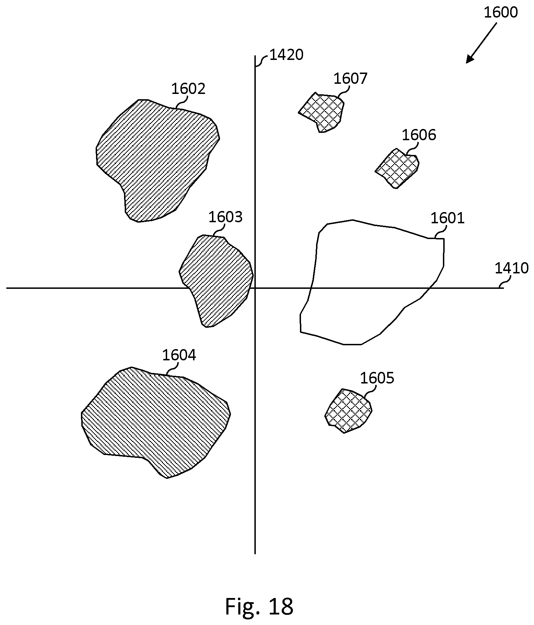

Optionally, in process S1405 of Figure 14, the controller 140 controls the map display device 142 to display any identified region(s) in at least one of the first map, the second map and a third map indicative of the at least a part of a visual field of the eye 160. Figure 18 shows an example third map 1600, which may be displayed by the map display device 142 in process S1405 of Figure 14. The third map 1600 is on a similar plot to the example first map 1400 and the example second map 1500 (i.e. a plot along the first axis 1410 corresponding to the first angle α, and the second axis 1420 corresponding to the second angle β). The third map 1600 shows a region 1601, which is an identified first region as described above, two regions 1602 and 1603 that are identified second regions as described above, a region 1604 which is an identified third region as described above, and three regions 1605, 1606 and 1607 that are identified fourth regions as described above. These regions 1601 to 1607 may alternatively be shown as superimposed on the first map, the second map or onto the superposition of the first and second maps, as described above.

Although the display of the first map indicative of the subject's ability to see optical stimuli as distributed over at least a part of a visual field of the eye 160 and of the second map indicative of the physiological response of the retina to optical stimuli indicated by the stored respective indications as distributed over the at least a part of the visual field of the eye 160 has been described above, the controller 140 may, in an alternative implementation of the example embodiment, control the map display device 142 to display a fourth map indicative of the subject's ability to see optical stimuli with the eye 160 as distributed over at least a part of the retina of the eye 160, and a fifth map indicative of the physiological response of the retina to the optical stimulus indicated by at least some of the stored ORG data as distributed over the at least a part of the retina of the eye 160. This may be preferable to the clinician as it allows a comparison of the visual field test data and ORG data when overlaid on an image of the retina of the eye 160 to be made, so to enable the two sets of data to be used to interpret structural features of the eye 160 in the image, for example. In particular, the fourth map may be that obtained from a microperimetry test of the eye 160, allowing the respective ORG data to be mapped to, and displayed alongside, microperimetry results acquired by the controller 140, as described below.

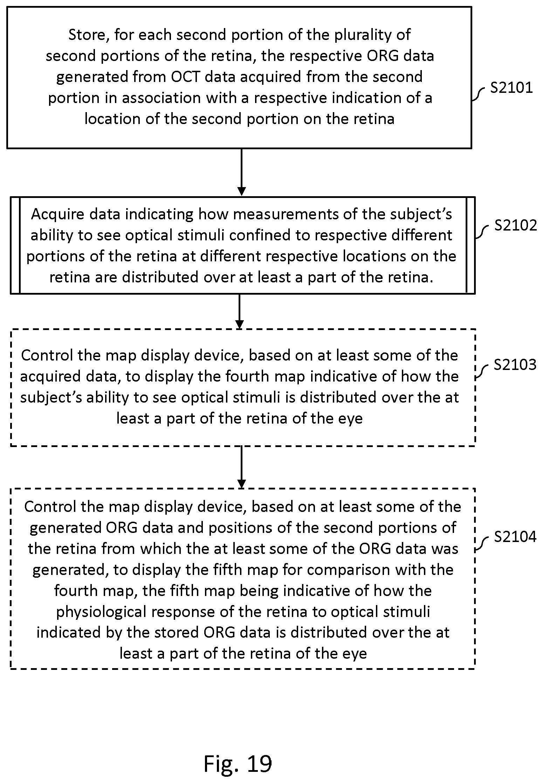

Figure 19 is a flow diagram illustrating a process by which the controller 140 may, as in the alternative implementation of the example embodiment, acquire data indicating how measurements of the subject's ability to see optical stimuli that are confined to respective different portions of the retina of the eye 160 at different respective locations on the retina are distributed over the at least a part of the retina of the eye 160, and control the map display device 142 to display the fourth map and the fifth map.

In process S2101 of Figure 19, the controller 140 stores, for each second portion of the plurality of second portions of the retina, the respective ORG data 150, which has been generated from OCT data 135 acquired from the second portion, in association with a respective indication of a location of the second portion on the retina.

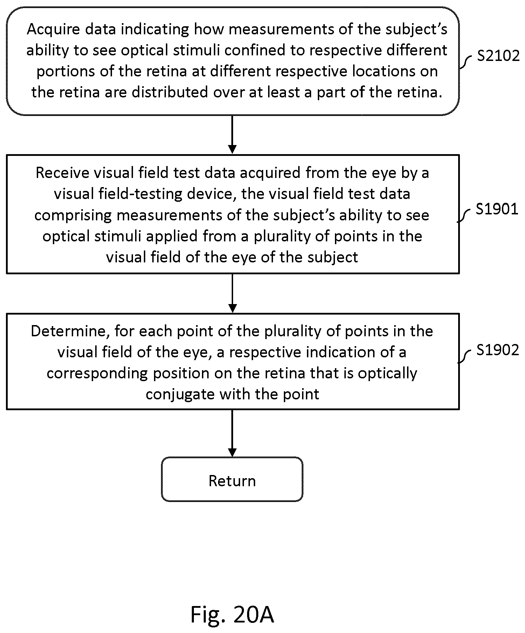

In process S2102 of Figure 19, the controller 140 acquires data indicating how measurements of the subject's ability to see optical stimuli that are confined to respective different portions of the retina of the eye 160 at different respective locations on the retina are distributed over at least a part of the retina. Figure 20A is a flow diagram illustrating a process by which the controller 140 may, as in the present example embodiment, acquire this data.

Referring to Figure 20A, in process S1901, the controller 140 receives visual field test data acquired from the eye 160 by a visual field-testing device, the visual field test data comprising measurements of the subject's ability to see optical stimuli applied from a plurality of points in the visual field of the eye 160 of the subject. The controller 140 may acquire the visual field test data from an HFA or other perimeter such as those described above, for example.

In process S1902 of Figure 20A, the controller 140 determines, for each point of the plurality of points in the visual field of the eye 160, a respective indication of a corresponding position on the retina that is optically conjugate with the point. The controller 140 may achieve this by using a mapping between coordinates of first points in the visual field of the eye and coordinates of second points on the retina that are optically conjugate to the first points, which may be obtained as described above, to generate the distribution information.

As an alternative to processes 51901 and S1902, the controller 140 may receive visual field test data acquired from the eye 160 by a visual field-testing device, the visual field test data indicating how measurements of the subject's ability to see optical stimuli applied to respective different locations on the retina of the eye 160 are distributed over at least a part of the retina of the eye 160. For example, the visual field test data may be acquired from a microperimetry device (e.g. Nidek^{™} MP-3 microperimeter, the Zeiss Humphrey^{™} Field Analyzer Model 860 or the Haag-Streit Octopus 900 Pro^{™}), the output of which also includes, as the distribution information, indications of the locations on the retina of the eye 160 (for example, in the form of coordinates of points on an image of the retina acquired by the microperimetry device) to which the optical stimuli from the microperimetry device were delivered.

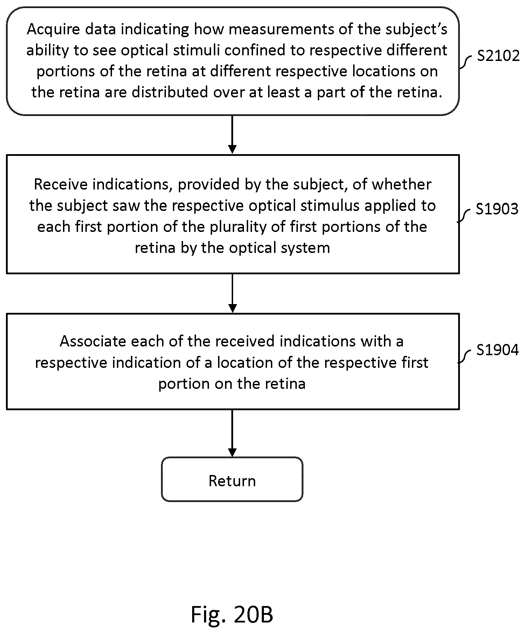

Figure 20B shows a flow diagram illustrating a process by which the controller 140 may alternatively acquire the aforementioned data.

In process 51903 of Figure 20B, the controller 140 receives indications, provided by the subject, of whether the subject saw the respective optical stimulus applied to each first portion of the plurality of first portions of the retina by the optical system 120. For example, the FD-OCT apparatus 110 may comprise a button (which may be a physical button or a virtual button on a display of the FD-OCT apparatus 110, for example) which can be pressed by the subject when the subject sees an optical stimulus provided by the optical system 120.

In process S1904 of Figure 20B, the controller 140 associates each of the received indications with a respective indication of a location of the respective first portion on the retina. The controller 140 may achieve this by, for example, matching the received indications from the subject with one of the time periods within which an optical stimulus was applied to a first portion of the plurality of first portions of the retina by the optical system 120.

Returning to Figure 19, in optional process S2103, the controller 140 controls the map display device 142, based on at least some of the data acquired in process S2101, to display the fourth map indicative of how the subject's ability to see optical stimuli (with the eye 160) is distributed over the at least a part of the retina of the eye 160. The fourth map may comprise an image of the part of the retina of the eye 160 as acquired, for example, by the microperimetry device (e.g., by a scanning laser ophthalmoscope included within the microperimetry device) or by the FD-OCT apparatus 100 (e.g. by the FD-OCT imaging system 130 or a scanning laser ophthalmoscope forming part of the -OCT apparatus 100).

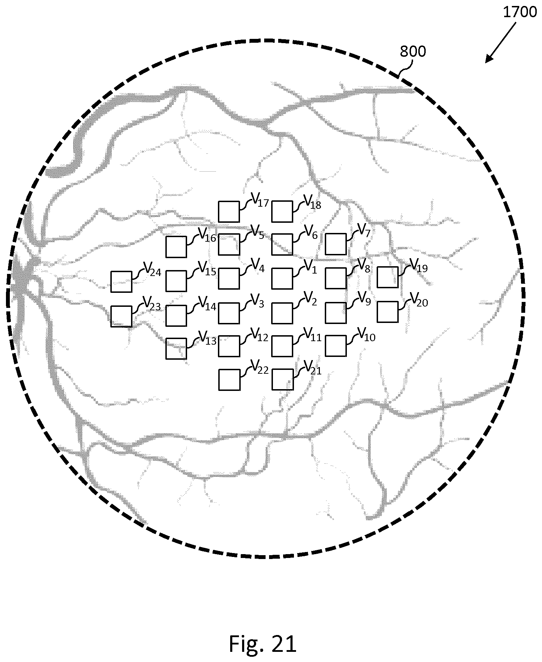

Figure 21 shows an example fourth map 1700, which shows the values V₁ to V₂₄ from the example first map 1400 in Figure 16 although these are distributed over an image of the region 800 of the retina of the eye 160. The locations of the values V₁ to V₂₄ in the region 800 are from the distribution information.

In optional process S2104 of Figure 19, the controller 140 controls the map display device 142, based on at least some of the generated ORG data and positions of the second portions of the retina from which the at least some of the ORG data was generated, to display the fifth map for comparison with the fourth map, the fifth map indicating how the physiological response of the retina to optical stimuli indicated by the at least some of the stored ORG data 150 is distributed over the at least a part of the retina of the eye 160. The fifth map may comprise an image of the part of the retina of the eye 160 in the same manner as described above for the fourth map.

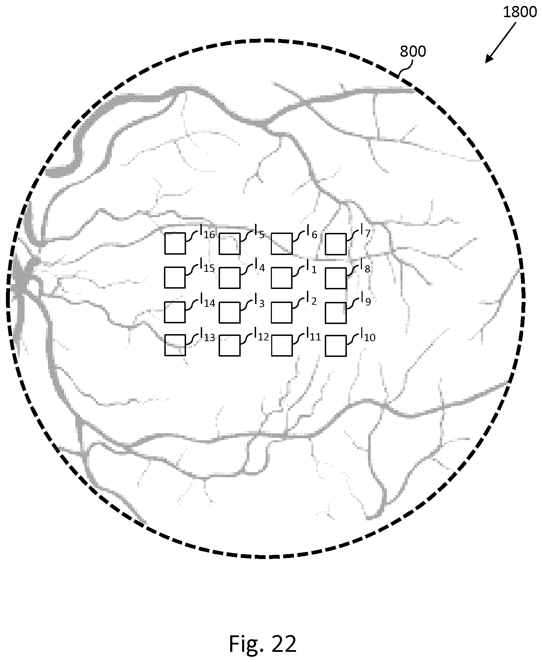

Figure 22 shows an example fifth map 1800, which shows the stored ORG data I₁ to I₁₆ from the example second map 1500 in Figure 17, although these are distributed over the image of the region 800 of the retina of the eye 160 in Figure 22. The locations of the stored ORG data I₁ to I₁₆ in the region 800 are determined as described above, and may be set at the same locations as the values of the visual field test data on the retina of the eye 160 by using the distribution information in process S2102 to, for example, determine the plurality of indicators in process S71 of Figure 7 or the locations of the second portions in process S73 of Figure 7, as described above. The use of the same locations for the respective ORG data 150 and the values of the visual field test data may facilitate the side-by-side comparison of the data sets by the clinician in allowing for the direct association between the data sets at each location.

The fourth map and the fifth map may be superimposed in the same manner described above with respect to the first map and second map Further, the controller 140 may identify one or more first regions, one or more second regions, one or more third regions, and one or more fourth regions as distributed over at least a part of the retina of the eye 160 by comparing the fourth map and the fifth map in the same manner to as described above with respect to the first map and the second map. In addition, these identified regions may be displayed by the map display device 142 in at least one of the fourth map, the fifth map and a sixth map indicative of the at least a part of the retina of the eye 160 in the same manner as described above for the first map, second map and the third map. Note that the controller 140 may perform the above-described processes to control the map display device 142 to display at least one of the fourth map, the fifth map and the sixth map in addition to at least one of the first map, the second map and the third map.

Although the FD-OCT apparatus 100 of the example embodiment described above comprises a steerable optical system 120, which is controllable by the controller 140 to vary a location on the retina at which the optical stimulus is to be applied whilst a fixed gaze direction of the eye is maintained by the fixation target 110 (by virtue of the position of the fixation target 110 relative to the eye 160 remaining fixed), and which applies a localised stimulus that is confined to a portion of the retina, the forms of the fixation target and of the optical system for providing the optical stimulus are not so limited. For example, in some example embodiments, particularly where relatively small amounts of ORG data are being acquired in each imaging session, the fixation target may be dispensed with altogether, and the subject may be asked to maintain a central gaze direction during the session. In other example embodiments, the optical system which provides the localised optical stimulus may not be steerable, and the location on the retina at which the optical stimulus is to be applied may be adjusted by instead varying the position of a fixation target relative to the eye 160, so as to perform eye-steering. In other example embodiments, a conventional optical system, which provides flood (widefield) illumination of the retina instead of a localised optical stimulus, may be employed. However, where such a conventional optical system is being used, the acquisition of OCT data 135 from a plurality of portions of the retina would generally necessitate the use of a light adaptation period between the acquisition of OCT data at each portion (since all portions intended for imaging would typically be illuminated by the optical system), which would slow down the generation of the ORG data 150 by the FD-OCT apparatus and may degrade its quality owing to movements of the subject caused by the more prolonged imaging procedure and associated likely increased degree of discomfort in interfacing with the FD-OCT apparatus 100.

In alternative example embodiments of the kinds set out above, the controller 140 may be generally arranged to: (i) control the FD-OCT imaging system 130 to acquire OCT data 135 of a plurality of portions of the retina such that, for each portion of the plurality of portions of the retina, at least some of the OCT data acquired from the portion is acquired after a respective optical stimulus has been applied to the portion by the optical system 120; (ii) generate, based on the acquired OCT data 135, respective ORG data 150 for each portion of the plurality of portions of the retina which is indicative of the respective response of the portion of the retina to the optical stimulus applied to the portion of the retina; and (iii) store, for each portion of the plurality of portions of the retina, the respective ORG data 150 generated for the portion in association with a respective indication of a position in a visual field of the eye 160 of a point that is optically conjugate with a corresponding position of the portion on the retina.

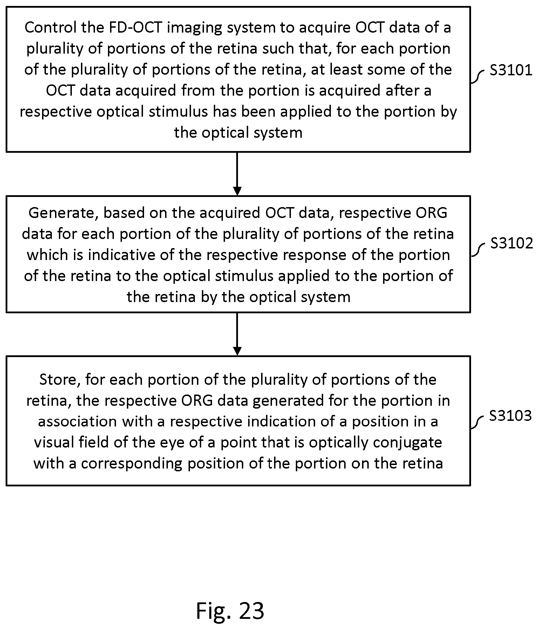

Furthermore, in such alternative example embodiments, the controller 140 may perform a method of controlling a FD-OCT apparatus to acquire ORG data 150 that is indicative of a physiological response of the retina of the eye 160 to an optical stimulus, as will now be described with reference to Figure 23. The FD-OCT apparatus being controlled in accordance with this method comprises an optical system 120 of one of the possible forms described above, for example, which is operable to apply the optical stimulus to the retina. The FD-OCT apparatus further comprises an FD-OCT imaging system 130 as described above, which is operable to acquire OCT data 135 by imaging a portion of the retina.

In process S3101 of Figure 23, the controller 140 controls the FD-OCT imaging system 130 to acquire OCT data 135 of a plurality of portions of the retina such that, for each portion of the plurality of portions of the retina, at least some of the OCT data acquired from the portion is acquired after a respective optical stimulus has been applied to the portion by the optical system 120.

In process S3102 of Figure 23, the controller 140 generates, based on the acquired OCT data 135, respective ORG data 150 for each portion of the plurality of portions of the retina which is indicative of the respective response of the portion of the retina to the optical stimulus applied to the portion of the retina by the optical system 120.

In process S3103 of Figure 23, the controller 140 stores, for each portion of the plurality of portions of the retina, the respective ORG data 150 generated for the portion in association with a respective indication of a position in a visual field of the eye 160 of a point that is optically conjugate with a corresponding position of the portion on the retina.

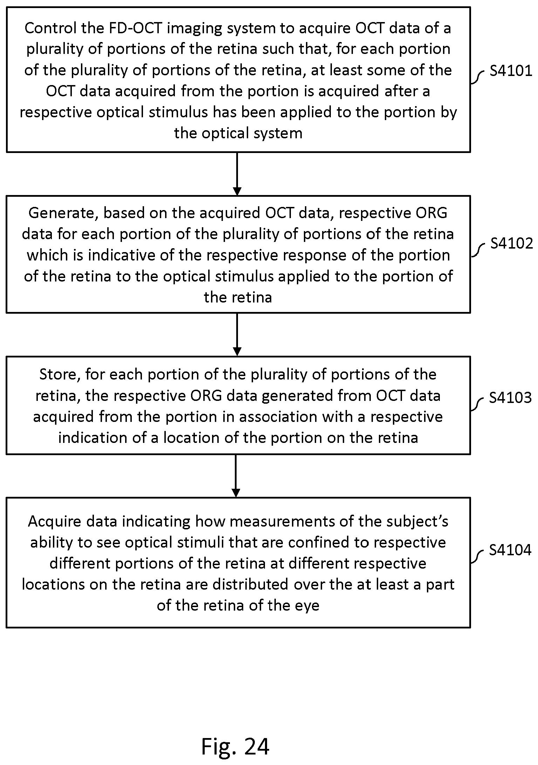

Alternatively, the controller 140 may perform a method of controlling a FD-OCT apparatus to acquire ORG data 150 that is indicative of a physiological response of the retina of the eye 160 to an optical stimulus, as will now be described with reference to Figure 24. The FD-OCT apparatus being controlled in accordance with this method comprises an optical system which is operable to apply a localised or a non-localised (wide-field) optical stimulus to the retina in one of the ways set out above. The FD-OCT apparatus further comprises an FD-OCT imaging system 130 as described above, which is operable to acquire OCT data 135 by imaging a portion of the retina.

In process S4101 of Figure 24, the controller 140 controls the FD-OCT imaging system 130 to acquire OCT data 135 of a plurality of portions of the retina such that, for each portion of the plurality of portions of the retina, at least some of the OCT data acquired from the portion is acquired after a respective optical stimulus has been applied to the portion by the optical system 120.

In process S4102 of Figure 24, the controller 140 generates, based on the acquired OCT data 135, respective ORG data 150 for each portion of the plurality of portions of the retina which is indicative of the respective response of the portion of the retina to the optical stimulus applied to the portion of the retina.

In process S4103 of Figure 22, the controller 140 stores, for each portion of the plurality of portions of the retina, the respective ORG data 150 generated from OCT data 135 acquired from the portion in association with a respective indication of a location of the portion on the retina.

In process S4104 of Figure 24, the controller 140 acquires data indicating how measurements of the subject's ability to see optical stimuli that are confined to respective different portions of the retina of the eye 160 at different respective locations on the retina are distributed over the at least a part of the retina of the eye 160. The controller 140 may be arranged to acquire the data, which indicates how measurements of the subject's ability to see optical stimuli applied to respective different locations on the retina of the eye 160 are distributed over at least a part of the retina, by: receiving visual field test data acquired from the eye 160 by a visual field-testing device, the visual field test data comprising measurements of the subject's ability to see optical stimuli applied from a plurality of points in the visual field of the eye 160; and determining, for each point of the plurality of points in the visual field, a respective indication of a corresponding position on the retina that is optically conjugate with the point. Alternatively, the controller 140 may be arranged to acquire the data, which indicates how measurements of the subject's ability to see optical stimuli applied to respective different locations on the retina of the eye 160 are distributed over at least a part of the retina, by: receiving indications, provided by the subject, of whether the subject saw the respective optical stimulus applied to each portion of the plurality of portions of the retina by the optical system 120; and associating each of the received indications with a respective indication of a location of the respective portion on the retina.

The controller 140 may then control the display device 142, based on at least some of the data acquired in process S4104 of Figure 24, to display a first map 1700 as described above, for example, which is indicative of how the subject's ability to see optical stimuli is distributed over at least a part of the retina of the eye 160, as well as a second map 1800 as described above, for example, for comparison with the first map 1700, the second map 1800 being indicative of how the response of the retina to optical stimuli indicated by the at least some of the generated ORG data 150 is distributed over the at least a part of the retina of the eye 160. The controller 140 may then compare data of the first map 1700 with data of the second map 1800 and identify, based on the comparison, at least one of the regions 1601 to 1607 as described above with reference to Figure 18.

The methods described above with reference to Figure 23 and 24 may be performed by a controller 140 of any kind described herein and, in general, by a computer executing computer-readable instructions of a computer program, which may be stored on a non-transitory storage medium (such as a CD, DVD or a hard disk, for example) or carried by a signal (e.g. in an Internet download).

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as functions of the controller 140, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the controller 140 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents. It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A Fourier-domain optical coherence tomography, FD-OCT, apparatus (100) arranged to acquire optoretinography, ORG, data (150) that is indicative of a physiological response of a retina of an eye (160) of a subject to an optical stimulus, the FD-OCT apparatus (100) comprising:
an optical system (120) operable to apply the optical stimulus to the retina;
an FD-OCT imaging system (130) operable to acquire OCT data (135) by imaging a portion of the retina; and
a controller (140) arranged to:
control the FD-OCT imaging system (130) to acquire OCT data (135) of a plurality of portions of the retina such that, for each portion of the plurality of portions of the retina, at least some of the OCT data acquired from the portion is acquired after a respective optical stimulus has been applied to the portion by the optical system (120);
generate, based on the acquired OCT data (135), respective ORG data (150) for each portion of the plurality of portions of the retina which is indicative of the respective response of the portion of the retina to the optical stimulus applied to the portion of the retina; and
store, for each portion of the plurality of portions of the retina, the respective ORG data (150) generated for the portion in association with a respective indication of a position in a visual field of the eye (160) of a point that is optically conjugate with a corresponding position of the portion on the retina.

2. The FD-OCT apparatus (100) according to Claim 1, wherein the controller (140) is further arranged to determine (S1101) a respective indication of a position on the retina of each portion of the plurality of portions of the retina at which OCT data (135) is to be acquired by the FD-OCT imaging system (130) by determining, for each point of a plurality of points in the visual field of the eye (160), a respective indication of a corresponding position on the retina that is optically conjugate with the point.

3. The FD-OCT apparatus according to Claim 1, wherein the controller (140) is further arranged to:
determine (S1202) a respective indication of a position on the retina of each portion of the plurality of portions of the retina at which OCT data (135) is to be acquired by the FD-OCT imaging system (130) based on an image of the retina of the eye (160); and
determine (S1203), for each portion of the plurality of portions of the retina, the respective indication of the position in the visual field of the eye (160) of the point that is optically conjugate with the position of the portion on the retina.

4. The FD-OCT apparatus according to any of Claims 1 to 3, further comprising a display device (142), wherein the controller (140) is further arranged to:
acquire (S1401) data indicating how measurements of the subject's ability to see optical stimuli applied confined to respective different portions of the retina of the eye (160) at different respective locations on the retina are distributed over at least a part of a visual field of the eye (160);
control (S1402) the display device (142), based on at least some of the acquired data, to display a first map (1400) indicative of how the subject's ability to see the optical stimuli is distributed over the at least a part of the visual field of the eye (160); and
control (51403) the display device (142), based on at least some of the stored ORG data (150) and the associated stored indications, to display a second map (1500) for comparison with the first map (1400), the second map (1500) indicating how the physiological response of the retina to the optical stimulus indicated by at least some of the stored ORG data (150) is distributed over the at least a part of the visual field of the eye (160).

5. A Fourier-domain optical coherence tomography, FD-OCT, apparatus (100) arranged to acquire optoretinography, ORG, data (150) that is indicative of a physiological response of a retina of an eye (160) of a subject to an optical stimulus, the FD-OCT apparatus (100) comprising:
an optical system (120) operable to apply the optical stimulus to the retina;
an FD-OCT imaging system (130) operable to acquire OCT data (135) by imaging a portion of the retina; and
a controller (140) arranged to:
control the FD-OCT imaging system (130) to acquire OCT data (135) of a plurality of portions of the retina such that, for each portion of the plurality of portions of the retina, at least some of the OCT data acquired from the portion is acquired after a respective optical stimulus has been applied to the portion by the optical system (120);
generate, based on the acquired OCT data (135), respective ORG data (150) for each portion of the plurality of portions of the retina which is indicative of the respective response of the portion of the retina to the optical stimulus applied to the portion of the retina;
store, for each portion of the plurality of portions of the retina, the respective ORG data (150) generated from OCT data (135) acquired from the portion in association with a respective indication of a location of the portion on the retina; and
acquire data indicating how measurements of the subject's ability to see optical stimuli that are confined to respective different portions of the retina of the eye (160) at different respective locations on the retina are distributed over the at least a part of the retina of the eye (160).

6. The FD-OCT apparatus (100) according to Claim 5, wherein the controller (140) is arranged to acquire the data which indicates how measurements of the subject's ability to see optical stimuli applied to respective different locations on the retina of the eye (160) are distributed over at least a part of the retina of the eye (160) by:
receiving (S1901) visual field test data acquired from the eye (160) by a visual field-testing device, the visual field test data comprising measurements of the subject's ability to see optical stimuli applied from a plurality of points in the visual field of the eye (160) of the subject; and
determining (S1902), for each point of the plurality of points in the visual field of the eye (160), a respective indication of a corresponding position on the retina that is optically conjugate with the point.

7. The FD-OCT apparatus (100) according to Claim 5, wherein the controller (140) is arranged to acquire the data which indicates how measurements of the subject's ability to see optical stimuli applied to respective different locations on the retina of the eye (160) are distributed over at least a part of the retina of the eye (160) by:
receiving (S1903) indications, provided by the subject, of whether the subject saw the respective optical stimulus applied to each portion of the plurality of portions of the retina by the optical system (120); and
associating (S1904) each of the received indications with a respective indication of a location of the respective portion on the retina.

8. The FD-OCT apparatus (100) according to any of Claims 5 to 7, further comprising a display device (142), wherein controller (140) is further arranged to:
control (S2103) the display device (142), based on at least some of the acquired data, to display a first map (1700) indicative of how the subject's ability to see the optical stimuli is distributed over at least a part of the retina of the eye (160); and
control (S2104) the display device (142), based on at least some of the generated ORG data (150) and positions of the portions of the retina from which the at least some of the ORG data was generated, to display a second map (1800) for comparison with the first map (1700), the second map (1800) being indicative of how the response of the retina to optical stimulus, which is indicated by the at least some of the generated ORG data (150), is distributed over the at least a part of the retina of the eye (160).

9. The FD-OCT apparatus (100) according to Claim 4 or Claim 8, wherein the controller (140) is further arranged to compare data of the first map (1400; 1700) with data of the second map (1500; 1800) and identify, based on the comparison, at least one of:
one or more first regions (1601) of the at least a part of the visual field, indicating that the subject is able to see visual field test optical stimuli in the one or more first regions (1601) of the at least a part of the visual field, and indicating that one or more corresponding regions of the retina provided a physiological response to the optical stimulus which satisfies a predetermined condition;
one or more second regions (1602, 1603) of the at least a part of the visual field, indicating that the subject has an inability to see visual field test optical stimuli in the one or more second regions (1602, 1603) of the at least a part of the visual field, and indicating that one or more corresponding regions of the retina provided a physiological response to the optical stimulus which satisfies the predetermined condition;
one or more third regions (1604) of the at least a part of the visual field, indicating that the subject is able to see visual field test optical stimuli in the one or more third regions (1604) of the at least a part of the visual field, and indicating that one or more corresponding regions of the retina provided a physiological response to the optical stimulus which does not satisfy the predetermined condition; or
one or more fourth regions (1605, 1606, 1607) of the at least a part of the visual field, indicating that the subject has an inability to see visual field test optical stimuli in the one or more fourth regions of the at least a part of the visual field, and indicating that one or more corresponding regions (1605, 1606, 1607) of the retina provided a physiological response to the optical stimulus which does not satisfy the predetermined condition.

10. The FD-OCT apparatus according to any of Claims 1 to 9, wherein
the FD-OCT apparatus (100) further comprises a fixation target (110) arranged to fix a gaze direction of the eye (160),
the optical system (120) is operable to apply the optical stimulus to the retina such that an illumination of the retina by the optical stimulus is confined to a portion of the retina, the optical system (120) being controllable by the controller (140) to vary a location on the retina at which the optical stimulus is to be applied while and comprising a light source (301) arranged to generate light which provides the optical stimulus, and one or more scanning elements (312, 314) arranged to direct the light to the retina,
the controller (140) is further arranged to:
acquire an indicator (141) of a target location on the retina at which the optical stimulus is to be applied by the optical system (120);
use the acquired indicator (141) to control the one or more scanning elements (312, 314) of the optical system (120), while a position of the fixation target (110) relative to the eye (160) remains fixed, to direct the light to a first portion of the retina which is at the target location;
control the FD-OCT imaging system (130) to acquire OCT data (135) of a second portion of the retina, wherein at least a part of the second portion of the retina is disposed in relation to the first portion so as to be stimulated by the applied optical stimulus during acquisition of at least some of the OCT data (135); and
generate ORG data (250) based on the acquired OCT data (135) of the second portion of the retina.

11. The FD-OCT apparatus (100) according to Claim 10, wherein
the FD-OCT imaging system (130, 320) comprises:
an interferometer (322) having a sample arm (324) and a reference arm (325); and
a detector (323) arranged to detect an interference between sample OCT light (Lₒ) propagating along the sample arm (324) after having been scattered from the retina, and reference OCT light (Lᵣ) propagating along the reference arm (325); and
at least one of the one or more scanning elements (213, 314) is further arranged to direct the sample OCT light (Lₒ) toward the second portion of the retina, and the sample OCT light (Lₒ) scattered from the second portion of the retina toward the detector (323).

12. The FD-OCT apparatus according to Claim 10, wherein
the FD-OCT imaging system (120, 361) comprises:
an interferometer (322) having a sample arm (324) and a reference arm (325);
one or more scanning elements (362, 363); and
a detector (323) arranged to detect an interference between sample OCT light (Lₒ) propagating along the sample arm (324) after having been scattered from the retina, and reference OCT light (Lᵣ) propagating along the reference arm (325),
wherein the one or more scanning elements (362, 363) are arranged to direct the sample OCT light (Lₒ) toward the second portion of the retina, and the sample OCT light (Lₒ) scattered from the second portion of the retina toward the detector (323), and
the one or more scanning elements (364, 365) of the optical system (120, 360) are different from the one or more scanning elements (362, 363) of the FD-OCT imaging system (120, 361).

13. A computer-implemented method of controlling a Fourier-domain optical coherence tomography, FD-OCT, apparatus (100) to acquire optoretinography, ORG, data (150) that is indicative of a physiological response of a retina of an eye (160) of a subject to an optical stimulus, the FD-OCT apparatus (100) comprising:
an optical system (120) operable to apply the optical stimulus to the retina; and
an FD-OCT imaging system (130) operable to acquire OCT data (135) by imaging a portion of the retina,
the method comprising:
controlling the FD-OCT imaging system (130) to acquire OCT data (135) of a plurality of portions of the retina such that, for each portion of the plurality of portions of the retina, at least some of the OCT data acquired from the portion is acquired after a respective optical stimulus has been applied to the portion by the optical system (120);
generating, based on the acquired OCT data (135), respective ORG data (150) for each portion of the plurality of portions of the retina which is indicative of the respective response of the portion of the retina to the optical stimulus applied to the portion of the retina; and
storing, for each portion of the plurality of portions of the retina, the respective ORG data (150) generated for the portion in association with a respective indication of a position in a visual field of the eye (160) of a point that is optically conjugate with a corresponding position of the portion on the retina.

14. A computer-implemented method of controlling a Fourier-domain optical coherence tomography, FD-OCT, apparatus (100) to acquire optoretinography, ORG, data (150) that is indicative of a physiological response of a retina of an eye (160) of a subject to an optical stimulus, the FD-OCT apparatus (100) comprising:
an optical system (120) operable to apply the optical stimulus to the retina; and
an FD-OCT imaging system (130) operable to acquire OCT data (135) by imaging a portion of the retina,
the method comprising:
controlling the FD-OCT imaging system (130) to acquire OCT data (135) of a plurality of portions of the retina such that, for each portion of the plurality of portions of the retina, at least some of the OCT data acquired from the portion is acquired after a respective optical stimulus has been applied to the portion by the optical system (120);
generating, based on the acquired OCT data (135), respective ORG data (150) for each portion of the plurality of portions of the retina which is indicative of the respective response of the portion of the retina to the optical stimulus applied to the portion of the retina;
storing, for each portion of the plurality of portions of the retina, the respective ORG data (150) generated from OCT data (135) acquired from the portion in association with a respective indication of a location of the portion on the retina; and
acquiring data indicating how measurements of the subject's ability to see optical stimuli that are confined to respective different portions of the retina of the eye (160) at different respective locations on the retina are distributed over the at least a part of the retina of the eye (160).

15. A computer program comprising computer-readable instructions which, when executed by a computer, cause the computer to perform a method according to at least one of Claims 13 and 14.

## Patentansprüche

1. Fourier-Bereichs-Optische-Kohärenz-Tomographie, FD-OCT, - Vorrichtung (100), die eingerichtet ist zum Erfassen von Optoretinographie, ORG, -Daten (150), die eine physiologische Antwort einer Retina eines Auges (160) einer Zielperson auf einen optischen Stimulus anzeigen, wobei die FD-OCT-Vorrichtung (100) umfasst:
ein optisches System (120), das betreibbar ist zum Anlegen des optischen Stimulus an die Retina;
ein FD-OCT-Abbildungssystem (130), das betreibbar ist zum Erfassen von OCT-Daten (135) durch Abbilden eines Abschnitts der Retina; und
eine Steuerung (140), die eingerichtet ist zum:
Steuern des FD-OCT-Abbildungssystems (130) zum Erfassen von OCT-Daten (135) von einer Vielzahl von Abschnitten der Retina, so dass für jeden Abschnitt der Vielzahl von Abschnitten der Retina zumindest ein Teil der OCT-Daten, die von dem Abschnitt erfasst wurden, erfasst wird, nachdem ein entsprechender optischer Stimulus durch das optische System (120) auf den Abschnitt angewendet wurde;
Erzeugen, auf Grundlage der erfassten OCT-Daten (135), jeweiliger ORG-Daten (150) für jeden Abschnitt der Vielzahl von Abschnitten der Retina, die die jeweilige Antwort des Abschnitts der Retina auf den optischen Stimulus angeben, der auf den Abschnitt der Retina angewendet wird; und
Speichern, für jeden Abschnitt der Vielzahl von Abschnitten der Retina, der jeweiligen ORG-Daten (150), die für den Abschnitt in Zusammenhang mit einer jeweiligen Angabe einer Position in einem Sichtfeld des Auges (160) eines Punktes, der optisch mit einer entsprechenden Position des Abschnitts auf der Retina konjugiert ist, erzeugt wurden.

2. FD-OCT-Vorrichtung (100) gemäß Anspruch 1, wobei die Steuerung (140) ferner eingerichtet ist zum Bestimmen (S1101) einer jeweiligen Angabe einer Position auf der Retina jedes Abschnitts der Vielzahl von Abschnitten der Retina, an dem OCT-Bilddaten (135) durch das FD-OCT-Abbildungssystem (130) zu erfassen sind, durch Bestimmen, für jeden Punkt einer Vielzahl von Punkten im Sichtfeld des Auges (160), einer jeweiligen Angabe einer entsprechenden Position auf der Retina, die optisch mit dem Punkt konjugiert ist.

3. FD-OCT-Vorrichtung gemäß Anspruch 1, wobei die Steuerung (140) ferner eingerichtet ist zum:
Bestimmen (S1202) einer jeweiligen Angabe einer Position auf der Retina jedes Abschnitts der Vielzahl von Abschnitten der Retina, an dem OCT-Daten (135) durch das FD-OCT-Abbildungssystem (130) auf Grundlage eines Bildes der Retina des Auges (160) erfasst werden sollen; und
Bestimmen (S1203), für jeden Abschnitt der Vielzahl von Abschnitten der Retina, die jeweilige Angabe der Position im Sichtfeld des Auges (160) des Punktes, der optisch mit der Position des Abschnitts auf der Retina konjugiert ist.

4. FD-OCT-Vorrichtung gemäß einem der Ansprüche 1 bis 3, ferner umfassend eine Anzeigevorrichtung (142), wobei die Steuerung (140) ferner eingerichtet ist zum:
Erfassen (S1401) von Daten, die angeben, wie Messungen der Fähigkeit der Zielperson, optische Stimuli zu sehen, die auf jeweilige unterschiedliche Abschnitte der Retina des Auges (160) an unterschiedlichen jeweiligen Positionen auf der Retina begrenzt sind, über mindestens einen Teil eines Sichtfeldes des Auges (160) verteilt sind;
Steuern (S1402) der Anzeigevorrichtung (142) auf Grundlage von zumindest einigen der erfassten Daten, um eine erste Karte (1400) anzuzeigen, die angibt, wie die Fähigkeit der Zielperson, die optischen Stimuli zu sehen, über den zumindest einen Teil des Sichtfeldes des Auges (160) verteilt ist; und
Steuern (S1403) der Anzeigevorrichtung (142) auf Grundlage mindestens einiger der gespeicherten ORG-Daten (150) und der zugehörigen gespeicherten Angaben, um eine zweite Karte (1500) zum Vergleich mit der ersten Karte (1400) anzuzeigen, wobei die zweite Karte (1500) angibt, wie die physiologische Antwort der Retina auf den optischen Stimulus, der durch mindestens einige der gespeicherten ORG-Daten (150) angegeben wird, über den mindestens einen Teil des Sichtfelds des Auges (160) verteilt ist.

5. Fourier-Bereichs-Optische-Kohärenz-Tomographie, FD-OCT, - Vorrichtung (100), die eingerichtet ist zum Erfassen von Optoretinographie, ORG, -Daten (150), die eine physiologische Antwort einer Retina eines Auges (160) einer Zielperson auf einen optischen Stimulus anzeigen, wobei die FD-OCT-Vorrichtung (100) umfasst:
ein optisches System (120), das betreibbar ist zum Anlegen des optischen Stimulus an die Retina;
ein FD-OCT-Abbildungssystem (130), das betreibbar ist zum Erfassen von OCT-Daten (135) durch Abbilden eines Abschnitts der Retina; und
eine Steuerung (140), die eingerichtet ist zum:
Steuern des FD-OCT-Abbildungssystems (130) zum Erfassen von OCT-Daten (135) von einer Vielzahl von Abschnitten der Retina, so dass für jeden Abschnitt der Vielzahl von Abschnitten der Retina zumindest ein Teil der OCT-Daten, die von dem Abschnitt erfasst wurden, erfasst wird, nachdem ein entsprechender optischer Stimulus durch das optische System (120) auf den Abschnitt angewendet wurde;
Erzeugen, auf Grundlage der erfassten OCT-Daten (135), jeweiliger ORG-Daten (150) für jeden Abschnitt der Vielzahl von Abschnitten der Retina, die die jeweilige Antwort des Abschnitts der Retina auf den optischen Stimulus angeben, der auf den Abschnitt der Retina angewendet wird;
Speichern, für jeden Abschnitt der Vielzahl von Abschnitten der Retina, der jeweiligen ORG-Daten (150), die aus den von dem Abschnitt erfassten OCT-Daten (135) erzeugt wurden, in Zusammenhang mit einer jeweiligen Angabe einer Position des Abschnitts auf der Retina; und
Erfassen von Daten, die angeben, wie Messungen der Fähigkeit der Zielperson, optische Stimuli zu sehen, die auf jeweilige unterschiedliche Abschnitte der Retina des Auges (160) an unterschiedlichen jeweiligen Positionen auf der Retina begrenzt sind, über mindestens einen Teil der Retina des Auges (160) verteilt sind.

6. FD-OCT Vorrichtung (100) gemäß Anspruch 5, wobei die Steuerung (140) eingerichtet ist zum Erfassen der Daten, die angeben, wie Messungen der Fähigkeit der Zielperson, optische Stimuli zu sehen, die auf entsprechende unterschiedliche Positionen auf der Retina des Auges (160) aufgebracht werden, über mindestens einen Teil der Retina des Auges (160) verteilt sind, durch:
Empfangen (S1901) von Sehfeldtestdaten, die von dem Auge (160) durch eine Sehfeldtestvorrichtung erfasst werden, wobei die Sehfeldtestdaten Messungen der Fähigkeit der Zielperson umfassen, optische Stimuli zu sehen, die von einer Vielzahl von Punkten im Sichtfeld des Auges (160) der Zielperson aufgebracht werden; und
Bestimmen (S1902), für jeden Punkt der Vielzahl von Punkten im Sichtfeld des Auges (160), eine jeweilige Angabe einer entsprechenden Position auf der Retina, die optisch mit dem Punkt konjugiert ist.

7. FD-OCT-Vorrichtung (100) gemäß Anspruch 5, wobei die Steuerung (140) eingerichtet ist zum Erfassen der Daten, die angeben, wie Messungen der Fähigkeit der Zielperson, optische Stimuli zu sehen, die auf entsprechende unterschiedliche Positionen auf der Retina des Auges (160) aufgebracht werden, über mindestens einen Teil der Retina des Auges (160) verteilt sind, durch:
Empfangen (S1903) von Angaben, die von der Zielperson bereitgestellt werden, ob die Zielperson den jeweiligen optischen Stimulus gesehen hat, der auf jeden Abschnitt der Vielzahl von Abschnitten der Retina durch das optische System (120) aufgebracht wurde; und
Assoziieren (S1904) jeder der empfangenen Angaben mit einer entsprechenden Angabe einer Position des jeweiligen Abschnitts auf der Retina.

8. FD-OCT-Vorrichtung (100) gemäß einem der Ansprüche 5 bis 7, ferner umfassend eine Anzeigevorrichtung (142), wobei die Steuerung (140) ferner eingerichtet ist zum:
Steuern (S2103) der Anzeigevorrichtung (142) auf Grundlage mindestens einiger der erfassten Daten, um eine erste Karte (1700) anzuzeigen, die angibt, wie die Fähigkeit der Zielperson, die optischen Stimuli zu sehen, über mindestens einen Teil der Retina des Auges (160) verteilt ist; und
Steuern (S2104) der Anzeigevorrichtung (142) auf Grundlage von zumindest einigen der erzeugten ORG-Daten (150) und Positionen der Abschnitte der Retina, von denen zumindest einige der ORG-Daten erzeugt wurden, um eine zweite Karte (1800) zum Vergleich mit der ersten Karte (1700) anzuzeigen, wobei die zweite Karte (1800) angibt, wie die Antwort der Retina auf den optischen Stimulus, die durch die zumindest einige der erzeugten ORG-Daten (150) angegeben wird, über den zumindest einen Teil der Retina des Auges (160) verteilt ist.

9. FD-OCT-Vorrichtung (100) gemäß Anspruch 4 oder Anspruch 8, wobei die Steuerung (140) ferner eingerichtet ist zum Vergleichen von Daten der ersten Karte (1400; 1700) mit Daten der zweiten Karte (1500; 1800) und zum Identifizieren, auf Grundlage des Vergleichs, mindestens eines von:
eine oder mehrere erste Regionen (1601) des mindestens einen Teils des Sichtfelds, die angeben, dass die Zielperson in der Lage ist, optische Stimuli des Sichtfeldtests in der einen oder den mehreren ersten Regionen (1601) des mindestens einen Teils des Sichtfelds zu sehen, und angeben, dass eine oder mehrere entsprechende Regionen der Retina eine physiologische Antwort auf den optischen Stimulus bereitstellten, die eine vorbestimmte Bedingung erfüllt;
eine oder mehrere zweite Regionen (1602, 1603) des mindestens einen Teils des Sichtfelds, die angeben, dass die Zielperson nicht in der Lage ist, optische Stimuli des Sichtfeldtests in der einen oder den mehreren zweiten Regionen (1602, 1603) des mindestens einen Teils des Sichtfelds zu sehen, und die angeben, dass eine oder mehrere entsprechende Regionen der Retina eine physiologische Antwort auf den optischen Stimulus bereitstellten, die die vorbestimmte Bedingung erfüllt;
eine oder mehrere dritte Regionen (1604) des mindestens einen Teils des Sichtfelds, die angeben, dass die Zielperson in der Lage ist, optische Stimuli für den Sichtfeldtest in der einen oder den mehreren dritten Regionen (1604) des mindestens einen Teils des Sichtfelds zu sehen, und angeben, dass eine oder mehrere entsprechende Regionen der Retina eine physiologische Antwort auf den optischen Stimulus bereitstellten, die die vorbestimmte Bedingung nicht erfüllt; oder
eine oder mehrere vierte Regionen (1605, 1606, 1607) des mindestens einen Teils des Sichtfeldes, die angeben, dass die Zielperson nicht in der Lage ist, optische Stimuli des Sichtfeldtests in der einen oder den mehreren vierten Regionen des mindestens einen Teils des Sichtfeldes zu sehen, und die angeben, dass eine oder mehrere entsprechende Regionen (1605, 1606, 1607) der Retina eine physiologische Antwort auf den optischen Stimulus bereitstellten, die die vorbestimmte Bedingung nicht erfüllt.

10. FD-OCT-Vorrichtung gemäß einem der Ansprüche 1 bis 9, wobei
die FD-OCT Vorrichtung (100) ferner ein Fixierungsziel (110) umfasst, das eingerichtet ist zum Fixieren einer Blickrichtung des Auges (160),
das optische System (120) betreibbar ist zum Aufbringen des optischen Stimulus auf die Retina, so dass eine Beleuchtung der Retina durch den optischen Stimulus auf einen Abschnitt der Retina begrenzt ist, wobei das optische System (120) durch die Steuerung (140) steuerbar ist, um eine Position auf der Retina zu verändern, an der der optische Stimulus aufgebracht werden soll, während es eine Lichtquelle (301) umfasst, die eingerichtet ist zum Erzeugen von Licht, das den optischen Stimulus liefert, und ein oder mehrere Abtastelemente (312, 314), die eingerichtet sind zum Richten des Lichts auf die Retina,
die Steuerung (140) ferner eingerichtet ist zum:
Erfassen eines Positionsgebers (141) für eine Position auf der Retina, auf die der optische Stimulus durch das optische System (120) angewendet werden soll;
Verwenden des erfassten Indikators (141) zum Steuern des einen oder der mehreren Abtastelemente (312, 314) des optischen Systems (120), während eine Position des Fixierziels (110) relativ zum Auge (160) fixiert bleibt, um das Licht auf einen ersten Abschnitt der Retina zu richten, der sich an der Zielposition befindet;
Steuern des FD-OCT-Abbildungssystems (130 zum Erfassen von OCT-Daten (135) eines zweiten Abschnitts der Retina, wobei mindestens ein Teil des zweiten Abschnitts der Retina in Bezug auf den ersten Abschnitt angeordnet ist, um durch den aufgebrachten optischen Stimulus während der Erfassung von mindestens einigen der OCT-Daten (135) stimuliert zu werden; und
Erzeugen von ORG-Daten (250) auf Grundlage der erfassten OCT-Daten (135) des zweiten Abschnitts der Retina.

11. FD-OCT-Vorrichtung (100) gemäß Anspruch 10, wobei
das FD-OCT Abbildungssystem (130, 320) umfasst:
ein Interferometer (322) mit einem Probenarm (324) und einem Referenzarm (325); und
einen Detektor (323), der eingerichtet ist zum Detektieren einer Interferenz zwischen Proben-OCT-Licht (Lₒ), das sich entlang des Probenarms (324) ausbreitet, nachdem es von der Retina gestreut wurde, und Referenzlicht (Lᵣ), das sich entlang des Referenzarms (325) ausbreitet; und
mindestens eines der einen oder mehreren Abtastelemente (213, 314), das ferner eingerichtet ist zum Richten des Proben-OCT-Lichts (Lₒ) auf den zweiten Abschnitt der Retina und des Proben-OCT-Lichts (Lₒ), das von dem zweiten Abschnitt der Retina gestreut wird, auf den Detektor (323).

12. FD-OCT-Vorrichtung gemäß Anspruch 10, wobei
das FD-OCT Abbildungssystem (120, 361) umfasst:
ein Interferometer (322) mit einem Probenarm (324) und einem Referenzarm (325);
ein oder mehrere Abtastelemente (362, 363); und
einen Detektor (323), der eingerichtet ist zum Detektieren einer Interferenz zwischen Proben-OCT-Licht (Lₒ), das sich entlang des Probenarms (324) ausbreitet, nachdem es von der Retina gestreut wurde, und Referenz-OCT-Licht (Lᵣ), das sich entlang des Referenzarms (325) ausbreitet,
wobei das eine oder die mehreren Abtastelemente (362, 363) eingerichtet sind zum Richten des Proben-OCT-Lichts (Lₒ) auf den zweiten Abschnitt der Retina und des Proben-OCT-Lichts (Lₒ), das von dem zweiten Abschnitt der Retina gestreut wird, auf den Detektor (323), und
das eine oder die mehreren Abtastelemente (364, 365) des optischen Systems (120, 360) von dem einen oder den mehreren Abtastelementen (362, 363) des FD-OCT-Abbildungssystems (120, 361) verschieden sind.

13. Computerimplementiertes Verfahren zum Steuern einer Fourier-Bereichs-Optische-Kohärenz-Tomographie, FD-OCT, - Vorrichtung (100) zum Erfassen von Optoretinographie, ORG, - Daten (150), die eine physiologische Antwort einer Retina eines Auges (160) einer Zielperson auf einen optischen Stimulus anzeigen, wobei die FD-OCT-Vorrichtung (100) umfasst:
ein optisches System (120), das betreibbar ist zum Anlegen des optischen Stimulus an die Retina; und
ein FD-OCT-Abbildungssystem (130), das betreibbar ist zum Erfassen von OCT-Daten (135) durch Abbilden eines Abschnitts der Retina,
wobei das Verfahren umfasst:
Steuern des FD-OCT-Abbildungssystems (130) zum Erfassen von OCT-Daten (135) von einer Vielzahl von Abschnitten der Retina, so dass für jeden Abschnitt der Vielzahl von Abschnitten der Retina zumindest ein Teil der OCT-Daten, die von dem Abschnitt erfasst wurden, erfasst wird, nachdem ein entsprechender optischer Stimulus durch das optische System (120) auf den Abschnitt angewendet wurde;
Erzeugen, auf Grundlage der erfassten OCT-Daten (135), jeweiliger ORG-Daten (150) für jeden Abschnitt der Vielzahl von Abschnitten der Retina, die die jeweilige Antwort des Abschnitts der Retina auf den optischen Stimulus angeben, der auf den Abschnitt der Retina angewendet wird; und
Speichern, für jeden Abschnitt der Vielzahl von Abschnitten der Retina, der jeweiligen ORG-Daten (150), die für den Abschnitt in Zusammenhang mit einer jeweiligen Angabe einer Position in einem Sichtfeld des Auges (160) eines Punktes, der optisch mit einer entsprechenden Position des Abschnitts auf der Retina konjugiert ist, erzeugt wurden.

14. Computerimplementiertes Verfahren zum Steuern einer Fourier-Bereichs-Optische-Kohärenz-Tomographie, FD-OCT, - Vorrichtung (100) zum Erfassen von Optoretinographie, ORG, - Daten (150), die eine physiologische Antwort einer Retina eines Auges (160) einer Zielperson auf einen optischen Stimulus anzeigen, wobei die FD-OCT-Vorrichtung (100) umfasst:
ein optisches System (120), das betreibbar ist zum Anlegen des optischen Stimulus an die Retina;
ein FD-OCT-Abbildungssystem (130), das betreibbar ist zum Erfassen von OCT-Daten (135) durch Abbilden eines Abschnitts der Retina,
wobei das Verfahren umfasst:
Steuern des FD-OCT-Abbildungssystems (130) zum Erfassen von OCT-Daten (135) von einer Vielzahl von Abschnitten der Retina, so dass für jeden Abschnitt der Vielzahl von Abschnitten der Retina zumindest ein Teil der OCT-Daten, die von dem Abschnitt erfasst wurden, erfasst wird, nachdem ein entsprechender optischer Stimulus durch das optische System (120) auf den Abschnitt angewendet wurde;
Erzeugen, auf Grundlage der erfassten OCT-Daten (135), jeweiliger ORG-Daten (150) für jeden Abschnitt der Vielzahl von Abschnitten der Retina, die die jeweilige Antwort des Abschnitts der Retina auf den optischen Stimulus angeben, der auf den Abschnitt der Retina angewendet wird;
Speichern, für jeden Abschnitt der Vielzahl von Abschnitten der Retina, der jeweiligen ORG-Daten (150), die aus den von dem Abschnitt erfassten OCT-Daten (135) erzeugt wurden, in Zusammenhang mit einer jeweiligen Angabe einer Position des Abschnitts auf der Retina; und
Erfassen von Daten, die angeben, wie Messungen der Fähigkeit der Zielperson, optische Stimuli zu sehen, die auf jeweilige unterschiedliche Abschnitte der Retina des Auges (160) an unterschiedlichen jeweiligen Positionen auf der Retina begrenzt sind, über mindestens einen Teil der Retina des Auges (160) verteilt sind.

15. Computerprogramm, das computerlesbare Anweisungen umfasst, die, wenn sie von einem Computer ausgeführt werden, den Computer veranlassen, ein Verfahren gemäß mindestens einem der Ansprüche 13 und 14 auszuführen.

## Revendications

1. Appareil de tomographie en cohérence optique dans le domaine de Fourier, TCO-DF, (100) agencé pour acquérir des données de rétinographie optique, RGO, (150) qui sont indicatives d'une réponse physiologique d'une rétine d'un œil (160) d'un sujet à un stimulus optique, l'appareil TCO-DF (100) comprenant :
un système optique (120) pouvant fonctionner pour appliquer le stimulus optique à la rétine ;
un système d'imagerie TCO-DF (130) pouvant fonctionner pour acquérir des données TCO (135) en imageant une partie de la rétine ; et
un contrôleur (140) agencé pour :
commander le système d'imagerie TCO-DF (130) pour acquérir des données TCO (135) d'une pluralité de parties de la rétine de sorte que, pour chaque partie de la pluralité de parties de la rétine, au moins certaines des données TCO acquises à partir de la partie soient acquises après qu'un stimulus optique respectif a été appliqué à la partie par le système optique (120) ;
générer, sur la base des données TCO acquises (135), des données RGO respectives (150) pour chaque partie de la pluralité de parties de la rétine, qui sont indicatives de la réponse respective de la partie de la rétine au stimulus optique appliqué à la partie de la rétine ; et
stocker, pour chaque partie de la pluralité de parties de la rétine, les données RGO respectives (150) générées pour la partie en association avec une indication respective d'une position dans un champ visuel de l'œil (160) d'un point qui est optiquement conjugué avec une position correspondante de la partie sur la rétine.

2. Appareil TCO-DF (100) selon la revendication 1, dans lequel le contrôleur (140) est en outre agencé pour déterminer (S1101) une indication respective d'une position sur la rétine de chaque partie de la pluralité de parties de la rétine au niveau de laquelle des données TCO (135) doivent être acquises par le système d'imagerie TCO-DF (130) en déterminant, pour chaque point d'une pluralité de points dans le champ visuel de l'oeil (160), une indication respective d'une position correspondante sur la rétine qui est optiquement conjuguée avec le point.

3. Appareil TCO-DF selon la revendication 1, dans lequel le contrôleur (140) est en outre agencé pour :
déterminer (S1202) une indication respective d'une position sur la rétine de chaque partie de la pluralité de parties de la rétine à laquelle les données TCO (135) doivent être acquises par le système d'imagerie TCO-DF (130) sur la base d'une image de la rétine de l'œil (160) ; et
déterminer (S1203), pour chaque partie de la pluralité de parties de la rétine, l'indication respective de la position dans le champ visuel de l'œil (160) du point qui est optiquement conjugué avec la position de la partie sur la rétine.

4. Appareil TCO-DF selon l'une quelconque des revendications 1 à 3, comprenant en outre un dispositif d'affichage (142), dans lequel le contrôleur (140) est en outre agencé pour :
acquérir (S1401) des données indiquant comment des mesures de la capacité du sujet à voir des stimuli optiques appliqués de manière confinée à différentes parties respectives de la rétine de l'œil (160) à différents emplacements respectifs sur la rétine sont réparties sur au moins une partie d'un champ visuel de l'œil (160) ;
commander (S1402) le dispositif d'affichage (142), sur la base d'au moins certaines des données acquises, afin d'afficher une première carte (1400) indiquant comment la capacité du sujet à voir les stimuli optiques est répartie sur la au moins une partie du champ visuel de l'œil (160) ; et
commander (S1403) le dispositif d'affichage (142), sur la base d'au moins certaines des données RGO stockées (150) et des indications stockées associées, pour afficher une seconde carte (1500) à des fins de comparaison avec la première carte (1400), la seconde carte (1500) indiquant comment la réponse physiologique de la rétine au stimulus optique indiqué par au moins certaines des données RGO stockées (150) est répartie sur la au moins une partie du champ visuel de l'œil (160) .

5. Appareil de tomographie en cohérence optique dans le domaine de Fourier, TCO-DF (100), agencé pour acquérir des données de rétinographie optique, RGO (150), qui sont indicatives d'une réponse physiologique d'une rétine d'un œil (160) d'un sujet à un stimulus optique, l'appareil TCO-DF (100) comprenant :
un système optique (120) pouvant fonctionner pour appliquer le stimulus optique à la rétine ;
un système d'imagerie TCO-DF (130) pouvant fonctionner pour acquérir des données TCO (135) en imageant une partie de la rétine ; et
un contrôleur (140) agencé pour :
commander le système d'imagerie TCO-DF (130) afin d'acquérir des données TCO (135) d'une pluralité de parties de la rétine de sorte que, pour chaque partie de la pluralité de parties de la rétine, au moins certaines des données TCO acquises à partir de la partie soient acquises après qu'un stimulus optique respectif a été appliqué à la partie par le système optique (120) ;
générer, sur la base des données TCO acquises (135), des données RGO respectives (150) pour chaque partie de la pluralité de parties de la rétine, qui sont indicatives de la réponse respective de la partie de la rétine au stimulus optique appliqué à la partie de la rétine ;
stocker, pour chaque partie de la pluralité de parties de la rétine, les données RGO respectives (150) générées à partir de données TCO (135) acquises à partir de la partie en association avec une indication respective d'un emplacement de la partie sur la rétine ; et
acquérir des données indiquant comment des mesures de la capacité du sujet à voir des stimuli optiques qui sont confinés à différentes parties respectives de la rétine de l'œil (160) à différents emplacements respectifs sur la rétine sont réparties sur la au moins une partie de la rétine de l'œil (160).

6. Appareil TCO-DF (100) selon la revendication 5, dans lequel le contrôleur (140) est agencé pour acquérir les données qui indiquent comment des mesures de la capacité du sujet à voir des stimuli optiques appliqués à différents emplacements respectifs sur la rétine de l'oeil (160) sont réparties sur au moins une partie de la rétine de l'oeil (160) en :
recevant (S1901) des données de test de champ visuel acquises à partir de l'œil (160) par un dispositif de test du champ visuel, les données de test de champ visuel comprenant des mesures de la capacité du sujet à voir des stimuli optiques appliqués à partir d'une pluralité de points dans le champ visuel de l'œil (160) du sujet ; et
déterminant (S1902), pour chaque point de la pluralité de points dans le champ visuel de l'œil (160), une indication respective d'une position correspondante sur la rétine qui est optiquement conjuguée avec le point.

7. Appareil TCO-DF (100) selon la revendication 5, dans lequel le contrôleur (140) est agencé pour acquérir les données qui indiquent comment des mesures de la capacité du sujet à voir des stimuli optiques appliqués à différents emplacements respectifs sur la rétine de l'oeil (160) sont réparties sur au moins une partie de la rétine de l'oeil (160) en :
recevant (S1903) des indications, fournies par le sujet, indiquant si le sujet a vu le stimulus optique respectif appliqué à chaque partie de la pluralité de parties de la rétine par le système optique (120) ; et
associant (S1904) chacune des indications reçues à une indication respective d'un emplacement de la partie respective sur la rétine.

8. Appareil TCO-DF (100) selon l'une quelconque des revendications 5 à 7, comprenant en outre un dispositif d'affichage (142), dans lequel le contrôleur (140) est en outre agencé pour :
commander (S2103) le dispositif d'affichage (142), sur la base d'au moins certaines des données acquises, afin d'afficher une première carte (1700) indiquant comment la capacité du sujet à voir les stimuli optiques est répartie sur au moins une partie de la rétine de l'œil (160) ; et
commander (S2104) le dispositif d'affichage (142), sur la base d'au moins certaines des données RGO générées (150) et des positions des parties de la rétine à partir desquelles les au moins certaines des données RGO ont été générées, afin d'afficher une seconde carte (1800) à des fins de comparaison avec la première carte (1700), la seconde carte (1800) indiquant comment la réponse de la rétine au stimulus optique, qui est indiquée par les au moins certaines des données RGO générées (150), est répartie sur la au moins une partie de la rétine de l'œil (160).

9. Appareil TCO-DF (100) selon la revendication 4 ou la revendication 8, dans lequel le contrôleur (140) est en outre agencé pour comparer des données de la première carte (1400 ; 1700) avec des données de la seconde carte (1500 ; 1800) et identifier, sur la base de la comparaison, au moins l'un des éléments suivants :
une ou plusieurs premières régions (1601) de la au moins une partie du champ visuel, indiquant que le sujet est capable de voir des stimuli optiques de test de champ visuel dans la ou les premières régions (1601) de la au moins une partie du champ visuel, et indiquant qu'une ou plusieurs régions correspondantes de la rétine ont fourni une réponse physiologique au stimulus optique qui satisfait une condition prédéterminée ;
une ou plusieurs secondes régions (1602, 1603) de la au moins une partie du champ visuel, indiquant que le sujet est incapable de voir les stimuli optiques de test de champ visuel dans la ou les secondes régions (1602, 1603) de la au moins une partie du champ visuel, et indiquant qu'une ou plusieurs régions correspondantes de la rétine ont fourni une réponse physiologique au stimulus optique qui satisfait la condition prédéterminée ;
une ou plusieurs troisièmes régions (1604) de la au moins une partie du champ visuel, indiquant que le sujet est capable de voir les stimuli optiques de test de champ visuel dans la ou les troisièmes régions (1604) de la au moins une partie du champ visuel, et indiquant qu'une ou plusieurs régions correspondantes de la rétine ont fourni une réponse physiologique au stimulus optique qui ne satisfait pas à la condition prédéterminée ; ou
une ou plusieurs quatrièmes régions (1605, 1606, 1607) de la au moins une partie du champ visuel, indiquant que le sujet est incapable de voir les stimuli optiques de test de champ visuel dans la ou les quatrièmes régions de la au moins une partie du champ visuel, et indiquant qu'une ou plusieurs régions correspondantes (1605, 1606, 1607) de la rétine ont fourni une réponse physiologique au stimulus optique qui ne satisfait pas à la condition prédéterminée.

10. Appareil TCO-DF selon l'une quelconque des revendications 1 à 9, dans lequel
l'appareil TCO-DF (100) comprend en outre une cible de fixation (110) agencée pour fixer une direction de regard de l'œil (160),
le système optique (120) pouvant fonctionner pour appliquer le stimulus optique à la rétine de sorte qu'une illumination de la rétine par le stimulus optique soit confinée à une partie de la rétine, le système optique (120) pouvant être commandé par le contrôleur (140) pour faire varier un emplacement sur la rétine auquel le stimulus optique doit être appliqué, et comprenant une source lumineuse (301) agencée pour générer de la lumière qui fournit le stimulus optique, et un ou plusieurs éléments de balayage (312, 314) agencés pour diriger la lumière vers la rétine,
le contrôleur (140) étant en outre agencé pour :
acquérir un indicateur (141) d'un emplacement cible sur la rétine auquel le stimulus optique doit être appliqué par le système optique (120) ;
utiliser l'indicateur acquis (141) pour commander le ou les éléments de balayage (312, 314) du système optique (120), tandis qu'une position de la cible de fixation (110) par rapport à l'œil (160) reste fixe, afin de diriger la lumière vers une première partie de la rétine qui se trouve à l'emplacement cible ;
commander le système d'imagerie TCO-DF (130) pour acquérir des données TCO (135) d'une seconde partie de la rétine, dans lequel au moins une partie de la seconde partie de la rétine est disposée par rapport à la première partie de manière à être stimulée par le stimulus optique appliqué pendant l'acquisition d'au moins certaines des données TCO (135) ; et
générer des données RGO (250) sur la base des données TCO acquises (135) de la seconde partie de la rétine.

11. Appareil TCO-DF (100) selon la revendication 10, dans lequel
le système d'imagerie TCO-DF (130, 320) comprend :
un interféromètre (322) ayant un bras d'échantillonnage (324) et un bras de référence (325) ; et
un détecteur (323) agencé pour détecter une interférence entre de la lumière TCO d'échantillonnage (Lₒ) se propageant le long du bras d'échantillonnage (324) après avoir été diffusée par la rétine, et de la lumière TCO de référence (Lᵣ) se propageant le long du bras de référence (325) ; et
au moins un du ou des éléments de balayage (213, 314) est en outre agencé pour diriger la lumière TCO d'échantillonnage (Lₒ) vers la seconde partie de la rétine, et la lumière TCO d'échantillonnage (Lₒ) diffusée par la seconde partie de la rétine vers le détecteur (323).

12. Appareil TCO-DF selon la revendication 10, dans lequel
le système d'imagerie TCO-DF (120, 361) comprend :
un interféromètre (322) ayant un bras d'échantillonnage (324) et un bras de référence (325) ;
un ou plusieurs éléments de balayage (362, 363) ; et
un détecteur (323) agencé pour détecter une interférence entre de la lumière TCO d'échantillonnage (Lₒ) se propageant le long du bras d'échantillonnage (324) après avoir été diffusée par la rétine, et de la lumière TCO de référence (Lᵣ) se propageant le long du bras de référence (325),
dans lequel le ou les éléments de balayage (362, 363) sont agencés pour diriger la lumière TCO d'échantillonnage (Lₒ) vers la seconde partie de la rétine, et la lumière TCO d'échantillonnage (Lₒ) diffusée par la seconde partie de la rétine vers le détecteur (323), et
le ou les éléments de balayage (364, 365) du système optique (120, 360) sont différents du ou des éléments de balayage (362, 363) du système d'imagerie TCO-DF (120, 361).

13. Procédé mis en œuvre par ordinateur pour commander un appareil de tomographie en cohérence optique dans le domaine de Fourier, TCO-DF (100), afin d'acquérir des données de rétinographie optique, RGO (150), qui sont indicatives d'une réponse physiologique d'une rétine d'un œil (160) d'un sujet à un stimulus optique, l'appareil TCO-DF (100) comprenant :
un système optique (120) pouvant fonctionner pour appliquer le stimulus optique à la rétine ; et
un système d'imagerie TCO-DF (130) pouvant fonctionner pour acquérir des données TCO (135) en imageant une partie de la rétine,
le procédé comprenant :
commander le système d'imagerie TCO-DF (130) pour acquérir des données TCO (135) d'une pluralité de parties de la rétine de sorte que, pour chaque partie de la pluralité de parties de la rétine, au moins certaines des données TCO acquises à partir de la partie soient acquises après qu'un stimulus optique respectif a été appliqué à la partie par le système optique (120) ;
générer, sur la base des données TCO acquises (135), des données RGO respectives (150) pour chaque partie de la pluralité de parties de la rétine, qui sont indicatives de la réponse respective de la partie de la rétine au stimulus optique appliqué à la partie de la rétine ; et
stocker, pour chaque partie de la pluralité de parties de la rétine, les données RGO respectives (150) générées pour la partie en association avec une indication respective d'une position dans un champ visuel de l'œil (160) d'un point qui est optiquement conjugué avec une position correspondante de la partie sur la rétine.

14. Procédé mis en oeuvre par ordinateur pour commander un appareil de tomographie en cohérence optique dans le domaine de Fourier, TCO-DF (100), afin d'acquérir des données de rétinographie optique, RGO (150), qui sont indicatives d'une réponse physiologique d'une rétine d'un oeil (160) d'un sujet à un stimulus optique, l'appareil TCO-DF (100) comprenant :
un système optique (120) pouvant fonctionner pour appliquer le stimulus optique à la rétine ; et
un système d'imagerie TCO-DF (130) pouvant fonctionner pour acquérir des données TCO (135) en imageant une partie de la rétine,
le procédé comprenant :
commander le système d'imagerie TCO-DF (130) pour acquérir des données TCO (135) d'une pluralité de parties de la rétine de sorte que, pour chaque partie de la pluralité de parties de la rétine, au moins certaines des données TCO acquises à partir de la partie soient acquises après qu'un stimulus optique respectif a été appliqué à la partie par le système optique (120) ;
générer, sur la base des données TCO acquises (135), des données RGO respectives (150) pour chaque partie de la pluralité de parties de la rétine, qui sont indicatives de la réponse respective de la partie de la rétine au stimulus optique appliqué à la partie de la rétine ;
stocker, pour chaque partie de la pluralité de parties de la rétine, les données RGO respectives (150) générées à partir de données TCO (135) acquises à partir de la partie en association avec une indication respective d'un emplacement de la partie sur la rétine ; et
acquérir des données indiquant comment des mesures de la capacité du sujet à voir des stimuli optiques qui sont confinés à différentes parties respectives de la rétine de l'œil (160) à différents emplacements respectifs sur la rétine sont réparties sur la au moins une partie de la rétine de l'œil (160).

15. Programme informatique comprenant des instructions lisibles par ordinateur qui, lorsqu'elles sont exécutées par un ordinateur, amènent l'ordinateur à exécuter un procédé selon au moins l'une des revendications 13 et 14.
